# EUROPEAN PATENT APPLICATION

(11) **EP 4 059 347 A1**
(43) Date of publication of application: **21.09.2022**
(21) Application number: 20886807.5
(22) Date of filing: 06.10.2020
(51) Int. Cl.: A01N 1/02, C07C 215/08, C07C 215/10, C07C 215/12, C07H 21/00, C07K 1/02, C12N 9/96

(54) **ORGANIC AMMONIUM SALT AND HYDROGEN BOND MATERIAL TREATING AGENT USING SAME**

(30) Priority: 14.11.2019 JP 2019206184
(71) Applicant: Miyoshi Oil & Fat Co., Ltd., Tokyo 124-8510 (JP)
(72) Inventor: KANEKO Kotaro, Tokyo 124-8510 (JP); KAWAI Koji, Tokyo 124-8510 (JP)
(74) Representative: Angerhausen, Christoph
(86) International application number: PCT/JP2020/037839
(87) International publication number: WO 2021/095398

(57) **Abstract**

Provided are a novel compound and a hydrogen-bonding material treatment agent that are useful for treating various hydrogen-bonding materials such as biological samples. They are an organic ammonium salt and a hydrogen-bonding material treatment agent containing the same. The organic ammonium salt is solid at 25°C, and contains an anion and an ammonium cation represented by the following formula (I):

N ⁺ R ₙ H ₄ - ₙ (I)

wherein R independently represents, for example, a hydroxyalkyl group in which there is at least one hydroxy group, an alkyl moiety is a linear or branched moiety having 1 to 10 carbon atoms, and the alkyl moiety may contain an oxygen atom(s), and n represents an integer of 0 to 4.

## Description

### TECHNICAL FIELD

The present invention relates to an organic ammonium salt and a hydrogen-bonding material treatment agent using the same.

### BACKGROUND ART

Many biological samples including, for example, biocatalysts such as enzymes and yeasts; peptides; proteins; nucleic acids; and poorly-soluble polysaccharides such as cellulose, are likely to have the steric structures of their molecules broken due to the impact of, for example, temperature, pH, a solvent or an electrostatic repulsive force between the molecules, and will thus exhibit an impaired activity i.e. catalytic capability. The steric structures of the active sites and the steric structures of the amino acid residues need to be retained when those samples are utilized to preserve a biological catalytic capability and perform a biocatalytic reaction, or when preserving a biological sample.

As a method for preserving a biocatalyst or biological sample for a long period of time, there are known a freeze-drying method where the biocatalyst or biological sample is to be preserved in the form of a powder; and a freeze storage method where the biocatalyst or biological sample is to be dissolved in a solution at a low concertation and preserved under an extremely low temperature.

In general, it is desired that preservation be carried out in the form of a solution in terms of ease of operation. However, in the case of the freeze storage method, other than the fact that a special device is required, there are a problem that the steric structure of a biocatalyst will be destroyed as ices will be generated at the time of freezing, and a problem that the activity of the biocatalyst will be impaired as the structure thereof changes after melting the frozen solution before use; further, an efficient preservation has been difficult due to a low preservation concentration of about 1 to 3 mg/mL in general in the case of, for example, urease and catalase.

Attempts have been made to add a stabilizer so as to prevent such deactivation of a biocatalyst and maintain the activity of an enzyme. For example, there are proposed a method of using a multivalent alcohol such as glycerin and sorbitol to preserve uricase (Patent document 1); and a method for stabilizing cholesterol oxidase by adding bovine serum albumin and saccharides to a cholesterol oxidase-containing solution (Patent document 2); these methods have a problem of an impaired enzyme activity with respect to a preservation concentration, preservation temperature and preservation period.

In order to solve the above problems, the applicant found that an ionic liquid which is liquid at room temperature can be used for preserving a biocatalyst and a biological sample (Patent documents 3 to 5). While an ionic liquid which is liquid at room temperature is superior in affinity to a biological sample and preservation thereof, it is not suitable for uses requiring a solid form at room temperature, such as uses of clinical reagents and biosensors i.e. further improvements are needed. Moreover, if preserved in the form of a liquid, it is difficult to preserve a biological sample at a high concentration in terms of a solubility between the biological sample and the liquid. Thus, freeze-drying and freeze storage have been employed where preservation is conducted in a solid state; there has been a problem of a low long-term stabilizing effect of a biological sample.

As an example of a solid enzyme stabilizer, there has been disclosed one using a plant-derived polypeptide as a hydrolysate of soy beans or the like (Patent document 6); the stabilizing effect thereof is insufficient.

Other than such biological sample preserving material, desired are a treatment agent capable of treating various hydrogen-bonding materials such as biological samples e.g. a treatment agent exhibiting a superior solubility or dispersibility when adding water, a solvent or the like to a hydrogen-bonding material that has been turned into a solid composition; as well as a novel compound enabling such treatment agent and having a favorable affinity to a hydrogen-bonding material.

### PRIOR ART DOCUMENTS

### Patent documents

Patent document 1: JP-A-Hei 06-70798
Patent document 2: JP-A-Hei 08-187095
Patent document 3: JP-A-2014-131974
Patent document 4: JP-A-2014-131975
Patent document 5: WO2015/156398
Patent document 6: JP-A-2006-42757

### SUMMARY OF THE INVENTION

### Problems to be solved by the invention

The present invention was made in view of the abovementioned circumstances, and one of the objects of the present invention is to provide a novel compound useful in, for example, treating various hydrogen-bonding materials.

Another object of the present invention is to provide a treatment agent useful in treating various hydrogen-bonding materials.

One other object of the present invention is to provide a treatment agent that is superior, when in a solid state, in retaining the steric structure of a biological sample even when the biological sample is to be preserved at a high concentration and a high temperature for a long period of time; and a biological sample solution that is superior, when in a solution state such as an aqueous solution, in retaining the steric structure of a biological sample even when the biological sample is to be preserved at a high concentration and a high temperature for a long period of time.

Yet another object of the present invention is to provide a treatment agent having an excellent solubility and dispersibility with regard to an organic or inorganic hydrogen-bonding material, and exhibiting a superior solubility or dispersibility when adding water, a solvent or the like to a solid composition that has been produced from the solution or dispersion liquid obtained.

### Means to solve the problems

In order to solve the aforementioned problems, an organic ammonium salt of the present invention is characterized by comprising an anion and an ammonium cation represented by the following formula (I):
[Chemical formula 1]

N ⁺ R ₙ H _{4 - n} ( I )

wherein R independently represents a hydroxyalkyl group in which there is at least one hydroxy group, an alkyl moiety is a linear or branched moiety having 1 to 10 carbon atoms, and the alkyl moiety may contain an oxygen atom(s); a carboxyalkyl group in which there is at least one carboxy group, an alkyl moiety is a linear or branched moiety having 1 to 10 carbon atoms, and the alkyl moiety may contain an oxygen atom(s); or a hydroxycarboxyalkyl group in which there are at least one hydroxy group and at least one carboxy group, an alkyl moiety is a linear or branched moiety having 1 to 10 carbon atoms, and the alkyl moiety may contain an oxygen atom(s), and
wherein n represents an integer of 0 to 4.

A hydrogen-bonding material treatment agent of the present invention contains the organic ammonium salt.

In a preferable example of the hydrogen-bonding material treatment agent, the hydrogen-bonding material is a biological sample (in this case, also referred to as a biological sample treatment agent hereunder).

A solid composition of the present invention contains the hydrogen-bonding material treatment agent and the hydrogen-bonding material.

A biological sample solution of the present invention contains the hydrogen-bonding material treatment agent (biological sample treatment agent), a biological sample and a solvent.

### Effects of the invention

According to the present invention, provided is a novel compound useful in, for example, treating various hydrogen-bonding materials.

Further, provided is a treatment agent useful in treating various hydrogen-bonding materials such as biological samples.

According to the present invention, provided are a treatment agent that is superior, when in a solid state, in retaining the steric structure of a biological sample even when the biological sample is to be preserved at a high concentration and a high temperature for a long period of time; and a biological sample solution that is superior, when in a solution state such as an aqueous solution, in retaining the steric structure of a biological sample even when the biological sample is to be preserved at a high concentration and a high temperature for a long period of time.

According to the present invention, provided is a treatment agent having an excellent solubility and dispersibility with regard to an organic or inorganic hydrogen-bonding material, and exhibiting a superior solubility or dispersibility when adding water, a solvent or the like to a solid composition that has been produced from the solution or dispersion liquid obtained.

### MODE FOR CARRYING OUT THE INVENTION

The present invention is described in detail hereunder.

### (Organic ammonium salt)

With regard to a cation of the organic ammonium salt of the present invention, R in the formula (I) represents a hydroxyalkyl group, a carboxyalkyl group or a hydroxycarboxyalkyl group.

The hydroxyalkyl group has at least one hydroxy group; the alkyl moiety of such hydroxyalkyl group is either a linear or branched moiety preferably having 1 to 10, more preferably 1 to 6 carbon atoms, and such alkyl moiety may also contain an oxygen atom(s).

The carboxyalkyl group has at least one carboxy group; the alkyl moiety of such carboxyalkyl group is either a linear or branched moiety preferably having 1 to 10, more preferably 1 to 6 carbon atoms, and such alkyl moiety may also contain an oxygen atom(s).

The hydroxycarboxyalkyl group has at least one hydroxy group and at least one carboxy group; the alkyl moiety of such hydroxycarboxyalkyl group is either a linear or branched moiety preferably having 1 to 10, more preferably 1 to 6 carbon atoms, and such alkyl moiety may also contain an oxygen atom(s).

Here, when the alkyl moiety contains an oxygen atom(s), such oxygen atom(s) may form, for example, an ether bond, a carbonyl group, an ester bond, an amide bond, a urea bond or a urethane bond in the alkyl moiety. Thus, in the present invention, the expression "the alkyl moiety contains an oxygen atom(s)" includes a case where the alkyl moiety is interrupted by a group that is an oxygen atom-containing atom group and also contains a hetero atom(s) such as a nitrogen atom.

Examples of the hydroxyalkyl group include monohydroxyalkyl groups and polyhydroxyalkyl groups, specific examples of which may include a hydroxyalkyl group whose alkyl moiety contains no oxygen atoms, a hydroxyalkoxyalkyl group, an alkoxyhydroxyalkyl group and a hydroxypolyalkyleneoxyalkyl group.

Although not particularly limited, examples of a monohydroxyalkyl group include a hydroxymethyl group, 1-hydroxyethyl group, 2-hydroxyethyl group, 1-hydroxypropan-1-yl group, 2-hydroxypropan-1-yl group, 3-hydroxypropan-1-yl group, 1-hydroxypropan-2-yl group, 2-hydroxypropan-2-yl group, 1-hydroxybutan-1-yl group, 2-hydroxybutan-1-yl group, 3-hydroxybutan-1-yl group, 4-hydroxybutan-1-yl group, 1-hydroxy-2-methylpropan-1-yl group, 2-hydroxy-2-methylpropan-1-yl group, 3-hydroxy-2-methylpropan-1-yl group, 1-hydroxybutan-2-yl group, 2-hydroxybutan-2-yl group, 3-hydroxybutan-2-yl group, 4-hydroxybutan-2-yl group, 1-hydroxy-2-methylpropan-2-yl group, 5-hydroxypentan-1-yl group, 6-hydroxyhexan-1-yl group, 7-hydroxyheptan-1-yl group, 8-hydroxyoctan-1-yl group, 9-hydroxynonan-1-yl group and 10-hydroxydecan-1-yl group. It is preferred that the monohydroxyalkyl group be that having 1 to 10, more preferably 1 to 6, even more preferably 1 to 3 carbon atoms.

Although not particularly limited, examples of a polyhydroxyalkyl group include a di-, tri-, tetra-, penta-, hexa-, hepta- or octahydroxyalkyl group. Specifically, there may be listed, for example, a dihydroxyethyl group such as 1,2-dihydroxyethyl group; a dihydroxypropan-1-yl group such as 1,2-dihydroxypropan-1-yl group and 2,3-dihydroxypropan-1-yl group; a dihydroxypropan-2-yl group such as 1,2-dihydroxypropan-2-yl group and 1,3-dihydroxypropan-2-yl group; a trihydroxypropan-1-yl group; a trihydroxypropan-2-yl group; a dihydroxybutan-1-yl group such as 1,2-dihydroxybutan-1-yl group, 1,3-dihydroxybutan-1-yl group, 1,4-dihydroxybutan-1-yl group, 2,3-dihydroxybutan-1-yl group, 2,4-dihydroxybutan-1-yl group and 3,4-dihydroxybutan-1-yl group; a trihydroxybutan-1-yl group such as 1,2,3-trihydroxybutan-1-yl group, 1,2,4-trihydroxybutan-1-yl group, 1,3,4-trihydroxybutan-1-yl group and 2,3,4-trihydroxybutan-1-yl group; a tetrahydroxybutan-1-yl group; a dihydroxy-2-methylpropan-1-yl group such as 1,2-dihydroxy-2-methylpropan-1-yl group, 1,3-dihydroxy-2-methylpropan-1-yl group and 2,3-dihydroxy-2-methylpropan-1-yl group; a trihydroxy-2-methylpropan-1-yl group; a tetrahydroxy-2-methylpropan-1-yl group; a dihydroxybutan-2-yl group such as 1,2-dihydroxybutan-2-yl group, 1,3-dihydroxybutan-2-yl group, 1,4-dihydroxybutan-2-yl group, 2,3-dihydroxybutan-2-yl group, 2,4-dihydroxybutan-2-yl group and 3,4-dihydroxybutan-2-yl group; a trihydroxybutan-2-yl group such as 1,2,3-trihydroxybutan-2-yl group, 1,2,4-trihydroxybutan-2-yl group, 1,3,4-trihydroxybutan-2-yl group and 2,3,4-trihydroxybutan-2-yl group; a tetrahydroxybutan-2-yl group; a 1,3-dihydroxy-2-methylpropan-2-yl group, 1,3-dihydroxy-2-ethylpropan-2-yl group and 1,3-dihydroxy-2-hydroxymethylpropan-2-yl group; a di-, tri-, tetra- or pentahydroxypentan-1-yl group; a di-, tri-, tetra-, penta- or hexahydroxyhexan-1-yl group; a di-, tri-, tetra-, penta-, hexa- or heptahydroxyheptan-1-yl group; and a di-, tri-, tetra-, penta-, hexa-, hepta- or octahydroxyoctan-1-yl group. It is preferred that the polyhydroxyalkyl group be that having 2 to 6 hydroxy groups and having 1 to 10, preferably 1 to 6 carbon atoms. Further, a preferable example may be a branched polyhydroxyalkyl group represented by the following formula (II). 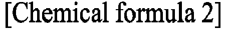 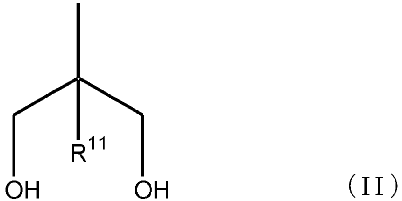 (In this formula, R¹¹ represents a hydrogen atom, a linear alkyl group having 1 to 4 carbon atoms, or a linear monohydroxyalkyl group having 1 to 4 carbon atoms.)

Even among the above polyhydroxyalkyl groups, preferred are 2,3-dihydroxypropan-1-yl group, 1,3-dihydroxypropan-2-yl group, 1,3-dihydroxy-2-ethylpropan-2-yl group, 1,3-dihydroxy-2-hydroxymethylpropan-2-yl group and pentahydroxyhexan-1-yl group.

Examples of the carboxyalkyl group include monocarboxyalkyl groups and polycarboxyalkyl groups, specific examples of which may include those obtained by substituting the hydroxy groups in the above exemplified mono-, di-, tri-, tetra-, penta-, hexa-, hepta- or octahydroxyalkyl groups with carboxy groups.

Although not particularly limited, examples of a monocarboxyalkyl group include a carboxymethyl group, 1-carboxyethyl group, 2-carboxyethyl group, 1-carboxypropan-1-yl group, 2-carboxypropan-1-yl group, 3-carboxypropan-1-yl group, 1-carboxypropan-2-yl group, 2-carboxypropan-2-yl group, 1-carboxybutan-1-yl group, 2-carboxybutan-1-yl group, 3-carboxybutan-1-yl group, 4-carboxybutan-1-yl group, 1-carboxy-2-methylpropan-1-yl group, 2-carboxy-2-methylpropan-1-yl group, 3-carboxy-2-methylpropan-1-yl group, 1-carboxybutan-2-yl group, 2-carboxybutan-2-yl group, 3-carboxybutan-2-yl group, 4-carboxybutan-2-yl group, 1-carboxy-2-methylpropan-2-yl group, 5-carboxypentan-1-yl group, 6-carboxyhexan-1-yl group, 7-carboxyheptan-1-yl group, 8-carboxyoctan-1-yl group, 9-carboxynonan-1-yl group and 10-carboxydecan-1-yl group. It is preferred that the carboxyalkyl group be that having 1 to 10, more preferably 1 to 6 carbon atoms.

Although not particularly limited, examples of the hydroxycarboxyalkyl group include those obtained by substituting part of the hydroxy groups in the above exemplified di-, tri-, tetra-, penta-, hexa-, hepta- or octahydroxyalkyl groups with carboxy groups.

With regard to the organic ammonium salt of the present invention, n in the formula (I) is an integer of 0 to 4, preferably an integer of 1 to 4. When R in the formula (I) is a polyhydroxyalkyl group, n is preferably 1 to 4, more preferably 1 to 3, even more preferably 1 to 2, particularly preferably 1.

There are no particular restrictions on an anion of the organic ammonium salt of the present invention, examples of which may include a carboxylic acid anion, halide anion, sulfur-based anion, fluorine-based anion, boron-based anion, nitrogen oxide-based anion, phosphorus-based anion and cyano-based anion.

The carboxylic acid anion is an organic acid anion having, per molecule, at least one carboxylic acid anion (-COO⁻), and may also contain a functional group(s) having hetero atoms such as oxygen atoms, nitrogen atoms and sulfur atoms. Although not particularly limited, examples of the carboxylic acid anion include a saturated aliphatic monocarboxylic acid anion, alicyclic carboxylic acid anion, unsaturated aliphatic monocarboxylic acid anion, saturated hydroxycarboxylic acid anion (e.g. saturated hydroxymonocarboxylic acid anion, saturated hydroxydicarboxylic acid anion, or saturated hydroxytricarboxylic acid anion), saturated dicarboxylic acid anion, unsaturated dicarboxylic acid anion, aromatic carboxylic acid anion, saturated carbonyl carboxylic acid anion, alkylether carboxylic acid anion and halogen carboxylic acid anion (carbon numbers of carboxylic acid anions that are mentioned hereunder include carbons of carboxy groups).

The saturated aliphatic monocarboxylic acid anion is comprised of a linear or branched aliphatic saturated hydrocarbon group and one carboxylic acid anion, may contain a carboxy group and/or a carboxylate group, and preferably has 1 to 22 carbon atoms. Specific examples thereof include anions obtained by dissociating protons from, for example, formic acid, acetic acid, propionic acid, butyric acid, valeric acid, caproic acid, enanthic acid, caprylic acid, pelargonic acid, capric acid, lauric acid, myristic acid, pentadecylic acid, palmitic acid, margaric acid, stearic acid, arachidic acid, heneicosylic acid, behenic acid, isobutyric acid, 2-methylbutyric acid, isovaleric acid, 2-ethylhexanoic acid, isononanoic acid, isopalmitic acid and isostearic acid.

When the anion of the organic ammonium salt is the saturated aliphatic monocarboxylic acid anion, examples of the compound of the formula (I) are as follows.
- R in the formula (I) is a linear or branched monohydroxyalkyl group having 1 to 10 carbon atoms, n is an integer of 1 to 4.
- R in the formula (I) is a linear monohydroxyalkyl group having 1 to 6 carbon atoms, n is an integer of 1 to 4.
- R in the formula (I) is 1-hydroxyethyl group, n=1.
- R in the formula (I) is 1-hydroxyethyl group, n=2.
- R in the formula (I) is 1-hydroxyethyl group, n=3.
- R¹¹ in the formula (II) is a hydrogen atom, a linear alkyl group having 1 to 4 carbon atoms, or a linear monohydroxyalkyl group having 1 to 4 carbon atoms, n=1.
- R in the formula (I) is 1,3-dihydroxypropan-2-yl group, n=1.
- R¹¹ in the formula (II) is a linear alkyl group having 1 to 4 carbon atoms, n=1.
- R in the formula (I) is 1,3-dihydroxy-2-ethylpropan-2-yl group, n=1.
- R¹¹ in the formula (II) is a linear monohydroxyalkyl group having 1 to 4 carbon atoms, n=1.
- R in the formula (I) is 1,3-dihydroxy-2-hydroxymethylpropan-2-yl group, n=1.
- R in the formula (I) is a linear or branched polyhydroxyalkyl group having at least two hydroxy groups and 1 to 10 carbon atoms, n is an integer of 1 to 4.
- R in the formula (I) is pentahydroxyhexan-1-yl group, n=1.
- R in the formula (I) is 1-hydroxyethyl group, n=3. The saturated aliphatic monocarboxylic acid anion is a formic acid anion.
- R in the formula (I) is 1,3-dihydroxy-2-ethylpropan-2-yl group, n=1; or R in the formula (I) is 1,3-dihydroxy-2-hydroxymethylpropan-2-yl group, n=1. The saturated aliphatic monocarboxylic acid anion is an acetic acid anion.
- R in the formula (I) is 1,3-dihydroxypropan-2-yl group, n=1; R in the formula (I) is 1,3-dihydroxy-2-hydroxymethylpropan-2-yl group, n=1; or R in the formula (I) is pentahydroxyhexan-1-yl group, n=1. The saturated aliphatic monocarboxylic acid anion is a butyric acid anion.
- R in the formula (I) is 1-hydroxyethyl group, n=1; R in the formula (I) is 1,3-dihydroxypropan-2-yl group, n=1; R in the formula (I) is 1,3-dihydroxy-2-hydroxymethylpropan-2-yl group, n=1; or R in the formula (I) is pentahydroxyhexan-1-yl group, n=1. The saturated aliphatic monocarboxylic acid anion is a caproic acid anion.
- R in the formula (I) is 1-hydroxyethyl group, n=1; R in the formula (I) is 1-hydroxyethyl group, n=3; R in the formula (I) is 1,3-dihydroxypropan-2-yl group, n=1; R in the formula (I) is 1,3-dihydroxypropan-2-yl group, n=1; R in the formula (I) is 1,3-dihydroxy-2-ethylpropan-2-yl group, n= 1; R in the formula (I) is 1,3 -dihydroxy-2-hydroxymethylpropan-2-yl group, n=1; or R in the formula (I) is pentahydroxyhexan-1-yl group, n=1. The saturated aliphatic monocarboxylic acid anion is a caprylic acid anion.
- R in the formula (I) is 1-hydroxyethyl group, n=1; R in the formula (I) is 1-hydroxyethyl group, n=3; R in the formula (I) is 1,3-dihydroxypropan-2-yl group, n=1; R in the formula (I) is 1,3-dihydroxy-2-ethylpropan-2-yl group, n=1; R in the formula (I) is 1,3-dihydroxy-2-hydroxymethylpropan-2-yl group, n=1; or R in the formula (I) is pentahydroxyhexan-1-yl group, n=1. The saturated aliphatic monocarboxylic acid anion is a capric acid anion.
- R in the formula (I) is 1-hydroxyethyl group, n=1; R in the formula (I) is 1-hydroxyethyl group, n=2; R in the formula (I) is 1-hydroxyethyl group, n=3; R in the formula (I) is 1,3-dihydroxypropan-2-yl group, n=1; R in the formula (I) is 1,3-dihydroxy-2-ethylpropan-2-yl group, n=1; R in the formula (I) is 1,3-dihydroxy-2-hydroxymethylpropan -2-yl group, n=1; or R in the formula (I) is pentahydroxyhexan-1-yl group, n=1. The saturated aliphatic monocarboxylic acid anion is a lauric acid anion.

The alicyclic carboxylic acid anion is comprised of a saturated or unsaturated carbon ring having no aromaticity and at least one carboxylic acid anion, and preferably has 6 to 20 carbon atoms. Particularly, preferred is a cyclohexane ring skeleton-containing alicyclic carboxylic acid anion, specific examples of which include anions obtained by dissociating protons from cyclohexane carboxylic acid and cyclohexane dicarboxylic acid.

When the anion of the organic ammonium salt is the alicyclic carboxylic acid anion, examples of the compound of the formula (I) are as follows.
- R in the formula (I) is a linear or branched monohydroxyalkyl group having 1 to 10 carbon atoms, n is an integer of 1 to 4.
- R in the formula (I) is a linear monohydroxyalkyl group having 1 to 6 carbon atoms, n is an integer of 1 to 4.
- R in the formula (I) is 1-hydroxyethyl group, n=1.
- R in the formula (I) is 1-hydroxyethyl group, n=2.
- R¹¹ in the formula (II) is a hydrogen atom, a linear alkyl group having 1 to 4 carbon atoms, or a linear monohydroxyalkyl group having 1 to 4 carbon atoms, n=1.
- R in the formula (I) is 1,3-dihydroxypropan-2-yl group, n=1.
- R in the formula (I) is 1-hydroxyethyl group, n=1; R in the formula (I) is 1-hydroxyethyl group, n=3; or R in the formula (I) is 1,3-dihydroxypropan-2-yl group, n=1. The alicyclic carboxylic acid anion is a cyclohexane carboxylic acid anion.

The unsaturated aliphatic monocarboxylic acid anion is comprised of a linear or branched aliphatic unsaturated hydrocarbon group and at least one carboxylic acid anion, may contain a carboxy group and/or a carboxylate group, and preferably has 3 to 22 carbon atoms. Specific examples thereof include anions obtained by dissociating protons from, for example, acrylic acid, methacrylic acid, crotonic acid, palmitoleic acid, oleic acid, vaccenic acid, linoleic acid, linolenic acid, eleostearic acid and arachidonic acid.

When the anion of the organic ammonium salt is the unsaturated aliphatic monocarboxylic acid anion, examples of the compound of the formula (I) are as follows.
- R in the formula (I) is a linear or branched monohydroxyalkyl group having 1 to 10 carbon atoms, n is an integer of 1 to 4.
- R in the formula (I) is a linear monohydroxyalkyl group having 1 to 6 carbon atoms, n is an integer of 1 to 4.
- R in the formula (I) is 1-hydroxyethyl group, n=1.
- R¹¹ in the formula (II) is a linear alkyl group having 1 to 4 carbon atoms, n=1.
- R in the formula (I) is 1,3-dihydroxy-2-ethylpropan-2-yl group, n=1.
- R in the formula (I) is 1-hydroxyethyl group, n=1. The unsaturated aliphatic monocarboxylic acid anion is a crotonic acid anion.
- R in the formula (I) is 1,3-dihydroxy-2-ethylpropan-2-yl group. The unsaturated aliphatic monocarboxylic acid anion is an oleic acid anion.

The saturated hydroxycarboxylic acid anion is comprised of a linear or branched aliphatic saturated hydrocarbon group, at least one carboxylic acid anion and at least one hydroxy group, may contain a carboxy group and/or a carboxylate group, and preferably has 2 to 24 carbon atoms. Particularly, preferred is a saturated aliphatic hydroxycarboxylic acid anion having 1 to 4 hydroxy groups and 2 to 7 carbon atoms. Specific examples thereof include anions obtained by dissociating protons from, for example, glycolic acid, lactic acid, tartronic acid, glyceric acid, hydroxyacetic acid, hydroxybutyric acid, 2-hydroxydecanoic acid, 3-hydroxydecanoic acid, 12-hydroxystearic acid, dihydroxystearic acid, cerebronic acid, malic acid, tartaric acid, citramalic acid, citric acid, isocitric acid, leucine acid, mevalonic acid, pantoic acid and quinic acid.

When the anion of the organic ammonium salt is the saturated aliphatic hydroxycarboxylic acid anion, and when the anion is particularly a saturated aliphatic monohydroxy carboxylic acid anion, it is preferred that the anion have 2 to 24, more preferably 2 to 7 carbon atoms. The number of the hydroxy groups is preferably 1 to 4. Examples of the compound of the formula (I) are as follows.
- R in the formula (I) is a linear or branched monohydroxyalkyl group having 1 to 10 carbon atoms, n is an integer of 1 to 4.
- R in the formula (I) is a linear monohydroxyalkyl group having 1 to 6 carbon atoms, n is an integer of 1 to 4.
- R in the formula (I) is 1-hydroxyethyl group, n=1.
- R in the formula (I) is 1-hydroxyethyl group, n=2.
- R in the formula (I) is 1-hydroxyethyl group, n=3.
- R¹¹ in the formula (II) is a hydrogen atom, a linear alkyl group having 1 to 4 carbon atoms, or a linear monohydroxyalkyl group having 1 to 4 carbon atoms, n=1.
- R in the formula (I) is 1,3-dihydroxypropan-2-yl group, n=1.
- R in the formula (I) is 1,3-dihydroxy-2-ethylpropan-2-yl group, n=1.
- R¹¹ in the formula (II) is a linear monohydroxyalkyl group having 1 to 4 carbon atoms, n=1.
- R in the formula (I) is 1,3-dihydroxy-2-hydroxymethylpropan-2-yl group, n=1.
- R in the formula (I) is a linear or branched polyhydroxyalkyl group having at least two hydroxy groups and 1 to 10 carbon atoms, n is an integer of 1 to 4.
- R in the formula (I) is pentahydroxyhexan-1-yl group, n=1.
- R in the formula (I) is 1,3-dihydroxy-2-ethylpropan-2-yl group, n=1; or R in the formula (I) is 1,3-dihydroxy-2-hydroxymethylpropan-2-yl group, n=1. The saturated aliphatic monohydroxy carboxylic acid anion is a glycolic acid anion.
- R in the formula (I) is 1,3-dihydroxypropan-2-yl group, n=1; R in the formula (I) is 1,3-dihydroxy-2-ethylpropan-2-yl group, n=1; R in the formula (I) is 1,3-dihydroxy-2-hydroxymethylpropan-2-yl group, n=1; or R in the formula (I) is pentahydroxyhexan-1-yl group, n=1. The saturated aliphatic monohydroxy carboxylic acid anion is 9,10-dihydroxystearic acid anion.
- R in the formula (I) is 1-hydroxyethyl group, n=1; R in the formula (I) is 1-hydroxyethyl group, n=2; R in the formula (I) is 1,3-dihydroxypropan-2-yl group, n=1; or R in the formula (I) is 1,3-dihydroxy-2-ethylpropan-2-yl group, n=1. The saturated aliphatic monohydroxy carboxylic acid anion is a quinic acid anion.
- R in the formula (I) is 1,3-dihydroxypropan-2-yl group, n=1. The saturated aliphatic monohydroxy carboxylic acid anion is 12-hydroxystearic acid anion.

When the anion of the organic ammonium salt is the saturated aliphatic hydroxycarboxylic acid anion, and when the anion is particularly a saturated aliphatic hydroxy dicarboxylic acid anion or a saturated aliphatic hydroxy tricarboxylic acid anion, it is preferred that the anion have 4 to 24 carbon atoms. The number of the hydroxy groups is preferably 1 to 3. Examples of the compound of the formula (I) are as follows.
- R in the formula (I) is a linear or branched monohydroxyalkyl group having 1 to 10 carbon atoms, n is an integer of 1 to 4.
- R in the formula (I) is a linear monohydroxyalkyl group having 1 to 6 carbon atoms, n is an integer of 1 to 4.
- R in the formula (I) is 1-hydroxyethyl group, n=1.
- R in the formula (I) is 1-hydroxyethyl group, n=1. The saturated aliphatic hydroxy dicarboxylic acid anion or the saturated aliphatic hydroxy tricarboxylic acid anion is a tartaric acid anion.
- R in the formula (I) is 1-hydroxyethyl group, n=1. The saturated aliphatic hydroxy dicarboxylic acid anion or the saturated aliphatic hydroxy tricarboxylic acid anion is a citric acid anion.

It is preferred that a saturated dicarboxylic acid anion have 2 to 24, more preferably 2 to 10 carbon atoms. Specific examples thereof include anions obtained by dissociating protons from, for example, oxalic acid, malonic acid, succinic acid, glutaric acid, adipic acid, pimelic acid, suberic acid, azelaic acid and sebacic acid.

When the anion of the organic ammonium salt is the saturated dicarboxylic acid anion, examples of the compound of the formula (I) are as follows.
- R in the formula (I) is a linear or branched monohydroxyalkyl group having 1 to 10 carbon atoms, n is an integer of 1 to 4.
- R in the formula (I) is a linear monohydroxyalkyl group having 1 to 6 carbon atoms, n is an integer of 1 to 4.
- R in the formula (I) is 1-hydroxyethyl group, n=1.
- R in the formula (I) is 1-hydroxyethyl group, n=3.
- R¹¹ in the formula (II) is a hydrogen atom, a linear alkyl group having 1 to 4 carbon atoms, or a linear monohydroxyalkyl group having 1 to 4 carbon atoms, n=1.
- R in the formula (I) is 1,3-dihydroxypropan-2-yl group, n=1.
- R¹¹ in the formula (II) is a linear monohydroxyalkyl group having 1 to 4 carbon atoms, n=1.
- R in the formula (I) is 1,3-dihydroxy-2-hydroxymethylpropan-2-yl group, n=1.
- R in the formula (I) is 1,3-dihydroxypropan-2-yl group, n=1. The saturated aliphatic monohydroxy carboxylic acid anion is an oxalic acid anion.
- R in the formula (I) is 1-hydroxyethyl group, n=1; or R in the formula (I) is 1,3-dihydroxy-2-hydroxymethylpropan-2-yl group, n=1. The saturated dicarboxylic acid anion is a succinic acid anion.
- R in the formula (I) is 1-hydroxyethyl group, n=1; R in the formula (I) is 1,3-dihydroxypropan-2-yl group, n=1; or R in the formula (I) is 1,3-dihydroxy-2-hydroxymethylpropan-2-yl group, n=1. The saturated dicarboxylic acid anion is an adipic acid anion.
- R in the formula (I) is 1,3-dihydroxypropan-2-yl group. The saturated dicarboxylic acid anion is a sebacic acid anion.

It is preferred that an unsaturated dicarboxylic acid anion have 2 to 24, more preferably 2 to 10 carbon atoms. Specific examples thereof include anions obtained by dissociating protons from, for example, maleic acid, fumaric acid, citraconic acid, mesaconic acid, 2-pentenedioic acid, methylenesuccinic acid, allylmalonic acid, isopropylidene succinic acid, adipic acid and 2,4-hexadienedioic acid.

When the anion of the organic ammonium salt is the unsaturated dicarboxylic acid anion, examples of the compound of the formula (I) are as follows.
- R in the formula (I) is a linear or branched monohydroxyalkyl group having 1 to 10 carbon atoms, n is an integer of 1 to 4.
- R in the formula (I) is a linear monohydroxyalkyl group having 1 to 6 carbon atoms, n is an integer of 1 to 4.
- R in the formula (I) is 1,3-dihydroxypropan-2-yl group, n=1.
- R in the formula (I) is 1,3-dihydroxypropan-2-yl group, n=1. The unsaturated dicarboxylic acid anion is a maleic acid anion.

The aromatic carboxylic acid anion contains a single or multiple ring(s) having aromaticity and at least one carboxylic acid anion, and preferably has 6 to 20 carbon atoms. Particularly, preferred is a benzene ring skeleton-containing aromatic carboxylic acid anion, specific examples of which include anions obtained by dissociating protons from, for example, benzoic acid, cinnamic acid, phthalic acid, isophthalic acid and terephthalic acid.

Among the above aromatic carboxylic acid anions, an aromatic hydroxycarboxylic acid anion is comprised of a single or multiple ring(s) having aromaticity, at least one carboxylic acid anion and at least one hydroxy group, and preferably has 6 to 20 carbon atoms. Particularly, preferred is a benzene ring skeleton-containing aromatic carboxylic acid anion having 1 to 3 hydroxy groups, specific examples of which include anions obtained by dissociating protons from, for example, salicylic acid, hydroxybenzoic acid, dihydroxybenzoic acid, trihydroxybenzoic acid, hydroxymethylbenzoic acid, vanillic acid, syringic acid, protocatechuic acid, gentisic acid, orsellinic acid, mandelic acid, benzylic acid, atrolactic acid, phloretic acid, coumaric acid, umbellic acid, caffeic acid, ferulic acid and sinapinic acid.

When the anion of the organic ammonium salt is the aromatic carboxylic acid anion, examples of the compound of the formula (I) are as follows.
- R in the formula (I) is a linear or branched monohydroxyalkyl group having 1 to 10 carbon atoms, n is an integer of 1 to 4.
- R in the formula (I) is a linear monohydroxyalkyl group having 1 to 6 carbon atoms, n is an integer of 1 to 4.
- R in the formula (I) is 1-hydroxyethyl group, n=1.
- R in the formula (I) is 1-hydroxyethyl group, n=2.
- R in the formula (I) is 1-hydroxyethyl group, n=3.
- R¹¹ in the formula (II) is a hydrogen atom, a linear alkyl group having 1 to 4 carbon atoms, or a linear monohydroxyalkyl group having 1 to 4 carbon atoms, n=1.
- R in the formula (I) is 1,3-dihydroxypropan-2-yl group, n=1.
- R¹¹ in the formula (II) is a linear alkyl group having 1 to 4 carbon atoms, n=1.
- R in the formula (I) is 1,3-dihydroxy-2-ethylpropan-2-yl group, n=1.
- R¹¹ in the formula (II) is a linear monohydroxyalkyl group having 1 to 4 carbon atoms, n=1.
- R in the formula (I) is 1,3-dihydroxy-2-hydroxymethylpropan-2-yl group, n=1.
- R in the formula (I) is a linear or branched polyhydroxyalkyl group having at least two hydroxy groups and 1 to 10 carbon atoms, n is an integer of 1 to 4.
- R in the formula (I) is pentahydroxyhexan-1-yl group, n=1.
- R in the formula (I) is 1-hydroxyethyl group, n=1; R in the formula (I) is 1-hydroxyethyl group, n=3; R in the formula (I) is 1,3-dihydroxypropan-2-yl group, n=1; R in the formula (I) is 1,3-dihydroxy-2-ethylpropan-2-yl group, n=1; R in the formula (I) is 1,3-dihydroxy-2-hydroxymethylpropan-2-yl group, n=1; or R in the formula (I) is pentahydroxyhexan-1-yl group, n=1. The aromatic carboxylic acid anion is a benzoic acid anion.
- R in the formula (I) is 1-hydroxyethyl group, n=1; R in the formula (I) is 1-hydroxyethyl group, n=2; R in the formula (I) is 1-hydroxyethyl group, n=3; or R in the formula (I) is 1,3-dihydroxypropan-2-yl group, n=1. The aromatic carboxylic acid anion is a terephthalic acid anion.
- R in the formula (I) is 1-hydroxyethyl group, n=1; R in the formula (I) is 1,3-dihydroxy-2-ethylpropan-2-yl group, n=1; or R in the formula (I) is 1,3-dihydroxy-2-hydroxymethylpropan-2-yl group, n=1. The aromatic carboxylic acid anion is a salicylic acid anion.
- R in the formula (I) is 1-hydroxyethyl group, n=1; R in the formula (I) is 1-hydroxyethyl group, n=3; or R in the formula (I) is 1,3-dihydroxypropan-2-yl group, n=1. The aromatic carboxylic acid anion is p-hydroxybenzoic acid anion.
- R in the formula (I) is 1-hydroxyethyl group, n=1. The aromatic carboxylic acid anion is a mandelic acid anion.

The saturated carbonyl carboxylic acid anion is a carboxylic acid anion having a carbonyl group(s) in the molecule and having 3 to 22 carbon atoms, preferably a saturated carbonyl carboxylic acid anion having 1 to 2 carbonyl groups and 3 to 7 carbon atoms. Particularly, preferred is a saturated carbonyl carboxylic acid anion expressed by CH₃((CH₂)ₚCO(CH₂)_{q})COO⁻ (p and q each represent an integer of 0 to 2). Specific examples thereof include anions obtained by dissociating protons from, for example, pyruvic acid.

When the anion of the organic ammonium salt is the saturated carbonyl carboxylic acid anion, examples of the compound of the formula (I) are as follows.
- R in the formula (I) is a linear or branched polyhydroxyalkyl group having at least two hydroxy groups and 1 to 10 carbon atoms, n is an integer of 1 to 4.
- R in the formula (I) is pentahydroxyhexan-1-yl group, n=1.
- R in the formula (I) is pentahydroxyhexan-1-yl group, n=1. The saturated carbonyl carboxylic acid anion is a pyruvic acid anion.

The alkylether carboxylic acid anion is a carboxylic acid anion having an ether group(s) in the molecule and having 2 to 22 carbon atoms, preferably an alkyl carboxylic acid anion having 1 to 2 ether groups and 2 to 12 carbon atoms, including a polyoxyalkylene alkylether carboxylic acid anion. Particularly, preferred are an alkylether carboxylic acid anion expressed by CH₃(CH₂)ᵣO(CH₂)ₛCOO⁻ (r and s each represent an integer of 0 to 4) and a polyoxyethylene alkylether carboxylic acid anion. Specific examples thereof include anions obtained by dissociating protons from, for example, methoxyacetic acid, ethoxyacetic acid, methoxybutyric acid and ethoxybutyric acid.

When the anion of the organic ammonium salt is the alkylether carboxylic acid anion, examples of the compound of the formula (I) are as follows.
- R¹¹ in the formula (II) is a linear alkyl group having 1 to 4 carbon atoms, n=1.
- R in the formula (I) is 1,3-dihydroxy-2-ethylpropan-2-yl group, n=1.
- R in the formula (I) is 1,3-dihydroxy-2-ethylpropan-2-yl group, n=1. The alkylether carboxylic acid anion is a methoxyacetic acid anion.

As the halogen carboxylic acid anion, a halogen carboxylic acid anion having 2 to 22 carbon atoms is preferred. Specific examples thereof include anions obtained by dissociating protons from fluorine-substituted halogen carboxylic acids or the like including trifluoroacetic acid, trichloroacetic acid, tribromoacetic acid, pentafluoropropionic acid, pentachloropropionic acid, pentabromopropionic acid, perfluorononanoic acid, perchlorononanoic acid and perbromononanoic acid.

When the anion of the organic ammonium salt is the halogen carboxylic acid anion, examples of the compound of the formula (I) are as follows.
- R in the formula (I) is a linear or branched monohydroxyalkyl group having 1 to 10 carbon atoms, n is an integer of 1 to 4.
- R in the formula (I) is a linear monohydroxyalkyl group having 1 to 6 carbon atoms, n is an integer of 1 to 4.
- R in the formula (I) is 1-hydroxyethyl group, n=1.
- R¹¹ in the formula (II) is a linear alkyl group having 1 to 4 carbon atoms, n=1.
- R in the formula (I) is 1,3-dihydroxy-2-ethylpropan-2-yl group, n=1.
- R¹¹ in the formula (II) is a linear monohydroxyalkyl group having 1 to 4 carbon atoms, n=1.
- R in the formula (I) is 1,3-dihydroxy-2-hydroxymethylpropan-2-yl group, n=1.
- R in the formula (I) is 1-hydroxyethyl group, n=1; R in the formula (I) is 1,3-dihydroxy-2-ethylpropan-2-yl group, n=1; or R in the formula (I) is 1,3-dihydroxy-2-hydroxymethylpropan-2-yl group, n=1. The halogen carboxylic acid anion is a trifluoroacetic acid anion.

There are no particular restrictions on the halide anion, examples of which may include a chloride ion, bromide ion and iodine ion.

When the anion of the organic ammonium salt is the halide anion, examples of the compound of the formula (I) are as follows.
- R in the formula (I) is 1,3-dihydroxy-2-hydroxymethylpropan-2-yl group, n=1.
- R in the formula (I) is 1,3-dihydroxy-2-hydroxymethylpropan-2-yl group, n=1. The halide anion is a bromide ion.
- R in the formula (I) is 1,3-dihydroxy-2-hydroxymethylpropan-2-yl group, n=1. The halide anion is a chloride ion.

Examples of the sulfur-based anion include a sulfate anion, sulfite anion, sulfonate anion, hydrogen sulfonate anion, alkylsulfonate anion (e.g. methane sulfonate, ethane sulfonate, butane sulfonate, benzene sulfonate, p-toluene sulfonate, 2,4,6-trimethylbenzene sulfonate, styrene sulfonate, 3-sulfopropyl methacrylate anion, 3-sulfopropyl acrylate), sulfate anion, hydrogen sulfate anion, and alkyl sulfate anion (e.g. methyl sulfate anion, ethyl sulfate anion, butyl sulfate anion, octyl sulfate anion, 2-(2-methoxyethoxy)ethyl sulfate anion).

When the anion of the organic ammonium salt is the sulfur-based anion, examples of the compound of the formula (I) are as follows.
- R¹¹ in the formula (II) is a linear monohydroxyalkyl group having 1 to 4 carbon atoms, n=1.
- R in the formula (I) is 1,3-dihydroxy-2-hydroxymethylpropan-2-yl group, n=1.
- R in the formula (I) is a linear or branched polyhydroxyalkyl group having at least two hydroxy groups and 1 to 10 carbon atoms, n is an integer of 1 to 4.
- R in the formula (I) is pentahydroxyhexan-1-yl group, n=1.
- R in the formula (I) is 1,3-dihydroxy-2-hydroxymethylpropan-2-yl group, n=1; or R in the formula (I) is pentahydroxyhexan-1-yl group, n=1. The sulfur-based anion is a sulfate anion.

Examples of the fluorine-based anion include a bis(fluorosulfonyl)imide anion, bis(perfluoroalkylsulfonyl)imide anion (e.g. bis(trifluoromethylsulfonyl)imide anion, bis(pentafluoroethylsulfonyl)imide, bis(heptafluoropropanesulfonyl)imide anion, bis(nonafluorobutylsulfonyl)imide), perfluoroalkyl sulfonate anion (e.g. trifluoromethane sulfonate anion, pentafluoroethane sulfonate anion, heptafluoropropane sulfonate anion, nonaflate anion, perfluorooctane sulfonate anion), fluorophosphate anion (e.g. hexafluorophosphate anion, tri(pentafluoroethyl)trifluorophosphate anion), tris(perfluoroalkylsulfonyl)methide anion (e.g. tris(trifluoromethanesulfonyl)methide anion, tris(pentafluoroethanesulfonyl)methide anion, tris(heptafluoropropanesulfonyl)methide anion, tris(nonafluorobutanesulfonyl)methide anion), and fluorohydrogenate anion.

When the anion of the organic ammonium salt is the fluorine-based anion, examples of the compound of the formula (I) are as follows.
- R¹¹ in the formula (II) is a hydrogen atom, a linear alkyl group having 1 to 4 carbon atoms, or a linear monohydroxyalkyl group having 1 to 4 carbon atoms, n=1.
- R in the formula (I) is 1,3-dihydroxypropan-2-yl group, n=1.
- R in the formula (I) is 1,3-dihydroxypropan-2-yl group, n=1. The fluorine-based anion is a bis(trifluoromethylsulfonyl)imide anion.

There are no particular restrictions on the boron-based anion, examples of which may include a tetraalkylborate anion such as a tetrafluoroborate anion, bisoxalateborate anion and tetraphenylborate anion.

There are no particular restrictions on the nitrogen oxide-based anion, examples of which may include a nitrate anion and nitrite anion.

Examples of the phosphorus-based anion include a phosphate anion, hydrogen phosphate anion, dihydrogen phosphate anion, phosphonate anion, hydrogen phosphonate anion, dihydrogen phosphonate anion, phosphinate anion, hydrogen phosphinate anion, alkyl phosphate anion (e.g. dimethylphosphate, diethylphosphate, dipropylphosphate anion, dibutylphosphate anion), alkyl phosphonate anion (e.g. methylphosphonate anion, ethylphosphonate anion, propylphosphonate anion, butylphosphonate anion, methylmethylphosphonate anion), alkyl phosphinate anion, and hexaalkyl phosphate anion.

When the anion of the organic ammonium salt is the phosphorus-based anion, examples of the compound of the formula (I) are as follows.
- R¹¹ in the formula (II) is a linear monohydroxyalkyl group having 1 to 4 carbon atoms, n=1.
- R in the formula (I) is 1,3-dihydroxy-2-hydroxymethylpropan-2-yl group, n=1.
- R in the formula (I) is a linear or branched polyhydroxyalkyl group having at least two hydroxy groups and 1 to 10 carbon atoms, n is an integer of 1 to 4.
- R in the formula (I) is pentahydroxyhexan-1-yl group, n=1.
- R in the formula (I) is 1,3-dihydroxy-2-hydroxymethylpropan-2-yl group, n=1; or R in the formula (I) is pentahydroxyhexan-1-yl group, n=1. The phosphorus-based anion is a dihydrogen phosphate anion.

There are no particular restrictions on the cyano-based anion, examples of which may include a tetracyanoborate anion, dicyanamide anion, thiocyanate anion and isothiocyanate anion.

When the anion of the organic ammonium salt is the cyano-based anion, examples of the compound of the formula (I) are as follows.
- R¹¹ in the formula (II) is a hydrogen atom, a linear alkyl group having 1 to 4 carbon atoms, or a linear monohydroxyalkyl group having 1 to 4 carbon atoms, n=1.
- R in the formula (I) is 1,3-dihydroxypropan-2-yl group, n=1.
- R¹¹ in the formula (II) is a linear monohydroxyalkyl group having 1 to 4 carbon atoms, n=1.
- R in the formula (I) is 1,3-dihydroxy-2-hydroxymethylpropan-2-yl group, n=1.
- R in the formula (I) is a linear or branched polyhydroxyalkyl group having at least two hydroxy groups and 1 to 10 carbon atoms, n is an integer of 1 to 4.
- R in the formula (I) is pentahydroxyhexan-1-yl group, n=1.
- R in the formula (I) is 1,3-dihydroxypropan-2-yl group, n=1; R in the formula (I) is 1,3-dihydroxy-2-hydroxymethylpropan-2-yl group, n=1; or R in the formula (I) is pentahydroxyhexan-1-yl group, n=1. The cyano-based anion is a dicyanamide anion.

Among the anions of the organic ammonium salt of the present invention, preferred are a carboxylic acid anion, halide anion, sulfur-based anion, fluorine-based anion, nitrogen oxide-based anion, phosphorus-based anion and cyano-based anion; particularly, a carboxylic acid anion is preferred in terms of safety, of which preferred are a saturated aliphatic monocarboxylic acid anion, alicyclic carboxylic acid anion, unsaturated aliphatic monocarboxylic acid anion, saturated hydroxycarboxylic acid anion, saturated dicarboxylic acid anion, unsaturated dicarboxylic acid anion, saturated hydroxydicarboxylic acid anion, saturated hydroxytricarboxylic acid anion, aromatic carboxylic acid anion, saturated carbonyl carboxylic acid anion, alkylether carboxylic acid anion and halogen carboxylic acid anion.

Although not particularly limited, the organic ammonium salt of the present invention may, for example, be synthesized in the following manner.

An alkanolamine having at least one hydroxy group, an amino acid having at least one carboxy group, or an aminohydroxyalkanoic acid having at least one hydroxy group and at least one carboxy group, each of which corresponds to R in the formula (I); and an organic acid or inorganic acid which corresponds to anion are to be reacted in a solvent such as water and an organic solvent. Alternatively, an alkanolamine having at least one hydroxy group, an amino acid having at least one carboxy group, or an aminohydroxyalkanoic acid having at least one hydroxy group and at least one carboxy group, each of which corresponds to R in the formula (I); and an organic halogen compound such as alkylene halohydrin, monohaloalkyl carboxylic acid and monohalohydroxyalkyl carboxylic acid are to be reacted in a solvent to obtain a compound, followed by further reacting such compound with an organic acid or inorganic acid which corresponds to the anion of the target compound in a solvent such as water and an organic solvent.

An alkanolamine (e.g. mono-, di-, trialkanolamine, 2-amino-1,3-propanediol, 2-amino-2-ethyl-1,3-propanediol, tris(hydroxymethyl)aminomethane, D-glucamine), an amino acid comprised of carboxyalkyl group(s) (e.g. glycine, aspartic acid, glutamic acid), or an aminohydroxyalkanoic acid having at least one hydroxy group and at least one carboxy group (e.g. 3-amino-2-hydroxy propionic acid), each of which corresponds to the ammonium cation of the formula (I); and an organic acid or inorganic acid which corresponds to anion are to be reacted in a polar solvent such as water and acetonitrile. A reaction temperature and a reaction time vary depending on, for example, the types of raw materials; for example, the reaction may be performed under room temperature for about 1 hour to 1 day. Next, the solvent is to be distilled away under a reduced pressure, followed by performing purification if necessary so as to obtain the target organic ammonium salt as a solid substance. Further, if the reaction is performed at an equimolar ratio and has then ended, a purification step will not be required, which leads to a further simplification of the production step(s).

Further, the compound where R in the formula (I) is comprised of a hydroxycarboxyalkyl group, hydroxyalkyl group or carboxyalkyl group, and where no hydrogen atom is contained (i.e. n is 4), may, for example, also be synthesized in the following manner.

As a first step, in order to match the structure of the ammonium cation of the formula (I), an aminohydroxyalkanoic acid having at least one hydroxy group and at least one carboxy group (e.g. 3-amino-2-hydroxy propionic acid); and a hydroxyalkyl halide having at least two hydroxy groups or a haloalkyl carboxylic acid having at least two carboxy groups are to be reacted in a polar solvent such as water and acetonitrile. A reaction temperature and a reaction time vary depending on, for example, the types of raw materials; for example, the reaction may be performed under room temperature for about a day. Next, by washing the reactant, there can be obtained a compound comprised of the ammonium cation of the formula (I) and a halide ion. Anion exchange is performed if the halide ion is to be further turned into a target anion. When performing anion exchange, for example, the compound obtained and an organic acid or inorganic acid which corresponds to the anion of the target compound are to be reacted in water. A reaction temperature and a reaction time vary depending on, for example, the types of raw materials; for example, the reaction may be performed under room temperature for about a day. Alternatively, a strong basic ion-exchange resin or the like may be used to perform anion exchange to turn the halide ion into a hydroxide anion, followed by further performing anion exchange with an organic acid or inorganic acid which corresponds to the anion of the target compound, thereby obtaining the target organic ammonium salt.

The organic ammonium salt of the present invention is solid at 25°C. Here, "solid" refers to a state exhibiting no fluidity at 25°C regardless of whether the substance is a crystalline or non-crystalline substance. Although the melting point (freezing point) may vary due to the purity of the organic ammonium salt, a synthesized compound of the formula (I) that is solid at 25°C shall be included in view of conventional common technical knowledge. The purity of the organic ammonium salt may vary depending on a purification method and a molar ratio between the anion and cation in the product; as for the melting point (freezing point) of the organic ammonium salt, since a compound synthesized and further purified in a manner as described in the working example(s) of the present application has a high purity according to the results of IR spectrum and NMR spectrum, and has a melting point that is unambiguously determined by the compound itself, the results of the working examples of the present application shall be referred to. The organic ammonium salt of the present invention may be either in an anhydrous state (anhydride), or a hydrate that has absorbed the water in the air. A hydrate refers to a compound whose moisture percentage has reached a saturated state as a result of absorbing water when left in the air at 25°C. A compound that does not absorb water when left in the air at 25°C contains no hydrate, and shall thus be regarded as an anhydride.

By selecting the functional group and characteristic group of the ammonium cation and the anion, the organic ammonium salt of the present invention is solid at 25°C, and includes, in a broad interpretation, an ionic liquid as an organic salt whose melting point is not higher than 100°C. When reacting in a high-temperature range, an ionic liquid is highly convenient because it is non-volatile due to its structural characteristics and also has a high decomposition temperature such that, for example, an ionic liquid can be reacted even at a temperature of not lower than 100°C which is higher than the temperature of water as the solvent, thereby allowing the applicable range of the reaction temperature to be expanded, and resulting in an extremely low inflammability and combustibility.

The organic ammonium salt of the present invention has a high affinity to a hydrogen-bonding material having a hydrogen-bonding functional group(s) or a hydrogen-bond accepting element(s) that interacts with the hydroxy group and carboxy group of the cation and hydrogen atoms bonded to nitrogen; the organic ammonium salt of the invention can be used for various purposes requiring an affinity to a hydrogen-bonding material capable of undergoing hydrogen bonding, coordinate bonding and ionic bonding.

The organic ammonium salt of the present invention is superior in affinity to a hydrogen-bonding functional group-containing compound or material, because in such organic ammonium salt, the hydrogen of the hydroxy group or carboxy group, or even the hydrogen bonded to nitrogen form hydrogen bonds with, although not particularly limited, for example, elements of a target compound or material that have lone electron pairs such as those of group 15 to group 17 and with a π-electron system compound; or because the oxygen atoms of the hydroxy group and carboxy group of the organic ammonium salt of the present invention form hydrogen bonds with the hydrogen atoms of the target compound or material.

Further, since the organic ammonium salt of the present invention is a hydroxy group and/or carboxy group-containing organic salt, it is superior in affinity to metals, and ionic compounds and materials due to coordinative interactions and electrostatic interactions. Although not particularly limited, as the metals, there may be listed, for example, elements of group 2 to group 14.

Examples of the hydrogen-bonding functional group include a carbon-carbon unsaturated bonding group, an oxygen-containing group, a nitrogen-containing group, a sulfur-containing group, a phosphorus-containing group, and a hydrogen atom directly bonded to nitrogen.

Although not particularly limited, examples of the carbon-carbon unsaturated bonding group include a vinyl group, a vinylene group, an ethynyl group and an unsaturated cyclic hydrocarbon group.

Although not particularly limited, examples of the oxygen-containing group include a hydroxy group, a carbonyl group, an ether group, an ester group, an aldehyde group, a carboxy group, a carboxylate group, a urea group, a urethane group, an amide group, an oxazole group, a morpholine group, a carbamic acid group and a carbamate group.

Although not particularly limited, examples of the nitrogen-containing group include an amino group and a nitro group.

Although not particularly limited, examples of the sulfur-containing group include a sulfate group (-O-S(=O)₂O-), a sulfonyl group (-S(=O)₂O-), a sulfonate group (-S(=O)₂-), a mercapto group (-SH), a thioether group (-S-), a thiocarbonyl group (-C(=S)-), a thiourea group (-NC(=S)-N-), a thiocarboxy group (-C(=S)OH), a thiocarboxylate group (-C(=S)O-), a dithiocarboxy group (-C(=S)SH), and a dithiocarboxylate group (-C(=S)S-).

Although not particularly limited, examples of the phosphorus-containing group include a phosphate group (-O-P(=O)(-O-)-O-), a phosphonate group (-P(=O)(-O-)-O-), a phosphinic acid group (-P(=O)-O-), a phosphorus acid group (-O-P(-O-)-O-), a phosphonous acid group (-P(-O-)-O-), a phosphinous acid group (-P-O-), and a pyrophosphate group [(-OP(=O)(-O-))₂-O-].

As the hydrogen-bonding material having a hydrogen-bond accepting element(s), there may be listed, for example, a material containing a compound having, for example, a hydrogen-bond accepting element(s) as a constituent element(s) of the molecule or a functional group(s), and/or an oxide(s) thereof; and a material surface-modified by a functional group(s) containing these elements and/or the oxides thereof.

The organic ammonium salt of the present invention is superior in affinity to a hydrogen-bonding material, since the organic ammonium salt has, in its cation, any one of a hydroxy group, a carboxy group and a nitrogen atom-bonded hydrogen atom as hydrogen-bonding functional groups. Further, the affinity to a hydrogen-bonding material will be even more excellent if a hydrogen-bonding functional group(s) are also present in the anion of the organic ammonium salt.

There are no particular restrictions on the hydrogen-bonding material; there may be listed, for example, a biological sample, and an organic or inorganic compound material.

Examples of the biological sample include a biocatalyst such as an enzyme; a peptide; a protein; a nucleic acid; poorly-soluble polysaccharides such as cellulose; cells; a cell tissue fluid; a cell membrane; blood; body tissues; and an antibody-antigen.

As the organic compound material, there may be used a compound having the abovementioned hydrogen-bonding functional group(s); although not particularly limited, there may be listed, for example, an organic resin, an organic pigment, an organic dye and an organic fluorescent pigment. Examples of the organic resin include a thermoplastic resin and a thermosetting resin. Although not particularly limited, examples of the organic pigment include an azo-based pigment, a diazo-based pigment, a condensed azo-based pigment, a thioindigobased pigment, an indanthrone-based pigment, a quinacridone-based pigment, an anthraquinone-based pigment, a benzimidazolone-based pigment, a perylene-based pigment, a phthalocyanine-based pigment, an anthrapyridine-based pigment and a dioxazine-based pigment. Although not particularly limited, examples of the organic fluorescent pigment include a rhodamine-based pigment, a squarylium-based pigment, a cyanine-based pigment, an aromatic hydrocarbon-based pigment, an oxazine-based pigment, a carbopyronine-based pigment and a pyrromethene-based pigment. As the organic dye, there may be used, for example, an organic solvent soluble dye categorized as a solvent dye in color index. Specific examples of a solvent dye include Vali Fast Black 3806, Vali Fast Black 3807, Vali Fast Black 3830, Spirit Black SB, Spilon Black GMH, Vali Fast Red 1320, Vali Fast Red 1308, Vali Fast Yellow AUM, Spilon Yellow C2GH, Spilon Violet CRH, Vali Fast Violet 1701, Spilon Red CGH, Spilon Pink BH, Nigrosine Base EX, Oil Blue 613, Neozapon Blue 808 and Vali Fast Blue 1621.

Although not particularly limited, examples of the inorganic compound material include a metal, a metal oxide, a rare-earth metal oxide, a hydroxide, a carbonate, a sulfate, a silicate, a nitride, a titanic acid compound and carbons. Although not particularly limited, examples of the metal include those of group 2 to group 14, such as iron, aluminum, chrome, nickel, cobalt, zinc, tungsten, indium, tin, palladium, zirconium, titanium, copper, silver, gold and platinum. A metal oxide can be preferably used as it is capable of favorably forming hydrogen bonds with the organic ammonium salt of the present invention. Although not particularly limited, examples of such metal oxide include silica, aluminum oxide (alumina), zirconia, titanium oxide, magnesium oxide, indium tin oxide (ITO), cobalt blue (CoO-Al₂O₃), antimony oxide, zinc oxide, cesium oxide, zirconium oxide, yttrium oxide, tungsten oxide, vanadium oxide, cadmium oxide, tantalum oxide, niobium oxide, tin oxide, bismuth oxide, cerium oxide, copper oxide, iron oxide, indium oxide, boron oxide, calcium oxide, barium oxide, thorium oxide, indium tin oxide and ferrite. Examples of the rare-earth metal oxide include dysprosium oxide, erbium oxide, europium oxide, gadolinium oxide, holmium oxide, lanthanum oxide, lutetium oxide, neodymium oxide, praseodymium oxide, samarium oxide, scandium oxide, terbium oxide, thulium oxide and ytterbium oxide. Examples of the hydroxide include calcium hydroxide, magnesium hydroxide, aluminum hydroxide, basic magnesium carbonate and iron hydroxide. Examples of the carbonate include calcium carbonate, magnesium carbonate, zinc carbonate, barium carbonate, dawsonite and hydrotalcite. Examples of the sulfate include calcium sulfate, barium sulfate and aluminum sulfate. Examples of the silicate include calcium silicate, wollastonite, xonotlite, kaolin, talc, clay, mica, montmorillonite, bentonite, dolomite, hydrotalcite, calcium silicate, aluminum silicate, magnesium silicate, zirconium silicate, activated white earth, sepiolite, imogolite, sericite, glass fibers, glass beads and silica-based balloons. Examples of the nitride include aluminum nitride, boron nitride and silicon nitride. Examples of the titanic acid compound include barium titanate, barium zirconate titanate, calcium titanate and strontium titanate. Examples of the carbons include carbon black, graphite, carbon fibers, carbon balloons, activated charcoal, bamboo charcoal, charcoal, single-walled carbon nanotubes, double-walled carbon nanotubes, multiwalled carbon nanotubes, carbon nanohorn and fullerene.

### (Hydrogen-bonding material treatment agent)

The organic ammonium salt of the present invention can be used as a treatment agent for an organic or inorganic hydrogen-bonding material. It is expected that the organic ammonium salt be utilized in, for example, a dispersion medium of a hydrogen-bonding material; a biological sample treatment agent such as a biological sample preserving material and a protein refolding agent; a moisture adjusting agent such as an antifog agent and a humidity conditioning agent; a surface treatment agent such as a dispersant for an inorganic or organic material; an electronic material such as an electrolyte material, an electrically conductive material and a fuel cell; a resin additive such as an antistatic agent; a life science material such as a medicinal product, a cosmetic product, a perfumery product, an antibacterial agent, a disinfectant and a deodorant; an industrial material such as a reaction aid, a heat medium, an adhesive agent, an antifog agent, an adsorbent, a heat insulator and a polymer base material; and an agricultural material such as an agricultural sustained release agent.

The hydrogen-bonding material treatment agent of the present invention contains the above-described organic ammonium salt of the present invention. Here, "contains" refers to a condition where while the treatment agent is mainly targeted at one comprised of the organic ammonium salt of the present invention, the treatment agent may also be that mixed with other allowable and optional ingredients depending on the purpose of use thereof. Although not particularly limited, examples of such other optional ingredients include a solvent and dispersion medium such as water and an organic solvent; the treatment agent may be used as a solution or dispersion liquid thereof. Even among solvents and dispersion medium, for example, water and alcohols having a high affinity to the organic ammonium salt of the present invention can be preferably used.

The organic ammonium salt of the present invention is capable of stably preserving and dispersing a hydrogen-bonding material in the form of a solution or dispersion liquid prepared by mixing the hydrogen-bonding material with water or a solvent. Even in a case where a solid composition is at first obtained by removing water or the solvent from the solution or dispersion liquid, and a solvent is then added thereto to again obtain a solution or dispersion liquid, the hydrogen-bonding material can still be stably preserved, dissolved and dispersed.

The hydrogen-bonding material treatment agent containing the organic ammonium salt of the present invention, a hydrogen-bonding material and a solvent are to be mixed together to obtain a solution or a dispersion liquid, followed by removing the solvent so as to obtain a solid composition with the hydrogen-bonding material being uniformly mixed in the hydrogen-bonding treatment agent. This is useful in terms of preserving the hydrogen-bonding material in the form of a solid. Moreover, this is also useful in terms of a method where a solution or dispersion liquid with the hydrogen-bonding material being uniformly dissolved or dispersed therein is again obtained by adding a solvent to the solid composition i.e. a method of again uniformly causing dissolution and dispersion after a solid state is achieved.

### (Biological sample treatment agent)

Among the above hydrogen-bonding material treatment agents, a biological sample treatment agent is to treat biological samples; as described above, there may be listed, for example, a biological sample preserving material, a protein refolding agent, a stabilizer for a biosensor, a surface treatment agent for a biological sample, a modifier for a biological sample, a hair treatment agent and a skin care agent.

While the hydrogen-bonding material treatment agent, particularly a biological sample treatment agent is mainly described hereunder, the descriptions below also include those of the hydrogen-bonding material treatment agent itself; as for the details of biological samples, and the effects of the organic ammonium salt of the present invention on various treatment agents that are brought about by the affinity to a target hydrogen-bonding material and the interactions with hydrogen-bonding functional groups, the following descriptions are to be referred to.

The hydrogen-bonding material treatment agent of the present invention can be used as a biological sample treatment agent; for example, it may be used for retaining the steric structure of a biological sample, activating a biological sample, retaining the activity of a biological sample, and preserving a biological sample for a long period of time with the activity thereof being retained. Thus, it is useful for, for example, refolding a protein, retaining the activity of an enzyme, and preserving a biological sample with the steric structure thereof being retained.

The biological sample treatment agent of the present invention contains the above-described organic ammonium salt of the present invention. Here, "contains" refers to a condition where while the treatment agent is mainly targeted at one comprised of the organic ammonium salt of the present invention, the treatment agent may also be that mixed with other optional ingredients that are allowable when treating a biological sample.

Examples of the biological sample include a biocatalyst such as an enzyme; a peptide; a protein; a nucleic acid; poorly-soluble polysaccharides such as cellulose; cells; a cell tissue fluid; a cell membrane; blood; body tissues; and an antibody-antigen. Particularly, preferred are a biocatalyst, a protein and a nucleic acid.

Among the biological samples, the biocatalyst refers to a catalyst for a biochemical reaction. The biocatalyst in the present invention includes, for example, microorganisms, animal and plant cells and tissues derived from living organisms, as well as enzymes derived from these organisms; and even artificial compounds having enzyme functions, as well as artificial enzymes endowed with novel properties as a result of artificially modifying natural enzymes and biomolecules.

An enzyme is such that while the primary structure thereof is established by having amino acids unidimensionally bonded together, the sequence and number of those amino acids determine the two-dimensional or higher structures. These structures determine properties unique to each enzyme.

The primary structure is such that 20 types of amino acids are unidimensionally sequenced via peptide binding. Many enzymes are each composed of 100 to 300 amino acids; the amino acid sequence order serves as a piece of information for determining the properties of an enzyme. The secondary structure is such that a certain part (multiple parts) in the entire primary sequence has a high-order and regular structure such as α-helix, β-sheet and β-turn. The tertiary structure is such that the primary and secondary structures are turned into a three-dimensional steric structure. This steric structure determines, for example, an active center as a site of catalytic reaction by an enzyme; and the three-dimensional structure of an amino acid residue composed of a hydrophilic moiety and/or a hydrophobic moiety, thereby causing chemical reactions having substrate specificities and reaction specificities that are unique to biocatalysts such as enzymes, and cannot be found in general proteins (structural proteins, transport proteins, storage proteins, contractile proteins, defensive proteins and hormone proteins). The quaternary structure is an aggregate comprised of multiple molecules of an enzyme having a three-dimensional structure. That is, a biocatalyst such as an enzyme possesses reaction specificities derived from the primary to quaternary structures in addition to the substrate specificities of a protein; in order to retain an activity to a catalytic reaction, it is also critical to retain the tertiary and quaternary structures other than the primary and secondary structures.

Examples of enzymes applicable in the present invention include an oxidoreductase, transferase, hydrolase, lyase, isomerase and synthetase (ligase).

Examples of the oxidoreductase include glucose oxidase, alcohol oxidase, glucose dehydrogenase, alcohol dehydrogenase, fructose dehydrogenase, gluconate dehydrogenase, aldehyde dehydrogenase, amine dehydrogenase, succinate dehydrogenase, p-cresol methylhydroxylase, histamine dehydrogenase, fumarate reductase, nitrate reductase, arsenate reductase, sulfite reductase, catalase, peroxidase and cytochrome P450.

Examples of the transferase include citrate synthase, methyltransferase, phosphotransferase, glycine hydroxymethyltransferase, transketolase, aspartate transaminase, hexokinase, glycerol kinase, creatine kinase, transaminase and transacylase.

Examples of the hydrolase include carboxylesterase, acetyl-CoA hydrolase, alkaline phosphatase, phospholipase, arylsulfatase, amylase, glucoamylase, cellulase, DNA glycosylase, trypsin, chymotrypsin, pepsin, urease, serine protease and lipase.

Examples of the lyase include alginate lyase, pyruvate decarboxylase, phosphoketolase, citrate lyase, phosphopyruvate hydratase, tryptophan synthase, pectin lyase, aspartate ammonia-lyase, cysteine lyase, adenylate cyclase and ferrochelatase.

Examples of the isomerase include amino-acid racemase, tartrate epimerase, glucose-6-phosphate 1-epimerase, maleate isomerase, phenylpyruvate tautomerase, phosphoglucose isomerase, phosphomannomutase and tyrosine 2,3-aminomutase.

Examples of the synthetase include tyrosine tRNA ligase, acetyl-CoA synthetase, asparagine synthetase, GMP synthase, pyruvate carboxylase and DNA ligase.

Examples of microorganisms applicable in the present invention include prokaryotes (bacteria, actinomycetes, archaea) and eukaryotes (molds, yeasts, mushrooms, algae, protozoa). Examples of the animal and plant cells include animal cells, plant cells, cultured animal cells and cultured plant cells.

Examples of the animal and plant-derived tissues include animal tissues and plant tissues.

Many biocatalysts such as enzymes and yeasts are likely to have the steric structures of their molecules broken due to the impact of, for example, temperature, pH, a solvent or an electrostatic repulsive force between the molecules, and will thus exhibit an impaired activity i.e. catalytic capability. Thus, as a method for preserving a biocatalyst for a long period of time, there are known a freeze-drying method where the biocatalyst is to be preserved in the form of a powder; and a freeze storage method where the biocatalyst is to be dissolved in a solution at a low concertation and preserved under an extremely low temperature. In the case of the freeze storage method, not only a special device will be required, but an impaired activity may often be exhibited due to a change(s) in the structure of the biocatalyst when using the frozen solution after melting the same; also, an efficient preservation is difficult due to the low preservation concentration.

In order to improve the affinity of an enzyme, various factors have to be taken into consideration. For example, it is critical to have more enzyme molecules exist per unit volume by inhibiting, via addition of a salt or the like, the interactions between the enzyme molecules that are caused by the repulsive forces (coulomb interactions) generated by the electric charges of the enzyme molecules; and improve an affinity of the amino acid residues such as hydroxy groups, carbonyl groups and amino groups that are present on the enzyme surface in a large amount to a preservative material.

Since an organic ammonium having a salt structure of anion and cation is capable of inhibiting the intermolecular interactions of enzymes themselves, it is expected that the affinity of an enzyme can be improved thereby; imidazolium-based and tetraalkylammonium-based organic ammonium salts that are conventionally known have a low affinity to enzyme surface. Meanwhile, multivalent alcohol-based compounds or the like such as glycerin, propyleneglycol, glucose and trehalose having hydroxy groups with an affinity to the amino acid residues such as hydroxy groups, carbonyl groups and amino groups on the enzyme surface, have a low effect of inhibiting the intermolecular interactions of enzymes, and thus have a low affinity to enzymes. Further, a hydroxy group-containing imidazolium-based organic ammonium salt has a low affinity to enzymes due to its rigid cyclic structure.

In contrast, since the organic ammonium salt used in the biological sample treatment agent of the present invention is an organic ammonium salt having a hydrogen-bonding functional group(s) in the cation or in both the cation and anion, the intermolecular interactions of enzymes can be inhibited; further, a high affinity can be achieved due to the hydrogen-bonding functional group(s) (hydroxy group, carboxy group, ether group, nitrogen atom-bonded hydrogen) that are present in the cation, a high affinity to the amino acid residues such as hydroxy groups, carbonyl groups and amino groups on the enzyme surface, a small molecular size, and a flexible structure. Moreover, the affinity can be further improved by having a hydrogen-bonding functional group(s) even in the anion.

In terms of preservability of enzyme activity, it is necessary to retain the steric structure of an enzyme. In general, an enzyme has substrate specificities and reaction specificities that are expressed from amino acid residues, and shall thus function as a reaction catalyst. A substrate specificity is such that only a particular substrate is to be reacted as a result of recognizing and selecting the structure of a substrate to bind, based on the steric structure and amino acid residues of a reaction site. A reaction specificity is such that the enzyme only catalyzes a particular chemical reaction, and that the steric structure and amino acid residues of a reaction stie as well as metal ions possessed by certain enzymes are involved. For example, metal ions present inside an enzyme such as an oxidoreductase express a catalytic action by three-dimensionally forming a complex with the amino acid residues. That is, the deactivation of, for example, substrate specificities, reaction specificities and metal ions is caused mainly by the destructions of the steric structures of the amino acid residues. Thus, it is critical to retain the steric structure of an enzyme by protecting the amino acid residues such as hydroxy groups, carbonyl groups and amino groups that impart a hydrophilicity to the enzyme surface; and the hydrophilic amino acid residues such as hydroxy groups, carbonyl groups and amino groups as well as hydrophobic functional group-containing amino acid residues inside an active site of the enzyme.

Conventionally, although water and a buffer used as solvents of an enzyme has a high affinity to the hydrophilic sites on the enzyme surface, a catalytic activity cannot be retained thereby as the steric structures of the hydrophobic sites inside the enzyme that are critical in terms of expressing substrate specificities and reaction specificities cannot be protected. While as a stabilizer, there may be used an aqueous solution of bovine serum albumin which is a protein, it is difficult to use the same in the medical field as there are concerns on infection diseases such as BSE. In the case of an aqueous solution using a multivalent alcohol-based stabilizer such as glycerin, propyleneglycol, glucose and trehalose, since there are observed an affinity between the hydrophilic sites on the enzyme surface and the hydroxy groups in these multivalent alcohols; an affinity between the hydrophilic sites inside the enzyme where active sites are present and the hydroxy groups of the multivalent alcohols; and an affinity between the hydrophobic sites inside the enzyme where active sites are present and the hydrophobic alkyl chains in the multivalent alcohols, the steric structure of the enzyme can be retained. However, a preservation stabilizing effect of such aqueous solution is low. In the case of an aqueous solution of a surfactant (amino acid) such as glycine and lycine, the hydrophobic sites in the surfactant shall bind to the hydrophobic amino acid residues in the hydrophobic region inside the enzyme so that such hydrophobic region will turn hydrophilic due to charge generation, and that the hydrophobic region will move to the hydrophilic surface, thereby causing the steric structure of the enzyme to collapse and the enzyme to be deactivated. Further, hydrogen-bonding functional group-free organic ammonium salts such as imidazolium-based and tetraalkylammonium-based organic ammonium salts that are conventionally known as organic ammonium salts, have a low affinity to enzymes as they are incapable of protecting the hydrophilic amino acid residues on the surfaces of and inside the enzymes.

In contrast, in the case of the organic ammonium salt used in the biological sample treatment agent of the present invention, the hydrogen-bonding functional group(s) present in the cation and/or anion composing the salt shall form hydrogen bonds with and thus protect the amino acid residues such as the hydroxy groups, carbonyl groups and amino groups on the surface of and inside an enzyme. Further, by simultaneously protecting the inner amino acid residues having hydrophobic functional groups at the hydrophobic sites of the alkyl chains in the organic ammonium salt, the steric structure of an enzyme can be retained so that the catalytic activity thereof can be maintained for a long period even at a high concentration.

Further, the organic ammonium salt used in the biological sample treatment agent of the present invention is comprised of the combination of the cation and anion having the hydrogen-bonding functional group(s) (hydroxy group, carboxy group, ether group, hydrogen bonded to nitrogen atom); due to an electrostatic action thereof, a higher retainability of the steric structure of an enzyme as well as a higher retainability of enzyme activity are exhibited as compared to non-ionic compounds having a hydrogen-bonding functional group(s).

Further, the organic ammonium salt used in the biological sample treatment agent of the present invention has a small molecular size and a flexible structure. Thus, as compared to an imidazolium-based organic ammonium salt having a rigid cyclic structure, the organic ammonium salt of the present invention, even when containing a hydroxy group(s), is capable of efficiently entering the inner region of a complex steric structure and protecting the amino acid residues without three-dimensionally distorting the structure, thereby resulting in a higher retainability of enzyme activity.

An oxidoreductase is an enzyme expressing a catalytic action by transfer of hydrogen atoms, transfer of electrons or addition of oxygen atoms from a substrate. Many oxidoreductases express catalytic actions by changes in valence that are caused by electromigration of metal ions in the enzyme.

A transferase is an enzyme catalyzing a reaction for transferring atom groups (functional groups) from one substrate to the other. Since the transfer reaction only involves the functional groups present in the substrate to be reacted, it is particularly critical that there be retained a steric structure for the substrate to be adapted.

A hydrolase is an enzyme for breaking (hydrolyzing) a particular bond(s) in a substrate by reacting the substrate with, for example, water and hydroxy groups in the amino acid residues of the enzyme.

A lyase is an enzyme for breaking a bond such as a carbon-carbon bond and a carbonoxygen bond in a substrate without relying on oxidation or hydrolyzation of substrate molecules. Many lyases break bonds in the substrate molecules by generating intermediates as a result of having metal ions react with the substrate.

An isomerase is an enzyme for converting a substrate into a stereoisomer with a different spatial arrangement. Thus, the steric structures of the amino acid residues in an enzyme for binding the substrate are critical.

A synthetase is an enzyme for binding a substrate to another substrate, utilizing the energy of ATP hydrolysis. The reaction thereof is such that a target substance is to be generated by reacting two substrates via an intermediate with ATP and a particular amino acid residue in the enzyme bonded together.

That is, with regard to each type of enzyme, what is critical are the amino acid residues, the steric structures of the amino acid residues, or the metal ions that have three-dimensionally formed a complex with the amino acid residues; they are responsible for expressing enzyme activities. Due to its structural characteristics, the biocatalyst solvent of the present invention is capable of protecting the amino acid residues or the metal ions that have formed a complex, and thus retaining the activity of a biocatalyst.

Further, the biological sample treatment agent of the present invention is capable of inhibiting denaturation by heat amongst various factors for denaturation such as heat (temperature) and pH. Heat breaks the hydrogen bonds between the amino acid residues so as to cause the steric structures to collapse, and the enzyme to thus denature; the biological sample treatment agent of the present invention is capable of inhibiting heat denaturation by more strongly retaining the steric structures as a result of forming a network of hydrogen bonds between the amino acid residues inside the enzyme and the hydrogen-bonding functional groups in the organic ammonium salt. That is, in the case of the biological sample treatment agent of the present invention, an enzyme can be preserved with its activity being retained for a long period of time at a high enzyme concentration even under a room temperature condition (25°C) which is higher than -20 to 5°C as a general enzyme preservation condition, or under a promotion condition of 40°C at which the enzyme shall be deactivated.

Among biological samples, there are no particular restrictions on a protein; for example, in terms of solution property, there may be listed an acidic protein containing a large amount of amino acids (e.g. aspartic acid, glutamic acid) having carboxy groups, an alkaline protein containing a large amount of amino acids (e.g. lysine, algin, histidine) having amino groups, and a neutral protein well-balanced between these amino acids.

In terms of composition element, there may be listed a simple protein only composed of amino acids, and a complex protein composed in such a manner that it also contains components other than amino acids. Examples of a simple protein include albumin, casein, collagen, keratin, protamine and histone; examples of a complex protein include a glycoprotein (e.g. luteinizing hormone, follicle stimulation hormone, thyroid-stimulating hormone, human chorionic gonadotropin, avidin, cadherin, proteoglycan, mucin), a lipoprotein (e.g. chylomicron, LDL, HDL), a nucleoprotein (e.g. histone proteins, telomerase, protamine), a chromoprotein (e.g. chlorophyll), a metalloprotein (e.g. hemoglobin, cytochrome C), and a phosphoprotein (e.g. casein in milk, vitellin in egg yolk). All enzymes are any of these proteins.

Further, in terms of molecular shape, there may be listed a fibrous protein (e.g. keratin, collagen), and a globular protein (e.g. hemoglobin); in terms of function, there may be listed an enzyme protein (enzyme), a structural protein (e.g. collagen, keratin), a transport protein (e.g. hemoglobin, albumin, apolipoprotein), a storage protein (e.g. ovalbumin contained in egg white, ferritin, hemosiderin), a contractile protein (e.g. actin, myosin), a defensive protein (e.g. globulin), and a regulatory protein (e.g. calmodulin).

In terms of molecular and intermolecular structure, there may be listed those having the primary structure (sequence of amino acids), the secondary structure (a-helix, β-structure, random coil), the tertiary structure (particular spatial arrangement) or the quaternary structure (e.g. hemoglobin, DNA polymerase, ionic channel).

Although not particularly limited, the protein is considered to be that having a molecular weight of 4,000 to 300,000.

Among biological samples, as a nucleic acid, there may be listed, for example, DNA and RNA. These nucleic acids are known to be easily hydrolyzed by their degrading enzymes in water; if using water as a solvent so as to preserve these nucleic acids, it is required that these nucleic acids be dissolved into a water from which the degrading enzymes have already been removed. By using the biological sample treatment agent of the present invention to preserve a nucleic acid, and thus preserving such nucleic acid in the form of a nucleic acid-containing solution, the nucleic acid can be preserved under an environment where the nucleic acid degrading enzymes are deactivated, and a long-term and stable preservation of the nucleic acid is easily achievable due to a non-volatility and a high heat stability.

In view of the aforementioned aspects, if using the organic ammonium salt of the present invention in the biological sample treatment agent, it is particularly preferred that the organic ammonium salt be an organic ammonium salt having a hydrogen-bonding functional group(s) in the anion i.e. an organic ammonium salt having a hydrogen-bonding functional group(s) in both the cation and anion. As the functional group(s) in the anion, there are included hydrogen-bondable groups such as an oxygen-containing group, a nitrogen-containing group, a sulfur-containing group and a phosphorus-containing group; preferred are the abovementioned carboxylic acid anion, sulfur-based anion, phosphorus-based anion, cyano-based anion and nitrogen oxide-based anion.

As the hydrogen-bonding functional group(s) present in the anion, preferred are, for example, a hydroxy group, a carbonyl group, a carboxy group, a carboxylate group, a sulfonyl group, a sulfate ester group, a phosphate group and a phosphate ester group; particularly, more preferred are a hydroxy group, a carboxy group, a carboxylate group, a sulfonyl group and a phosphate group.

Since the organic ammonium salt used in the biological sample treatment agent of the present invention is solid at 25°C as described above, and has a hydrogen-bonding functional group(s) in the cation or in both the cation and anion, it has a high affinity to a biological sample, and is thus superior in preserving a biological sample. If preserving a biological sample by dissolving and dispersing the same in an organic ammonium salt that is liquid at 25°C, there will be imposed restrictions such as a solubility and a uniform dispersion stability of the biological sample itself; if the organic ammonium salt is solid at 25°C, a biological sample can be mixed therewith at a higher concentration and preserved at a higher concentration as well. Further, the biological sample treatment agent of the present invention is capable of preserving a biological sample for a long period of time even under a high-temperature environment; for example, a biological sample can be preserved under a high temperature for a long period of time with the structures of the protein, nucleic acid and enzyme being retained, thereby allowing the activity of the enzyme to be retained.

The hydrogen-bonding material treatment agent of the present invention is capable of, for example, forming hydrogen bonds with the hydrogen bond-accepting functional groups in a carbonyl group or ether group-containing biological sample such as an enzyme, a peptide, a protein, a nucleic acid and a poorly-soluble polysaccharide including cellulose, which allows the organic ammonium salt to enter the complexly intertwined structures of the biological sample and then untangle the biomolecular structure so as to reduce the interactions between the biopolymers, thereby making it possible to activate the inactive and denatured proteins, thus making the hydrogen-bonding material treatment agent of the present invention useful as a protein refolding agent.

Protein refolding is to restore a protein that has been insolubilized or lost a higher-order structure to a natural (activated) protein having a higher-order structure. For example, a protein that has been insolubilized or lost a higher-order structure may be directly solubilized and refolded by the refolding solution containing the organic ammonium salt of the present invention with the aid of a denaturant, if necessary. Alternatively, a protein that has been insolubilized or lost a higher-order structure may at first be solubilized by a general protein solubilizer with the aid of a denaturant, if necessary; a solubilized liquid thus obtained is then dissolved into the refolding solution containing the organic ammonium salt of the present invention to restore the higher-order structure to the protein, thereby obtaining an active protein.

The biological sample solution of the present invention includes the biological sample treatment agent, biological sample and solvent of the present invention. As a biological sample, preferred are a biocatalyst, a protein or a nucleic acid. When used as a biological sample solution, since the organic ammonium salt of the present invention is highly hydrophilic due to its structural characteristics and thus has a high affinity to a biological sample, the organic ammonium salt of the present invention can be used not only in the form of a solid alone, but also in the form of a solution or dispersion liquid prepared by mixing the organic ammonium salt with other solvent components such as water and a polar solvent as described above. Further, an additive(s) may also be added thereto before use.

The biological sample solution may, for example, include a solution containing an activated biological sample; and a solution for preserving a biological sample while retaining its activated state.

Although depending on the type or the like of a biocatalyst, a biocatalyst as an activated biological sample can be preserved for, for example, 30 days or longer, or even 60 days or longer.

As for a preservation temperature of a biocatalyst, the biocatalyst solution of the present invention can be preserved under a severe condition such as a high-temperature and humidity condition; for example, under a temperature of not higher than 40°C, a biocatalyst can be preserved in the form of a liquid and with the activity thereof being retained for a long period of time.

### WORKING EXAMPLES

The present invention is described in greater detail hereunder with reference to working examples; the present invention shall not be limited to these working examples.

Compounds of working examples 1 to 108 shown in Tables 1 to 6 were synthesized as follows.

### <Working example 1> Synthesis of compound 1

A compound represented by the following formula was synthesized.

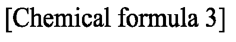

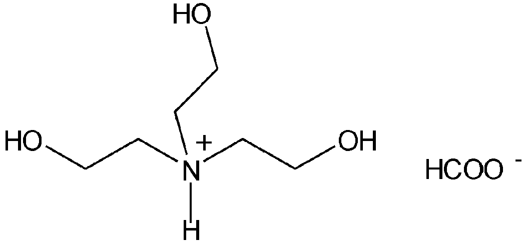

Triethanolamine (19.11 g, 0.128 mol) and formic acid (5.89 g, 0.128 mmol) were reacted in 100 mL of water under room temperature for three hours, followed by distilling away water under a reduced pressure to obtain a light yellow solid. By washing the solid thus obtained, a white solid as a compound 1 (triethanolamine formate) was obtained.

FT-IR(KBr): 3,360 cm⁻¹: O-H stretching vibration 2,950 cm⁻¹: C-H stretching vibration 1,558 cm⁻¹: COO⁻ stretching vibration

¹H-NMR (D₂O 400 MHz): δ 3.31(t, 6H, N⁺CH₂CH₂OH), δ 3.85 (t, 6H, N⁺CH₂CH₂OH), δ 8.36 (s, 1H, HCOO⁻).

¹³C-NMR (D₂O 100 MHz): δ 55.4 (N⁺CH₂CH₂OH), δ 55.6 (N⁺CH₂CH₂OH), δ 171.0 (HCOO⁻).

### <Working examples 2 to 87>

Compounds 2 to 87 of the working examples 2 to 87 shown in Tables 1 to 5 were synthesized by a synthesis method similar to that of the working example 1, and at compounding molar ratios shown in Tables 7 to 9. Property values are shown below.

### <Working example 2> Synthesis of compound 2

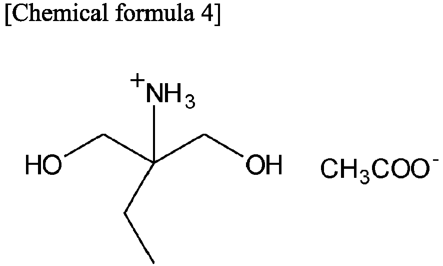

FT-IR(KBr): 3,388 cm⁻¹: O-H stretching vibration 2,922 cm⁻¹: C-H stretching vibration 1,543 cm⁻¹: COO⁻ stretching vibration

¹H-NMR (D₂O 400 MHz):δ 0.73-0.77 (m, 3H, NH₃⁺C(CH₂OH)₂CH₂CH₃), δ 1.50-1.56 (m, 2H, NH₃⁺C(CH₂OH)₂CH₂CH₃), δ 1.84 (s, 3H, CH₃COO⁻), δ 3.49-3.50 (m, 4H, NH₃⁺C(CH₂OH)₂CH₂CH₃).

¹³C-NMR (D₂O 100 MHz): δ 6.3 (NH₃⁺C(CH₂OH)₂CH₂CH₃), δ 23.2 (NH₃⁺C(CH₂OH)₂CH₂CH₃), δ 23.3 (CH₃COO⁻), δ 60.5 (NH₃⁺C(CH₂OH)₂CH₂CH₃), δ 63.3 (NH₃⁺C(CH₂OH)₂CH₂CH₃), δ 181.4 (CH₃COO⁻).

### <Working example 3> Synthesis of compound 3

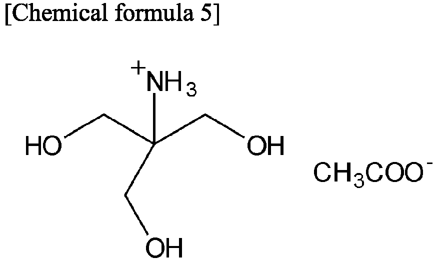

FT-IR(KBr): 3,145 cm⁻¹: O-H stretching vibration 2,946 cm⁻¹: C-H stretching vibration 1,572 cm⁻¹: COO⁻ stretching vibration

¹H-NMR (D₂O 400 MHz): δ 1.84 (s, 3H, CH₃COO⁻), δ 3.57 (s, 6H, NH₃⁺C(CH₂OH)₃). ¹³C-NMR (D₂O 100 MHz): δ 23.3 (CH₃COO⁻), δ 59.2 (NH₃⁺C(CH₂OH)₃), δ 61.4 (NH₃⁺C(CH₂OH)₃), δ 181.4 (CH₃COO⁻).

### <Working example 4> Synthesis of compound 4

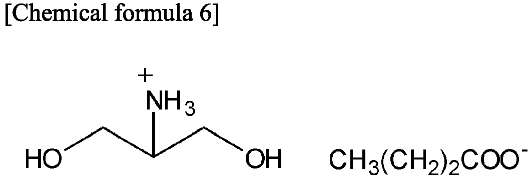

FT-IR(KBr): 3,375 cm⁻¹: O-H stretching vibration 2,955 cm⁻¹: C-H stretching vibration 1,576 cm⁻¹: COO⁻ stretching vibration

¹H-NMR (D₂O 400 MHz): δ 0.80 (t, 3H, CH₃CH₂), δ 1.44 (m, 2H, CH₃CH₂), δ 2.06 (t, 2H, CH₂COO⁻), δ 3.32-3.38 (m, 1H, HOCH₂CH(N⁺H₃)CH₂OH), δ 3.61-3.77 (m, 4H, HOCH₂CH(N⁺H₃)CH₂OH).

¹³C-NMR (D₂O 100 MHz): δ 13.2 (CH₃CH₂), δ 19.3 (CH₃CH₂), δ 39.6 (CH₂COO⁻), δ 54.1 (HOCH₂CH(N⁺H₃)CH₂OH), δ 58.6 (HOCH₂CH(N⁺H₃)CH₂OH), δ 184.1 (CH₂COO⁻).

### <Working example 5> Synthesis of compound 5

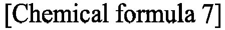

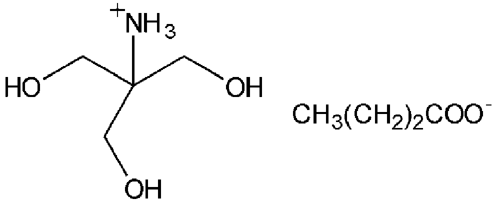

FT-IR(KBr): 3,145 cm⁻¹: O-H stretching vibration 2,946 cm⁻¹: C-H stretching vibration 1,572 cm⁻¹: COO⁻ stretching vibration

¹H-NMR (D₂O 400 MHz): δ 0.80 (t, 3H, CH₃CH₂), δ 1.44 (m, 2H, CH₃CH₂), δ 2.06 (t, 2H, CH₂COO⁻), δ 3.57 (s, 6H, NH₃⁺C(CH₂OH)₃).

¹³C-NMR (D₂O 100 MHz): δ 13.2 (CH₃CH₂), δ 19.3 (CH₃CH₂), δ 39.6 (CH₂COO⁻), δ 59.2 (NH₃⁺C(CH₂OH)₃), δ 61.4 (NH₃⁺C(CH₂OH)₃), δ 184.1 (CH₂COO⁻).

### <Working example 6> Synthesis of compound 6

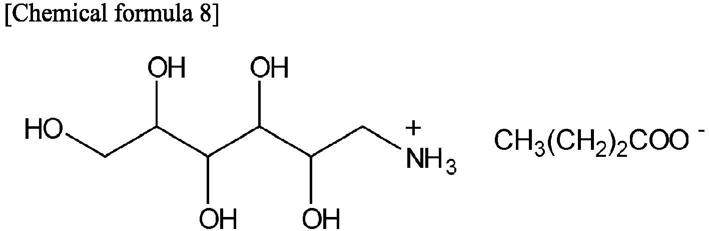

FT-IR(KBr): 3,167 cm⁻¹: O-H stretching vibration 2,920 cm⁻¹: C-H stretching vibration 1,573 cm⁻¹: COO⁻ stretching vibration

¹H-NMR (D₂O 400 MHz): δ 0.80 (t, 3H, CH₃CH₂), δ 1.44 (m, 2H, CH₃CH₂), δ 2.06 (t, 2H, CH₂COO⁻), δ 2.94-3.13 (m, 2H, HOCH₂(CH(OH))₃CH(OH)CH₂NH₃⁺), δ 3.51-3.72 (m, 5H, HOCH₂(CH(OH))₃CH(OH)CH₂NH₃⁺), δ 3.89-3.96 (m, 1H, HOCH₂(CH(OH))₃CH(OH)CH₂NH₃⁺).

¹³C-NMR (D₂O 100 MHz): δ 13.2 (CH₃CH₂), δ 19.3 (CH₃CH₂), δ 39.6 (CH₂COO⁻), δ 41.7 (HOCH₂(CH(OH))₃CH(OH)CH₂NH₃⁺),δ 62.6 (HOCH₂(CH(OH))₃CH(OH)CH₂NH₃⁺), δ 68.9-70.9 (HOCH₂(CH(OH))₃CH(OH)CH₂NH₃⁺), δ 184.1 (CH₂COO⁻).

### <Working example 7> Synthesis of compound 7

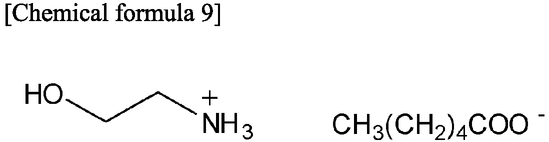

FT-IR(KBr): 3,177 cm⁻¹: O-H stretching vibration 2,950 cm⁻¹: C-H stretching vibration 1,563 cm⁻¹: COO⁻ stretching vibration

¹H-NMR (D₂O 400 MHz): δ 0.80 (t, 3H, CH₃CH₂), δ 1.17 (m, 4H, CH₃CH₂CH₂), δ 1.44 (m, 2H, CH₂CH₂COO⁻), δ 2.10 (t, 2H, CH₂COO⁻), δ 3.04 (m, 2H, N⁺CH₂CH₂OH), δ 3.77 (m, 2H, N⁺CH₂CH₂OH).

¹³C-NMR (D₂O 100 MHz): δ 13.3 (CH₃CH₂), δ 21.8 (CH₃CH₂), δ 25.5 (CH₃CH₂CH₂), δ 31.0 (CH₂CH₂COO⁻), δ 37.5 (CH₂COO⁻), δ 41.2 (N⁺CH₂CH₂OH), δ 57.6 (N⁺CH₂CH₂OH), δ 184.2 (CH₂COO⁻).

### <Working example 8> Synthesis of compound 8

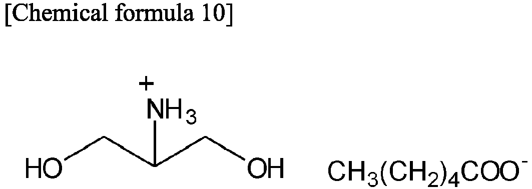

FT-IR(KBr): 3,375 cm⁻¹: O-H stretching vibration 2,955 cm⁻¹: C-H stretching vibration 1,576 cm⁻¹: COO⁻ stretching vibration

¹H-NMR (D₂O 400 MHz): δ 0.80 (t, 3H, CH₃CH₂), δ 1.17 (m, 4H, CH₃CH₂CH₂), δ 1.44 (m, 2H, CH₂CH₂COO⁻), δ 2.10 (t, 2H, CH₂COO⁻), δ 3.32-3.38 (m, 1H, HOCH₂CH(N⁺H₃)CH₂OH), δ 3.61-3.77 (m, 4H, HOCH₂CH(N⁺H₃)CH₂OH).

¹³C-NMR (D₂O 100 MHz): 13.3 (CH₃CH₂), δ 21.8 (CH₃CH₂), δ 25.5 (CH₃CH₂CH₂), δ 31.0 (CH₂CH₂COO⁻), δ 37.5 (CH₂COO⁻), δ 54.1 (HOCH₂CH(N⁺H₃)CH₂OH), δ 58.6 (HOCH₂CH(N⁺H₃)CH₂OH), δ 184.2 (CH₂COO⁻).

### <Working example 9> Synthesis of compound 9

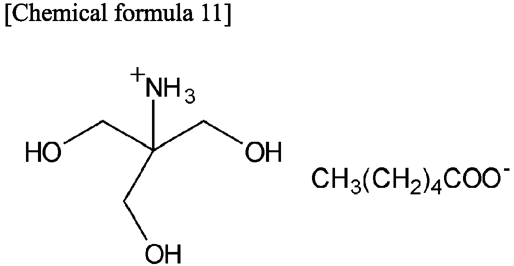

FT-IR(KBr): 3,145 cm⁻¹: O-H stretching vibration 2,946 cm⁻¹: C-H stretching vibration 1,572 cm⁻¹: COO⁻ stretching vibration

¹H-NMR (D₂O 400 MHz): δ 0.80 (t, 3H, CH₃CH₂), δ 1.17 (m, 4H, CH₃CH₂CH₂), δ 1.44 (m, 2H, CH₂CH₂COO⁻), δ 2.10 (t, 2H, CH₂COO⁻), δ 3.57 (s, 6H, NH₃⁺C(CH₂OH)₃).

¹³C-NMR (D₂O 100 MHz): δ 13.3 (CH₃CH₂), δ 21.8 (CH₃CH₂), δ 25.5 (CH₃CH₂CH₂), δ 31.0 (CH₂CH₂COO⁻), δ 37.5 (CH₂COO⁻), δ 59.2 (NH₃⁺C(CH₂OH)₃), δ 61.4 (NH₃⁺C(CH₂OH)₃), δ 184.2 (CH₂COO⁻).

### <Working example 10> Synthesis of compound 10

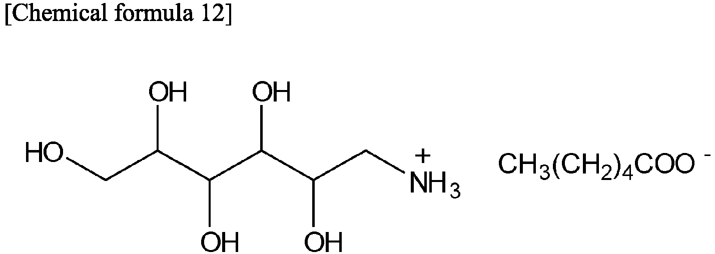

FT-IR(KBr): 3,167 cm⁻¹: O-H stretching vibration 2,920 cm⁻¹: C-H stretching vibration 1,573 cm⁻¹: COO⁻ stretching vibration

¹H-NMR (D₂O 400 MHz): δ 0.80 (t, 3H, CH₃CH₂), δ 1.17 (m, 4H, CH₃CH₂CH₂), δ 1.44 (m, 2H, CH₂CH₂COO⁻), δ 2.10 (t, 2H, CH₂COO⁻), δ 2.94-3.13 (m, 2H, HOCH₂(CH(OH))₃CH(OH)CH₂NH₃⁺), δ 3.51-3.72 (m, 5H, HOCH₂(CH(OH))₃CH(OH)CH₂NH₃⁺), δ 3.89-3.96 (m, 1H, HOCH₂(CH(OH))₃CH(OH)CH₂NH₃⁺).

¹³C-NMR (D₂O 100 MHz): δ 13.3 (CH₃CH₂), δ 21.8 (CH₃CH₂), δ 25.5 (CH₃CH₂CH₂), δ 31.0 (CH₂CH₂COO⁻), δ 37.5 (CH₂COO⁻), δ 41.7 (HOCH₂(CH(OH))₃CH(OH)CH₂NH₃⁺), δ 62.6 (HOCH₂(CH(OH))₃CH(OH)CH₂NH₃⁺), δ 68.9-70.9 (HOCH₂(CH(OH))₃CH(OH)CH₂NH₃⁺), δ 184.2 (CH₂COO⁻).

### <Working example 11> Synthesis of compound 11

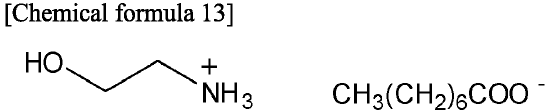

FT-IR(KBr): 3,177 cm⁻¹: O-H stretching vibration 2,950 cm⁻¹: C-H stretching vibration 1,563 cm⁻¹: COO⁻ stretching vibration

¹H-NMR (D₂O 400 MHz): δ 0.79 (t, 3H, CH₃CH₂), δ 1.21 (m, 8H, CH₃(CH₂)₄), δ 1.48 (m, 2H, CH₂CH₂COO⁻), δ 2.09 (m, 2H, CH₂COO⁻), δ 3.04 (m, 2H, N⁺CH₂CH₂OH), δ 3.77 (m, 2H, N⁺CH₂CH₂OH).

¹³C-NMR (D₂O 100 MHz): δ 13.4 (CH₃CH₂), δ 22.0 (CH₃CH₂CH₂), δ 25.9 (CH₃CH₂CH₂), δ 28.3 (CH₃CH₂CH₂CH₂), δ 28.7 (CH₂CH₂CH₂COO⁻), δ 31.0 (CH₂CH₂COO⁻), δ 37.7 (CH₂COO⁻), δ 41.2 (N⁺CH₂CH₂OH), δ 57.6 (N⁺CH₂CH₂OH), δ 184.3 (CH₂COO⁻).

### <Working example 12> Synthesis of compound 12

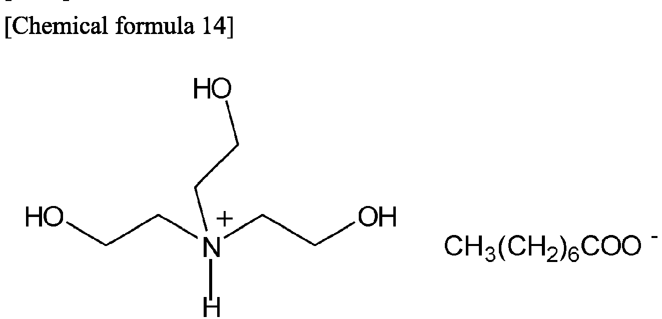

FT-IR(KBr): 3,360 cm⁻¹: O-H stretching vibration 2,950 cm⁻¹: C-H stretching vibration 1,558 cm⁻¹: COO⁻ stretching vibration

¹H-NMR (D₂O 400 MHz): δ 0.79 (t, 3H, CH₃CH₂), δ 1.21 (m, 8H, CH₃(CH₂)₄), δ 1.48 (m, 2H, CH₂CH₂COO⁻), δ 2.09 (m, 2H, CH₂COO⁻), δ 3.31(t, 6H, N⁺CH₂CH₂OH), δ 3.85 (t, 6H, N⁺CH₂CH₂OH).

¹³C-NMR (D₂O 100 MHz): δ 13.4 (CH₃CH₂), δ 22.0 (CH₃CH₂CH₂), δ 25.9 (CH₃CH₂CH₂), δ 28.3 (CH₃CH₂CH₂CH₂), δ 28.7 (CH₂CH₂CH₂COO⁻), δ 31.0 (CH₂CH₂COO⁻), δ 37.7 (CH₂COO⁻), δ 55.4 (N⁺CH₂CH₂OH), δ 55.6 (N⁺CH₂CH₂OH), δ 184.3 (CH₂COO⁻).

### <Working example 13> Synthesis of compound 13

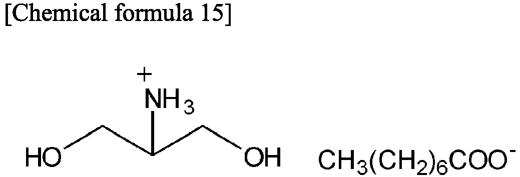

FT-IR(KBr): 3,375 cm⁻¹: O-H stretching vibration 2,955 cm⁻¹: C-H stretching vibration 1,576 cm⁻¹: COO⁻ stretching vibration

¹H-NMR (D₂O 400 MHz): δ 0.79 (t, 3H, CH₃CH₂), δ 1.21 (m, 8H, CH₃(CH₂)₄), δ 1.48 (m, 2H, CH₂CH₂COO⁻), δ 2.09 (m, 2H, CH₂COO⁻), δ 3.32-3.38 (m, 1H, HOCH₂CH(N⁺H₃)CH₂OH), δ 3.61-3.77 (m, 4H, HOCH₂CH(N⁺H₃)CH₂OH).

¹³C-NMR (D₂O 100 MHz): δ 13.4 (CH₃CH₂), δ 22.0 (CH₃CH₂CH₂), δ 25.9 (CH₃CH₂CH₂), δ 28.3 (CH₃CH₂CH₂CH₂), δ 28.7 (CH₂CH₂CH₂COO⁻), δ 31.0 (CH₂CH₂COO⁻), δ 37.7 (CH₂COO⁻), δ 54.1 (HOCH₂CH(N⁺H₃)CH₂OH), δ 58.6 (HOCH₂CH(N⁺H₃)CH₂OH), δ 184.3 (CH₂COO⁻).

### <Working example 14> Synthesis of compound 14

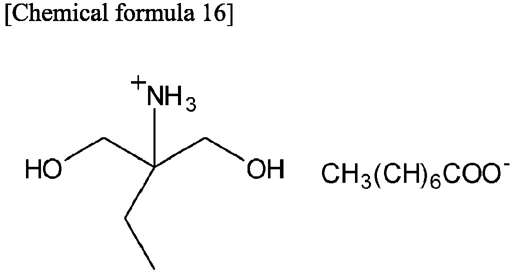

FT-IR(KBr): 3,388 cm⁻¹: O-H stretching vibration 2,922 cm⁻¹: C-H stretching vibration 1,543 cm⁻¹: COO⁻ stretching vibration

¹H-NMR (D₂O 400 MHz): δ 0.73-0.77 (m, 3H, NH₃⁺C(CH₂OH)₂CH₂CH₃), δ 0.79 (t, 3H, CH₃CH₂), δ 1.21 (m, 8H, CH₃(CH₂)₄), δ 1.50-1.56 (m, 2H, NH₃⁺C(CH₂OH)₂CH₂CH₃), δ 1.48 (m, 2H, CH₂CH₂COO⁻), δ 2.09 (m, 2H, CH₂COO⁻), δ 3.49-3.50 (m, 4H, NH₃⁺C(CH₂OH)₂CH₂CH₃).

¹³C-NMR (D₂O 100 MHz): δ 6.3 (NH₃⁺C(CH₂OH)₂CH₂CH₃), δ 13.4 (CH₃CH₂), δ 22.0 (CH₃CH₂CH₂), δ 23.2 (NH₃⁺C(CH₂OH)₂CH₂CH₃), δ 25.9 (CH₃CH₂CH₂), δ 28.3 (CH₃CH₂CH₂CH₂), δ 28.7 (CH₂CH₂CH₂COO⁻), δ 31.0 (CH₂CH₂COO⁻), δ 37.7 (CH₂COO⁻), δ 60.5 (NH₃⁺C(CH₂OH)₂CH₂CH₃), δ 63.3 (NH₃⁺C(CH₂OH)₂CH₂CH₃), δ 184.3 (CH₂COO⁻).

### <Working example 15> Synthesis of compound 15

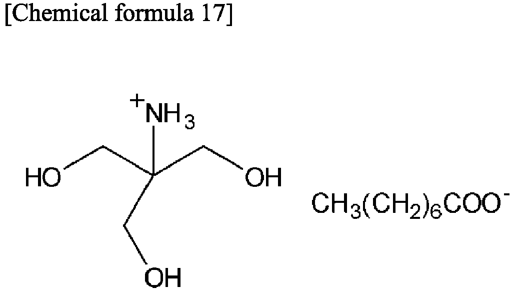

FT-IR(KBr): 3,145 cm⁻¹: O-H stretching vibration 2,946 cm⁻¹: C-H stretching vibration 1,572 cm⁻¹: COO⁻ stretching vibration

¹H-NMR (D₂O 400 MHz): δ 0.79 (t, 3H, CH₃CH₂), δ 1.21 (m, 8H, CH₃(CH₂)₄), δ 1.48 (m, 2H, CH₂CH₂COO⁻), δ 2.09 (m, 2H, CH₂COO⁻), δ 3.57 (s, 6H, NH₃⁺C(CH₂OH)₃).

¹³C-NMR (D₂O 100 MHz): δ 13.4 (CH₃CH₂), δ 22.0 (CH₃CH₂CH₂), δ 25.9 (CH₃CH₂CH₂), δ 28.3 (CH₃CH₂CH₂CH₂), δ 28.7 (CH₂CH₂CH₂COO⁻), δ 31.0 (CH₂CH₂COO⁻), δ 37.7 (CH₂COO⁻), δ 59.2 (NH₃⁺C(CH₂OH)₃), δ 61.4 (NH₃⁺C(CH₂OH)₃), δ 184.3 (CH₂COO⁻).

### <Working example 16> Synthesis of compound 16

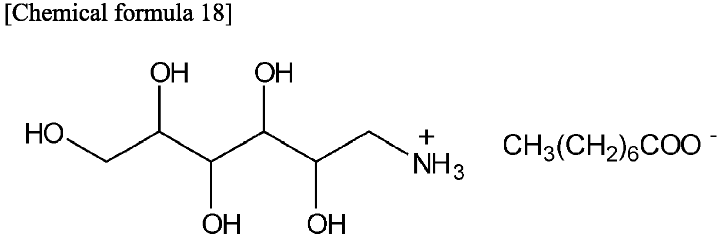

FT-IR (KBr): 3,167 cm⁻¹: O-H stretching vibration 2,920 cm⁻¹: C-H stretching vibration 1,573 cm⁻¹: COO⁻ stretching vibration

¹H-NMR (D₂O 400 MHz): δ 0.79 (t, 3H, CH₃CH₂), δ 1.21 (m, 8H, CH₃(CH₂)₄), δ 1.48 (m, 2H, CH₂CH₂COO⁻), δ 2.09 (m, 2H, CH₂COO⁻), δ 2.94-3.13 (m, 2H, HOCH₂(CH(OH))₃CH(OH)CH₂NH₃⁺), δ 3.51-3.72 (m, 5H, HOCH₂(CH(OH))₃CH(OH)CH₂NH₃⁺), δ 3.89-3.96 (m, 1H, HOCH₂(CH(OH))₃CH(OH)CH₂NH₃⁺).

¹³C-NMR (D₂O 100 MHz): δ 13.4 (CH₃CH₂), δ 22.0 (CH₃CH₂CH₂), δ 25.9 (CH₃CH₂CH₂), δ 28.3 (CH₃CH₂CH₂CH₂), δ 28.7 (CH₂CH₂CH₂COO⁻), δ 31.0 (CH₂CH₂COO⁻), δ 37.7 (CH₂COO⁻), δ 41.7 (HOCH₂(CH(OH))₃CH(OH)CH₂NH₃⁺), δ 62.6 (HOCH₂(CH(OH))₃CH(OH)CH₂NH₃⁺), δ 68.9-70.9 (HOCH₂(CH(OH))3CH(OH)CH₂NH₃⁺), δ 184.3 (CH₂COO⁻).

### <Working example 17> Synthesis of compound 17

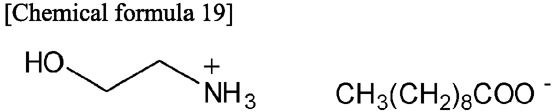

FT-IR(KBr): 3,177 cm⁻¹: O-H stretching vibration 2,950 cm⁻¹: C-H stretching vibration 1,563 cm⁻¹: COO⁻ stretching vibration

¹H-NMR (D₂O 400 MHz): δ 0.79 (t, 3H, CH₃CH₂), δ 1.21 (m, 12H, CH₃(CH₂)₆), δ 1.47 (m, 2H, CH₂CH₂COO⁻), δ 2.10 (t, 2H, CH₂COO⁻), δ 3.04 (m, 2H, N⁺CH₂CH₂OH), δ 3.77 (m, 2H, N⁺CH₂CH₂OH).

¹³C-NMR (D₂O 100 MHz): δ 13.5 (CH₃CH₂), δ 22.2 (CH₃CH₂CH₂), δ 26.0 (CH₃CH₂CH₂), δ 28.7 (CH₃CH₂CH₂CH₂), δ 28.8 (CH₃CH₂CH₂CH₂CH₂), δ 29.0 ((CH₂)₂CH₂CH₂COO⁻), δ 31.4 (CH₂CH₂COO⁻), δ 37.5 (CH₂COO⁻), δ 41.2 (N⁺CH₂CH₂OH), δ 57.6 (N⁺CH₂CH₂OH), δ 183.6 (CH₂COO⁻).

### <Working example 18> Synthesis of compound 18

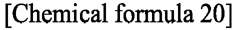

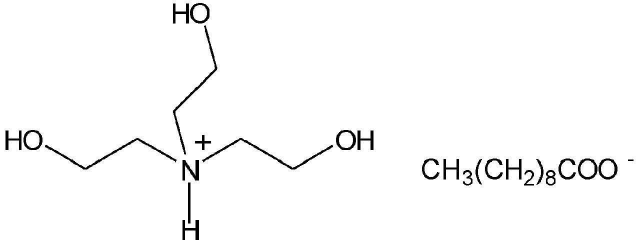

FT-IR(KBr): 3,360 cm⁻¹: O-H stretching vibration 2,950 cm⁻¹: C-H stretching vibration 1,558 cm⁻¹: COO⁻ stretching vibration

¹H-NMR (D₂O 400 MHz): δ 0.79 (t, 3H, CH₃CH₂), δ 1.21 (m, 12H, CH₃(CH₂)₆), δ 1.47 (m, 2H, CH₂CH₂COO⁻), δ 2.10 (t, 2H, CH₂COO⁻), δ 3.31(t, 6H, N⁺CH₂CH₂OH), δ 3.85 (t, 6H, N⁺CH₂CH₂OH).

¹³C-NMR (D₂O 100 MHz): δ 13.5 (CH₃CH₂), δ 22.2 (CH₃CH₂CH₂), δ 26.0 (CH₃CH₂CH₂), δ 28.7 (CH₃CH₂CH₂CH₂), δ 28.8 (CH₃CH₂CH₂CH₂CH₂), δ 29.0 ((CH₂)₂CH₂CH₂COO⁻), δ 31.4 (CH₂CH₂COO⁻), δ 37.5 (CH₂COO⁻), δ 55.4 (N⁺CH₂CH₂OH), δ 55.6 (N⁺CH₂CH₂OH), δ 183.6 (CH₂COO⁻).

### <Working example 19> Synthesis of compound 19

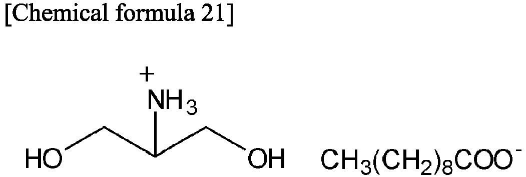

FT-IR(KBr): 3,375 cm⁻¹: O-H stretching vibration 2,955 cm⁻¹: C-H stretching vibration 1,576 cm⁻¹: COO⁻ stretching vibration

¹H-NMR (D₂O 400 MHz): δ 0.79 (t, 3H, CH₃CH₂), δ 1.21 (m, 12H, CH₃(CH₂)₆), δ 1.47 (m, 2H, CH₂CH₂COO⁻), δ 2.10 (t, 2H, CH₂COO⁻), δ 3.32-3.38 (m, 1H, HOCH₂CH(N⁺H₃)CH₂OH), δ 3.61-3.77 (m, 4H, HOCH₂CH(N⁺H₃)CH₂OH).

¹³C-NMR (D₂O 100 MHz): δ 13.5 (CH₃CH₂), δ 22.2 (CH₃CH₂CH₂), δ 26.0 (CH₃CH₂CH₂), δ 28.7 (CH₃CH₂CH₂CH₂), δ 28.8 (CH₃CH₂CH₂CH₂CH₂), δ 29.0 ((CH₂)₂CH₂CH₂COO⁻), δ 31.4 (CH₂CH₂COO⁻), δ 37.5 (CH₂COO⁻), δ 54.1 (HOCH₂CH(N⁺H₃)CH₂OH), δ 58.6 (HOCH₂CH(N⁺H₃)CH₂OH), δ 183.6 (CH₂COO⁻).

### <Working example 20> Synthesis of compound 20

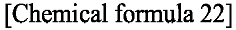

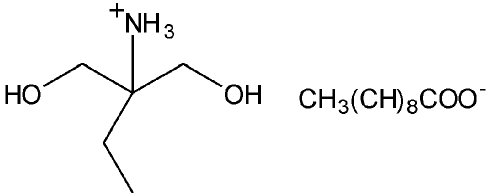

FT-IR(KBr): 3,388 cm⁻¹: O-H stretching vibration 2,922 cm⁻¹: C-H stretching vibration 1,543 cm⁻¹: COO⁻ stretching vibration

¹H-NMR (D₂O 400 MHz): δ 0.73-0.77 (m, 3H, NH₃⁺C(CH₂OH)₂CH₂CH₃), δ 0.79 (t, 3H, CH₃CH₂), δ 1.21 (m, 12H, CH₃(CH₂)₆), δ 1.47 (m, 2H, CH₂CH₂COO⁻), δ 1.50-1.56 (m, 2H, NH₃⁺C(CH₂OH)₂CH₂CH₃), δ 2.10 (t, 2H, CH₂COO⁻), δ 3.49-3.50 (m, 4H, NH₃⁺C(CH₂OH)₂CH₂CH₃).

¹³C-NMR (D₂O 100 MHz): δ 6.3 (NH₃⁺C(CH₂OH)₂CH₂CH₃), δ 13.5 (CH₃CH₂), δ 22.2 (CH₃CH₂CH₂), δ 23.2 (NH₃⁺C(CH₂OH)₂CH₂CH₃), δ 26.0 (CH₃CH₂CH₂), δ 28.7 (CH₃CH₂CH₂CH₂), δ 28.8 (CH₃CH₂CH₂CH₂CH₂), δ 29.0 ((CH₂)₂CH₂CH₂COO⁻), δ 31.4 (CH₂CH₂COO⁻), δ 37.5 (CH₂COO⁻), δ 60.5 (NH₃C(CH₂OH)₂CH₂CH₃), δ 63.3 (NH₃⁺C(CH₂OH)₂CH₂CH₃), δ 183.6 (CH₂COO⁻).

### <Working example 21> Synthesis of compound 21

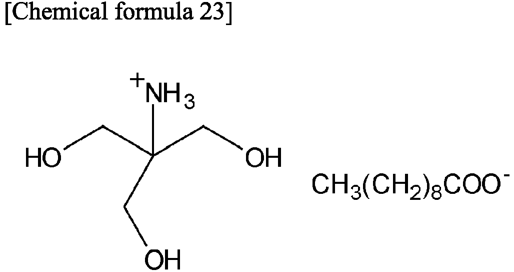

FT-IR(KBr): 3,145 cm⁻¹: O-H stretching vibration 2,946 cm⁻¹: C-H stretching vibration 1,572 cm⁻¹: COO⁻ stretching vibration

¹H-NMR (D₂O 400 MHz): δ 0.79 (t, 3H, CH₃CH₂), δ 1.21 (m, 12H, CH₃(CH₂)₆), δ 1.47 (m, 2H, CH₂CH₂COO⁻), δ 2.10 (t, 2H, CH₂COO⁻), δ 3.57 (s, 6H, NH₃⁺C(CH₂OH)₃).

¹³C-NMR (D₂O 100 MHz): δ 13.5 (CH₃CH₂), δ 22.2 (CH₃CH₂CH₂), δ 26.0 (CH₃CH₂CH₂), δ 28.7 (CH₃CH₂CH₂CH₂), δ 28.8 (CH₃CH₂CH₂CH₂CH₂), δ 29.0 ((CH₂)₂CH₂CH₂COO⁻), δ 31.4 (CH₂CH₂COO⁻), δ 37.5 (CH₂COO⁻), δ 59.2 (NH₃⁺C(CH₂OH)₃), δ 61.4 (NH₃⁺C(CH₂OH)₃), δ 183.6 (CH₂COO⁻).

### <Working example 22> Synthesis of compound 22

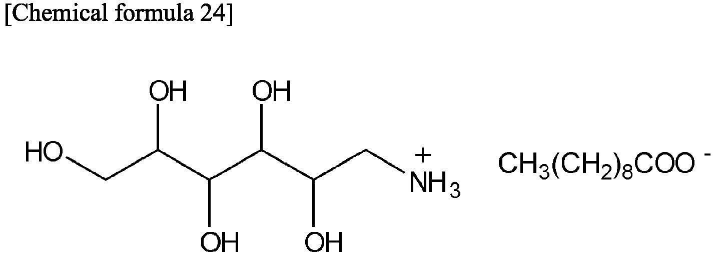

FT-IR(KBr): 3,167 cm⁻¹: O-H stretching vibration 2,920 cm⁻¹: C-H stretching vibration 1,573 cm⁻¹: COO⁻ stretching vibration

¹H-NMR (D₂O 400 MHz): δ 0.79 (t, 3H, CH₃CH₂), δ 1.21 (m, 12H, CH₃(CH₂)₆), δ 1.47 (m, 2H, CH₂CH₂COO⁻), δ 2.10 (t, 2H, CH₂COO⁻), δ 2.94-3.13 (m, 2H, HOCH₂(CH(OH))₃CH(OH)CH₂NH₃⁺), δ 3.51-3.72 (m, 5H, HOCH₂(CH(OH))₃CH(OH)CH₂NH₃⁺), δ 3.89-3.96 (m, 1H, HOCH₂(CH(OH))₃CH(OH)CH₂NH₃⁺).

¹³C-NMR (D₂O 100 MHz): δ 13.5 (CH₃CH₂), δ 22.2 (CH₃CH₂CH₂), δ 26.0 (CH₃CH₂CH₂), δ 28.7 (CH₃CH₂CH₂CH₂), δ 28.8 (CH₃CH₂CH₂CH₂CH₂), δ 29.0 ((CH₂)₂CH₂CH₂COO⁻), δ 31.4 (CH₂CH₂COO⁻), δ 37.5 (CH₂COO⁻), δ 41.7 (HOCH₂(CH(OH))₃CH(OH)CH₂NH₃⁺), δ 62.6 (HOCH₂(CH(OH))₃CH(OH)CH₂NH₃⁺), δ 68.9-70.9 (HOCH₂(CH(OH))₃CH(OH)CH₂NH₃⁺), δ 183.6 (CH₂COO⁻).

### <Working example 23> Synthesis of compound 23

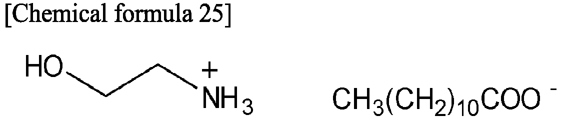

FT-IR(KBr): 3,177 cm⁻¹: O-H stretching vibration 2,950 cm⁻¹: C-H stretching vibration 1,563 cm⁻¹: COO⁻ stretching vibration

¹H-NMR (D₂O 400 MHz): δ 0.79 (t, 3H, CH₃CH₂), δ 1.21 (m, 16H, CH₃(CH₂)₈), δ 1.47 (m, 2H, CH₂CH₂COO⁻), δ 2.10 (t, 2H, CH₂COO⁻), δ 3.04 (m, 2H, NCH₂CH₂OH), δ 3.77 (m, 2H, N⁺CH₂CH₂OH).

¹³C-NMR (D₂O 100 MHz): δ 13.5 (CH₃CH₂), δ 22.2 (CH₃CH₂CH₂), δ 26.0 (CH₃CH₂CH₂), δ 28.7 (CH₃CH₂CH₂CH₂), δ 28.8 (CH₃CH₂CH₂CH₂CH₂), δ 29.0 ((CH₂)₄CH₂CH₂COO⁻), δ 31.4 (CH₂CH₂COO⁻), δ 37.5 (CH₂COO⁻), δ 41.2 (N⁺CH₂CH₂OH), δ 57.6 (N⁺CH₂CH₂OH), δ 183.6 (CH₂COO⁻).

### <Working example 24> Synthesis of compound 24

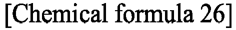

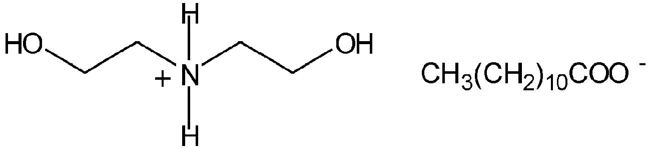

FT-IR(KBr): 3,306 cm⁻¹: O-H stretching vibration 2,923 cm⁻¹: C-H stretching vibration 1,559 cm⁻¹: COO⁻ stretching vibration

¹H-NMR (D₂O 400 MHz): δ 0.79 (t, 3H, CH₃CH₂), δ 1.21 (m, 16H, CH₃(CH₂)₈), δ 1.47 (m, 2H, CH₂CH₂COO⁻), δ 2.10 (t, 2H, CH₂COO⁻), δ 3.15 (t, 4H, N⁺CH₂CH₂OH), δ 3.79 (t, 4H, N⁺CH₂CH₂OH).

¹³C-NMR (D₂O 100 MHz): δ 13.5 (CH₃CH₂), δ 22.2 (CH₃CH₂CH₂), δ 26.0 (CH₃CH₂CH₂), δ 28.7 (CH₃CH₂CH₂CH₂), δ 28.8 (CH₃CH₂CH₂CH₂CH₂), δ 29.0 ((CH₂)₄CH₂CH₂COO⁻), δ 31.4 (CH₂CH₂COO⁻), δ 37.5 (CH₂COO⁻), δ 48.9 (N⁺CH₂CH₂OH), δ 56.6 (N⁺CH₂CH₂OH), δ 183.6 (CH₂COO⁻).

### <Working example 25> Synthesis of compound 25

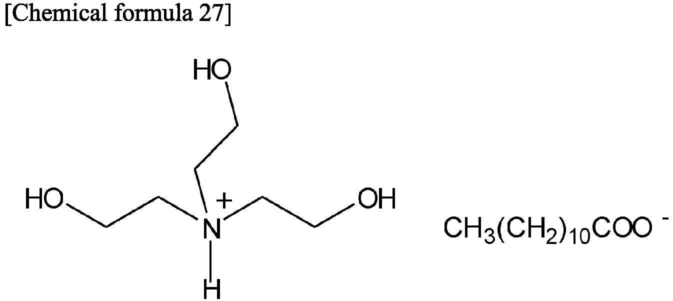

FT-IR(KBr): 3,360 cm⁻¹: O-H stretching vibration 2,950 cm⁻¹: C-H stretching vibration 1,558 cm⁻¹: COO⁻ stretching vibration

¹H-NMR (D₂O 400 MHz): δ 0.79 (t, 3H, CH₃CH₂), δ 1.21 (m, 16H, CH₃(CH₂)₈), δ 1.47 (m, 2H, CH₂CH₂COO⁻), δ 2.10 (t, 2H, CH₂COO⁻), δ 3.31(t, 6H, N⁺CH₂CH₂OH), δ 3.85 (t, 6H, N⁺CH₂CH₂OH).

¹³C-NMR (D₂O 100 MHz): δ 13.5 (CH₃CH₂), δ 22.2 (CH₃CH₂CH₂), δ 26.0 (CH₃CH₂CH₂), δ 28.7 (CH₃CH₂CH₂CH₂), δ 28.8 (CH₃CH₂CH₂CH₂CH₂), δ 29.0 ((CH₂)₄CH₂CH₂COO⁻), δ 31.4 (CH₂CH₂COO⁻), δ 37.5 (CH₂COO⁻), δ 55.4 (N⁺CH₂CH₂OH), δ 55.6 (N⁺CH₂CH₂OH), δ 183.6 (CH₂COO⁻).

### <Working example 26> Synthesis of compound 26

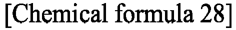

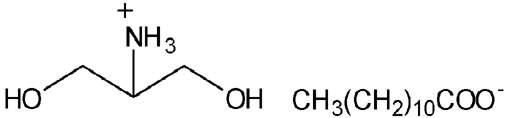

FT-IR(KBr): 3,375 cm⁻¹: O-H stretching vibration 2,955 cm⁻¹: C-H stretching vibration 1,576 cm⁻¹: COO⁻ stretching vibration

¹H-NMR (D₂O 400 MHz): δ 0.79 (t, 3H, CH₃CH₂), δ 1.21 (m, 16H, CH₃(CH₂)₈), δ 1.47 (m, 2H, CH₂CH₂COO⁻), δ 2.10 (t, 2H, CH₂COO⁻), δ 3.32-3.38 (m, 1H, HOCH₂CH(N⁺H₃)CH₂OH), δ 3.61-3.77 (m, 4H, HOCH₂CH(N⁺H₃)CH₂OH).

¹³C-NMR (D₂O 100 MHz): δ 13.5 (CH₃CH₂), δ 22.2 (CH₃CH₂CH₂), δ 26.0 (CH₃CH₂CH₂), δ 28.7 (CH₃CH₂CH₂CH₂), δ 28.8 (CH₃CH₂CH₂CH₂CH₂), δ 29.0 ((CH₂)₄CH₂CH₂COO⁻), δ 31.4 (CH₂CH₂COO⁻), δ 37.5 (CH₂COO⁻), δ 54.1 (HOCH₂CH(N⁺H₃)CH₂OH), δ 58.6 (HOCH₂CH(N⁺H₃)CH₂OH), δ 183.6 (CH₂COO⁻).

### <Working example 27> Synthesis of compound 27

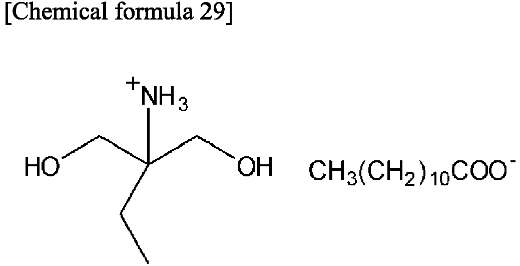

FT-IR(KBr): 3,388 cm⁻¹: O-H stretching vibration 2,922 cm⁻¹: C-H stretching vibration 1,543 cm⁻¹: COO⁻ stretching vibration

¹H-NMR (D₂O 400 MHz): δ 0.73-0.77 (m, 3H, NH₃⁺C(CH₂OH)₂CH₂CH₃), δ 0.79 (t, 3H, CH₃CH₂), δ 1.21 (m, 16H, CH₃(CH₂)₈), δ 1.47 (m, 2H, CH₂CH₂COO⁻), δ 1.50-1.56 (m, 2H, NH₃⁺C(CH₂OH)₂CH₂CH₃), δ 3.49-3.50 (m, 4H, NH₃⁺C(CH₂OH)₂CH₂CH₃), δ 2.10 (t, 2H, CH₂COO⁻).

¹³C-NMR (D₂O 100 MHz): δ 6.3 (NH₃⁺C(CH₂OH)₂CH₂CH₃), δ 13.5 (CH₃CH₂), δ 22.2 (CH₃CH₂CH₂), δ 23.2 (NH₃⁺C(CH₂OH)₂CH₂CH₃), δ 26.0 (CH₃CH₂CH₂), δ 28.7 (CH₃CH₂CH₂CH₂), δ 28.8 (CH₃CH₂CH₂CH₂CH₂), δ 29.0 ((CH₂)₄CH₂CH₂COO⁻), δ 31.4 (CH₂CH₂COO⁻), δ 37.5 (CH₂COO⁻), δ 60.5 (NH₃⁺C(CH₂OH)₂CH₂CH₃), δ 63.3 (NH₃⁺C(CH₂OH)₂CH₂CH₃), δ 183.6 (CH₂COO⁻).

### <Working example 28> Synthesis of compound 28

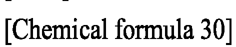

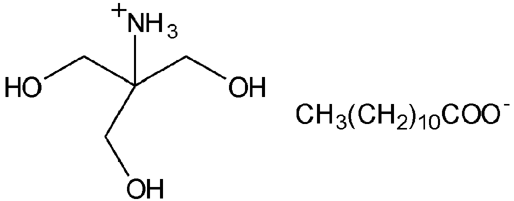

FT-IR(KBr): 3,145 cm⁻¹: O-H stretching vibration 2,946 cm⁻¹: C-H stretching vibration 1,572 cm⁻¹: COO⁻ stretching vibration

¹H-NMR (D₂O 400 MHz): δ 0.79 (t, 3H, CH₃CH₂), δ 1.21 (m, 16H, CH₃(CH₂)₈), δ 1.47 (m, 2H, CH₂CH₂COO⁻), δ 2.10 (t, 2H, CH₂COO⁻), δ 3.57 (s, 6H, NH₃⁺C(CH₂OH)₃).

¹³C-NMR (D₂O 100 MHz): δ 13.5 (CH₃CH₂), δ 22.2 (CH₃CH₂CH₂), δ 26.0 (CH₃CH₂CH₂), δ 28.7 (CH₃CH₂CH₂CH₂), δ 28.8 (CH₃CH₂CH₂CH₂CH₂), δ 29.0 ((CH₂)₄CH₂CH₂COO⁻), δ 31.4 (CH₂CH₂COO⁻), δ 37.5 (CH₂COO⁻), δ 59.2 (NH₃⁺C(CH₂OH)₃), δ 61.4 (NH₃⁺C(CH₂OH)₃), δ 183.6 (CH₂COO⁻).

### <Working example 29> Synthesis of compound 29

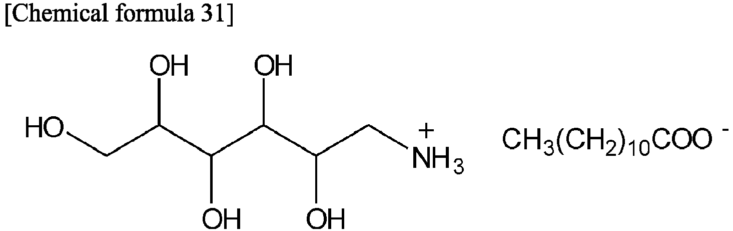

FT-IR(KBr): 3,167 cm⁻¹: O-H stretching vibration 2,920 cm⁻¹: C-H stretching vibration 1,573 cm⁻¹: COO⁻ stretching vibration

¹H-NMR (D₂O 400 MHz): δ 0.79 (t, 3H, CH₃CH₂), δ 1.21 (m, 16H, CH₃(CH₂)₈), δ 1.47 (m, 2H, CH₂CH₂COO⁻), δ 2.10 (t, 2H, CH₂COO⁻), δ 2.94-3.13 (m, 2H, HOCH₂(CH(OH))₃CH(OH)CH₂NH₃⁺), δ 3.51-3.72 (m, 5H, HOCH₂(CH(OH))₃CH(OH)CH₂NH₃⁺), δ 3.89-3.96 (m, 1H, HOCH₂(CH(OH))₃CH(OH)CH₂NH₃⁺).

¹³C-NMR (D₂O 100 MHz): δ 13.5 (CH₃CH₂), δ 22.2 (CH₃CH₂CH₂), δ 26.0 (CH₃CH₂CH₂), δ 28.7 (CH₃CH₂CH₂CH₂), δ 28.8 (CH₃CH₂CH₂CH₂CH₂), δ 29.0 ((CH₂)₄CH₂CH₂COO⁻), δ 31.4 (CH₂CH₂COO⁻), δ 37.5 (CH₂COO⁻), δ 41.7 (HOCH₂(CH(OH))₃CH(OH)CH₂NH₃⁺), δ 62.6 (HOCH₂(CH(OH))₃CH(OH)CH₂NH₃⁺), δ 68.9-70.9 (HOCH₂(CH(OH))₃CH(OH)CH₂NH₃⁺), δ 183.6 (CH₂COO⁻).

### <Working example 30> Synthesis of compound 30

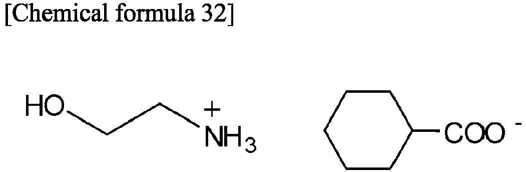

FT-IR(KBr): 3,177 cm⁻¹: O-H stretching vibration 2,950 cm⁻¹: C-H stretching vibration 1,563 cm⁻¹: COO⁻ stretching vibration

¹H-NMR (D₂O 400 MHz): δ 1.13 (m, 6H, CHCH₂(CH₂)₃), δ 1.55 (m, 4H, CHCH₂), δ 2.03 (m, 1H, CHCOO⁻), δ 3.04 (m, 2H, N⁺CH₂CH₂OH), δ 3.77 (m, 2H, N⁺CH₂CH₂OH).

¹³C-NMR (D₂O 100 MHz): δ 25.5 (CH₂CH₂CHCOO⁻), δ 25.7 (CH₂CHCOO⁻), δ 29.9 (CH₂CH₂CH₂CHCOO⁻), δ 41.2 (N⁺CH₂CH₂OH), δ 47.1 (CHCOO⁻), δ 57.6 (N⁺CH₂CH₂OH), δ 186.8 (CHCOO⁻).

### <Working example 31> Synthesis of compound 31

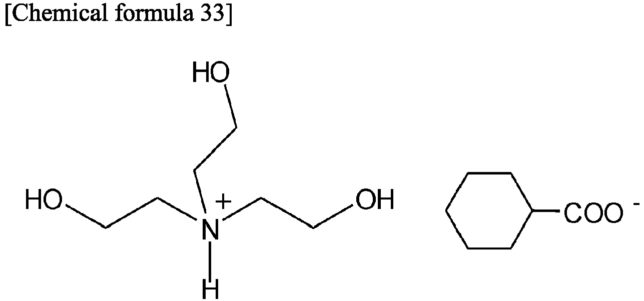

FT-IR(KBr): 3,360 cm⁻¹: O-H stretching vibration 2,950 cm⁻¹: C-H stretching vibration 1,558 cm⁻¹: COO⁻ stretching vibration

¹H-NMR (D₂O 400 MHz): δ 1.13 (m, 6H, CHCH₂(CH₂)₃), δ 1.55 (m, 4H, CHCH₂), δ 2.03 (m, 1H, CHCOO⁻), δ 3.31(t, 6H, N⁺CH₂CH₂OH), δ 3.85 (t, 6H, W⁺CH₂CH₂OH).

¹³C-NMR (D₂O 100 MHz): δ 25.5 (CH₂CH₂CHCOO⁻), δ 25.7 (CH₂CHCOO⁻), δ 29.9 (CH₂CH₂CH₂CHCOO⁻), δ 47.1 (CHCOO⁻), δ 55.4 (N⁺CH₂CH₂OH), δ 55.6 (N⁺CH₂CH₂OH), δ 186.8 (CHCOO⁻).

### <Working example 32> Synthesis of compound 32

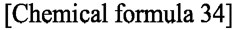

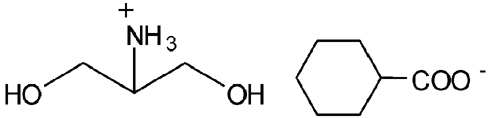

FT-IR(KBr): 3,375 cm⁻¹: O-H stretching vibration 2,955 cm⁻¹: C-H stretching vibration 1,576 cm⁻¹: COO⁻ stretching vibration

¹H-NMR (D₂O 400 MHz): δ 1.13 (m, 6H, CHCH₂(CH₂)₃), δ 1.55 (m, 4H, CHCH₂), δ 2.03 (m, 1H, CHCOO⁻), δ 3.32-3.38 (m, 1H, HOCH₂CH(N⁺H₃)CH₂OH), δ 3.61-3.77 (m, 4H, HOCH₂CH(N⁺H₃)CH₂OH).

¹³C-NMR (D₂O 100 MHz): δ 25.5 (CH₂CH₂CHCOO⁻), δ 25.7 (CH₂CHCOO⁻), δ 29.9 (CH₂CH₂CH₂CHCOO⁻), δ 47.1 (CHCOO⁻), δ 54.1 (HOCH₂CH(N⁺H₃)CH₂OH), δ 58.6 (HOCH₂CH(N⁺H₃)CH₂OH), δ 186.8 (CHCOO⁻).

### <Working example 33> Synthesis of compound 33

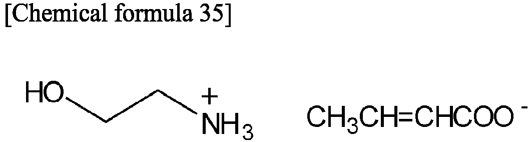

FT-IR(KBr): 3,177 cm⁻¹: O-H stretching vibration 2,950 cm⁻¹: C-H stretching vibration 1,563 cm⁻¹: COO⁻ stretching vibration

¹H-NMR (D₂O 400 MHz): δ 1.68-1.70 (m, 3H, CH₃CHCHCOO⁻), δ 3.04 (m, 2H, N⁺CH₂CH₂OH), δ 3.77 (m, 2H, N⁺CH₂CH₂OH), δ 5.69-5.75 (m, 1H, CH₃CHCHCOO⁻), δ 6.49-6.53 (m, 1H, CH₃CHCHCOO⁻).

¹³C-NMR (D₂O 100 MHz): δ 16.9 (CH₃CHCHCOO⁻), δ 41.2 (N⁺CH₂CH₂OH), δ 57.6 (N⁺CH₂CH₂OH), δ 127.2 (CH₃CHCHCOO⁻), δ 141.2 (CH₃CHCHCOO⁻), δ 175.9 (CH₃CHCHCOO⁻).

### <Working example 34> Synthesis of compound 34

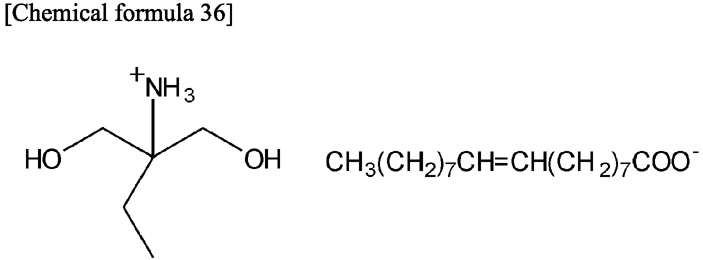

FT-IR(KBr): 3,388 cm⁻¹: O-H stretching vibration 2,922 cm⁻¹: C-H stretching vibration 1,543 cm⁻¹: COO⁻ stretching vibration

¹H-NMR (D₂O 400 MHz): δ 0.73-0.77 (m, 3H, NH₃⁺C(CH₂OH)₂CH₂CH₃), δ 0.89 (t, 3H, CH₃CH₂), δ 1.27 (m, 20H, CH₃(CH₂)₆CH₂, (CH₂)₄ CH₂CH₂COO⁻), δ 1.50-1.56 (m, 2H, NH₃⁺C(CH₂OH)₂CH₂CH₃), δ 1.53 (m, 2H, CH₂CH₂COO⁻), δ 2.00 (m, 4H, CH₂CH=CHCH₂), δ 2.14 (t, 2H, CH₂CH₂COO⁻), δ 2.99 (t, 2H, N⁺CH₂CH₂OH), δ 3.49-3.50 (m, 4H, NH₃⁺C(CH₂OH)₂CH₂CH₃), δ 3.76 (t, 2H, N⁺CH₂CH₂OH), δ 5.32 (m, 2H, CH=CH).

¹³C-NMR (D₂O 100 MHz): δ 6.3 (NH₃⁺C(CH₂OH)₂CH₂CH₃), δ 14.0 (CH₃CH₂), δ 22.6 (CH₃CH₂), δ 23.2 (NH₃⁺C(CH₂OH)₂CH₂CH₃), δ 26.4 (CH₂CH₂COO⁻), δ 27.2 (CH₂CH=CHCH₂), δ 29.3 (CH₃CH₂CH₂(CH₂)₄, (CH₂)₄CH₂CH₂COO⁻), δ 31.9 (CH₃CH₂CH₂), δ 37.8 (CH₂CH₂COO⁻), δ 41.9 (N⁺CH₂CH₂OH), δ 58.6 (N⁺CH₂CH₂OH), δ 60.5 (NH₃⁺C(CH₂OH)₂CH₂CH₃), δ 63.3 (NH₃⁺C(CH₂OH)₂CH₂CH₃), δ 129.7 (CH=CH), δ 181.7 (COO).

### <Working example 35> Synthesis of compound 35

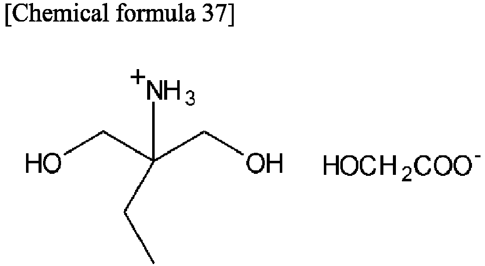

FT-IR(KBr): 3,388 cm⁻¹: O-H stretching vibration 2,922 cm⁻¹: C-H stretching vibration 1,543 cm⁻¹: COO⁻ stretching vibration

¹H-NMR (D₂O 400 MHz): δ 0.73-0.77 (m, 3H, NH₃⁺C(CH₂OH)₂CH₂CH₃), δ 1.50-1.56 (m, 2H, NH₃⁺C(CH₂OH)₂CH₂CH₃), δ 3.49-3.50 (m, 4H, NH₃⁺C(CH₂OH)₂CH₂CH₃), δ 3.87 (s, 2H, HOCH₂COO⁻).

¹³C-NMR (D₂O 100 MHz): δ 6.3 (NH₃⁺C(CH₂OH)₂CH₂CH₃), δ 23.2 (NH₃⁺C(CH₂OH)₂CH₂CH₃), δ 60.5 (NH₃⁺C(CH₂OH)₂CH₂CH₃), δ 61.3 (HOCH₂COO⁻), δ 63.3 (NH₃⁺C(CH₂OH)₂CH₂CH₃), δ 179.9 (HOCH₂COO⁻).

### <Working example 36> Synthesis of compound 36

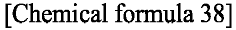

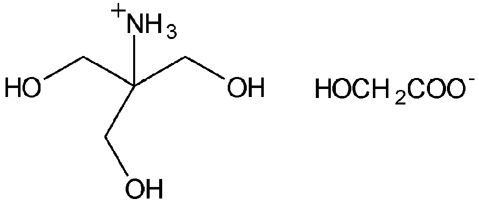

FT-IR(KBr): 3,145 cm⁻¹: O-H stretching vibration 2,946 cm⁻¹: C-H stretching vibration 1,572 cm⁻¹: COO⁻ stretching vibration

¹H-NMR (D₂O 400 MHz): δ 3.57 (s, 6H, NH₃⁺C(CH₂OH)₃), δ 3.87 (s, 2H, HOCH₂COO⁻). ¹³C-NMR (D₂O 100 MHz): δ 59.2 (NH₃⁺C(CH₂OH)₃), δ 61.3 (HOCH₂COO⁻), δ 61.4 (NH₃⁺C(CH₂OH)₃), δ 179.9 (HOCH₂COO⁻).

### <Working example 37> Synthesis of compound 37

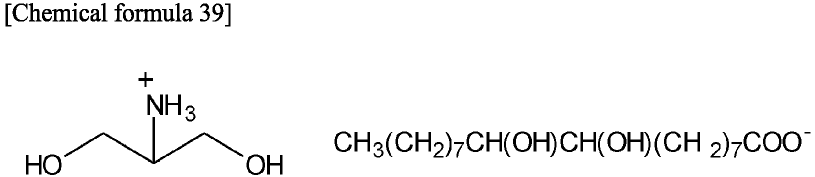

FT-IR(KBr): 3,375 cm⁻¹: O-H stretching vibration 2,955 cm⁻¹: C-H stretching vibration 1,576 cm⁻¹: COO⁻ stretching vibration

¹H-NMR (D₂O 400 MHz): δ 0.89 (t, 3H, CH₃CH₂), δ 1.27 (m, 20H, CH₃(CH₂)₆CH₂, (CH₂)₄ CH₂CH₂COO⁻), δ 1.53 (m, 2H, CH₂CH₂COO⁻), δ 1.54 (m, 4H, CH₂CH(OH)CH(OH)CH₂), δ 2.14 (t, 2H, CH₂CH₂COO⁻), δ 3.29 (m, 2H, CH(OH)CH(OH)), δ 3.32-3.38 (m, 1H, HOCH₂CH(N⁺H₃)CH₂OH), δ 3.61-3.77 (m, 4H, HOCH₂CH(N⁺H₃)CH₂OH).

¹³C-NMR (D₂O 100 MHz): δ 14.0 (CH₃CH₂), δ 22.6 (CH₃CH₂), δ 26.4 (CH₂CH₂COO⁻), δ 29.3 (CH₃CH₂CH₂(CH₂)₄, (CH₂)₄CH₂CH₂COO⁻), δ 31.7 (CH₂CH(OH)CH(OH)CH₂), δ 31.9 (CH₃CH₂CH₂), δ 37.8 (CH₂CH₂COO⁻), δ 54.1 (HOCH₂CH(NH₃)CH₂OH), δ 58.6 (HOCH₂CH(N⁺H₃)CH₂OH), δ 77.2 (CH(OH)CH(OH)), δ 181.7 (COO⁻).

### <Working example 38> Synthesis of compound 38

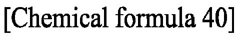

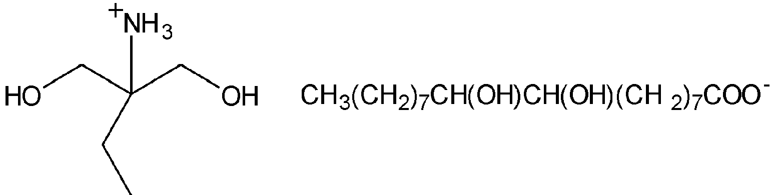

FT-IR(KBr): 3,388 cm⁻¹: O-H stretching vibration 2,922 cm⁻¹: C-H stretching vibration 1,543 cm⁻¹: COO⁻ stretching vibration

¹H-NMR (D₂O 400 MHz): δ 0.73-0.77 (m, 3H, NH₃⁺C(CH₂OH)₂CH₂CH₃), δ 0.89 (t, 3H, CH₃CH₂), δ 1.27 (m, 20H, CH₃(CH₂)₆CH₂, (CH₂)₄ CH₂CH₂COO⁻), δ 1.50-1.56 (m, 2H, NH₃⁺C(CH₂OH)₂CH₂CH₃), δ 1.53 (m, 2H, CH₂CH₂COO⁻), δ 1.54 (m, 4H, CH₂CH(OH)CH(OH)CH₂), δ 2.14 (t, 2H, CH₂CH₂COO⁻), δ 3.29 (m, 2H, CH(OH)CH(OH)), δ 3.49-3.50 (m, 4H, NH₃⁺C(CH₂OH)₂CH₂CH₃).

¹³C-NMR (D₂O 100 MHz): δ 6.3 (NH₃⁺C(CH₂OH)₂CH₂CH₃), δ 14.0 (CH₃CH₂), δ 22.6 (CH₃CH₂), δ 23.2 (NH₃⁺C(CH₂OH)₂CH₂CH₃), δ 26.4 (CH₂CH₂COO⁻), δ 29.3 (CH₃CH₂CH₂(CH₂)₄, (CH₂)₄CH₂CH₂COO⁻), δ 31.7 (CH₂CH(OH)CH(OH)CH₂), δ 31.9 (CH₃CH₂CH₂), δ 37.8 (CH₂CH₂COO⁻), δ 60.5 (NH₃⁺C(CH₂OH)₂CH₂CH₃), δ 63.3 (NH₃⁺C(CH₂OH)₂CH₂CH₃), δ 77.2 (CH(OH)CH(OH)), δ 181.7 (COO⁻).

### <Working example 39> Synthesis of compound 39

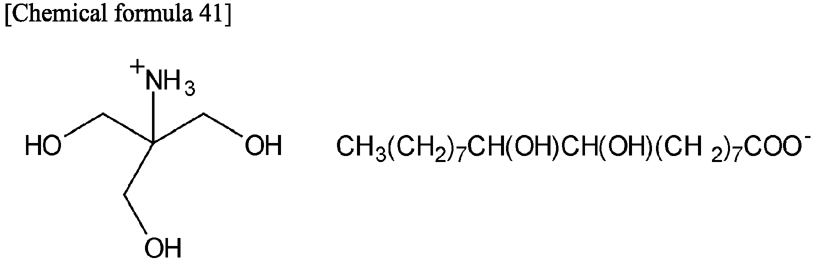

FT-IR(KBr): 3,145 cm⁻¹: O-H stretching vibration 2,946 cm⁻¹: C-H stretching vibration 1,572 cm⁻¹: COO⁻ stretching vibration

¹H-NMR (D₂O 400 MHz): δ 0.89 (t, 3H, CH₃CH₂), δ 1.27 (m, 20H, CH₃(CH₂)₆CH₂, (CH₂)₄ CH₂CH₂COO⁻), δ 1.53 (m, 2H, CH₂CH₂COO⁻), δ 1.54 (m, 4H, CH₂CH(OH)CH(OH)CH₂), δ 2.14 (t, 2H, CH₂CH₂COO⁻), δ 3.29 (m, 2H, CH(OH)CH(OH)), δ 3.57 (s, 6H, NH₃⁺C(CH₂OH)₃).

¹³C-NMR (D₂O 100 MHz): δ 14.0 (CH₃CH₂), δ 22.6 (CH₃CH₂), δ 26.4 (CH₂CH₂COO⁻), δ 29.3 (CH₃CH₂CH₂(CH₂)₄, (CH₂)₄CH₂CH₂COO⁻), δ 31.7 (CH₂CH(OH)CH(OH)CH₂), δ 31.9 (CH₃CH₂CH₂), δ 37.8 (CH₂CH₂COO⁻), δ 59.2 (NH₃⁺C(CH₂OH)₃), δ 61.4 (NH₃⁺C(CH₂OH)₃), δ 77.2 (CH(OH)CH(OH)), δ 181.7 (COO⁻).

### <Working example 40> Synthesis of compound 40

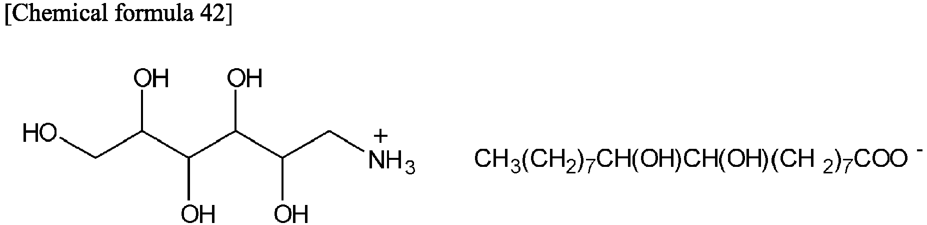

FT-IR(KBr): 3,167 cm⁻¹: O-H stretching vibration 2,920 cm⁻¹: C-H stretching vibration 1,573 cm⁻¹: COO⁻ stretching vibration

¹H-NMR (D₂O 400 MHz): δ 0.89 (t, 3H, CH₃CH₂), δ 1.27 (m, 20H, CH₃(CH₂)₆CH₂, (CH₂)₄ CH₂CH₂COO⁻), δ 1.53 (m, 2H, CH₂CH₂COO⁻), δ 1.54 (m, 4H, CH₂CH(OH)CH(OH)CH₂), δ 2.14 (t, 2H, CH₂CH₂COO⁻), δ 2.94-3.13 (m, 2H, HOCH₂(CH(OH))₃CH(OH)CH₂NH₃⁺), δ 3.29 (m, 2H, CH(OH)CH(OH)), δ 3.51-3.72 (m, 5H, HOCH₂(CH(OH))₃CH(OH)CH₂NH₃⁺), δ 3.89-3.96 (m, 1H, HOCH₂(CH(OH))₃CH(OH)CH₂NH₃⁺).

¹³C-NMR (D₂O 100 MHz): δ 14.0 (CH₃CH₂), δ 22.6 (CH₃CH₂), δ 26.4 (CH₂CH₂COO⁻), δ 29.3 (CH₃CH₂CH₂(CH₂)₄, (CH₂)₄CH₂CH₂COO⁻), δ 31.7 (CH₂CH(OH)CH(OH)CH₂), δ 31.9 (CH₃CH₂CH₂), δ 37.8 (CH₂CH₂COO⁻), δ 41.7 (HOCH₂(CH(OH))₃CH(OH)CH₂NH₃⁺), δ 62.6 (HOCH₂(CH(OH))₃CH(OH)CH₂NH₃⁺), δ 68.9-70.9 (HOCH₂(CH(OH))₃CH(OH)CH₂NH₃⁺), δ 77.2 (CH(OH)CH(OH)), δ 181.7 (COO⁻).

### <Working example 41> Synthesis of compound 41

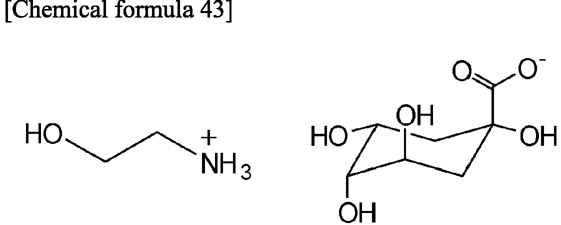

FT-IR(KBr): 3,177 cm⁻¹: O-H stretching vibration 2,950 cm⁻¹: C-H stretching vibration 1,563 cm⁻¹: COO⁻ stretching vibration

¹H-NMR (D₂O 400 MHz): δ 1.66-1.89 (m, 4H, CH₂C(OH)(COO⁻)), δ 3.04 (m, 2H, N⁺CH₂CH₂OH), δ 3.35 (m, 1H, CH(OH)CH(OH)CH(OH)), δ 3.77 (m, 2H, N⁺CH₂CH₂OH), δ 3.79-3.95 (m, 2H, CH₂CH(OH)CH(OH)).

¹³C-NMR (D₂O 100 MHz): δ 25.5 (CH₂CH₂CHCOO⁻), δ 25.7 (CH₂CHCOO⁻), δ 29.9 (CH₂CH₂CH₂CHCOO⁻), δ 37.3 (CH₂C(OH)(COO⁻)), δ 40.6 (CH₂C(OH)(COO⁻)), δ 41.2 (N⁺CH₂CH₂OH), δ 47.1 (CHCOO⁻), δ 57.6 (N⁺CH₂CH₂OH), δ 66.9 (CH₂CH(OH)CH(OH)), δ 70.3 (CH₂CH(OH)CH(OH)), δ 75.1 (CH(OH)CH(OH)CH(OH)), δ 76.9 (CH₂C(OH)(COO⁻)), δ 181.3 (CH₂C(OH)(COO⁻)).

### <Working example 42> Synthesis of compound 42

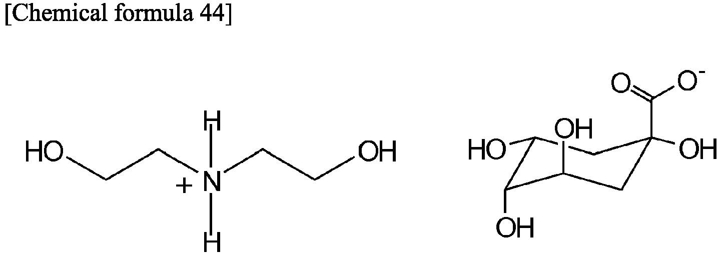

FT-IR(KBr): 3,306 cm⁻¹: O-H stretching vibration 2,923 cm⁻¹: C-H stretching vibration 1,559 cm⁻¹: COO⁻ stretching vibration

¹H-NMR (D₂O 400 MHz): δ 1.66-1.89 (m, 4H, CH₂C(OH)(COO⁻)), δ 3.15 (t, 4H, N⁺CH₂CH₂OH), δ 3.35 (m, 1H, CH(OH)CH(OH)CH(OH)), δ 3.79 (t, 4H, N⁺CH₂CH₂OH), δ 3.79-3.95 (m, 2H, CH₂CH(OH)CH(OH)).

¹³C-NMR (D₂O 100 MHz): δ 37.3 (CH₂C(OH)(COO⁻)), δ 40.6 (CH₂C(OH)(COO⁻)), δ 48.9 (N⁺CH₂CH₂OH), δ 56.6 (N⁺CH₂CH₂OH), δ 66.9 (CH₂CH(OH)CH(OH)), δ 70.3 (CH₂CH(OH)CH(OH)), δ 75.1 (CH(OH)CH(OH)CH(OH)), δ 76.9 (CH₂C(OH)(COO⁻)), δ 181.3 (CH₂C(OH)(COO⁻)).

### <Working example 43> Synthesis of compound 43

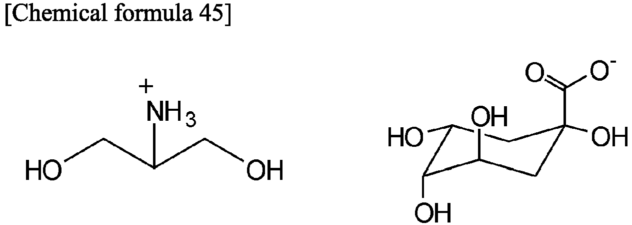

FT-IR(KBr): 3,375 cm⁻¹: O-H stretching vibration 2,955 cm⁻¹: C-H stretching vibration 1,576 cm⁻¹: COO⁻ stretching vibration

¹H-NMR (D₂O 400 MHz): δ 1.66-1.89 (m, 4H, CH₂C(OH)(COO-)), δ 3.32-3.38 (m, 1H, HOCH₂CH(N⁺H₃)CH₂OH), δ 3.35 (m, 1H, CH(OH)CH(OH)CH(OH)), δ 3.61-3.77 (m, 4H, HOCH₂CH(N⁺H₃)CH₂OH), δ 3.79-3.95 (m, 2H, CH₂CH(OH)CH(OH)).

¹³C-NMR (D₂O 100 MHz): δ 37.3 (CH₂C(OH)(COO⁻)), δ 40.6 (CH₂C(OH)(COO⁻)), δ 54.1 (HOCH₂CH(N⁺H₃)CH₂OH), δ 58.6 (HOCH₂CH(N⁺H₃)CH₂OH), δ 66.9 (CH₂CH(OH)CH(OH)), δ 70.3 (CH₂CH(OH)CH(OH)), δ 75.1 (CH(OH)CH(OH)CH(OH)), δ 76.9 (CH₂C(OH)(COO⁻)), δ 181.3 (CH₂C(OH)(COO⁻)).

### <Working example 44> Synthesis of compound 44

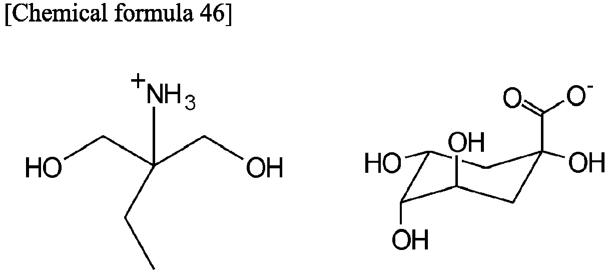

FT-IR(KBr): 3,388 cm⁻¹: O-H stretching vibration 2,922 cm⁻¹: C-H stretching vibration 1,543 cm⁻¹: COO⁻ stretching vibration

¹H-NMR (D₂O 400 MHz): δ 0.73-0.77 (m, 3H, NH₃⁺C(CH₂OH)₂CH₂CH₃), δ 1.50-1.56 (m, 2H, NH₃⁺C(CH₂OH)₂CH₂CH₃), δ 1.66-1.89 (m, 4H, CH₂C(OH)(COO⁻)), δ 3.35 (m, 1H, CH(OH)CH(OH)CH(OH)), δ 3.49-3.50 (m, 4H, NH₃⁺C(CH₂OH)₂CH₂CH₃), δ 3.79-3.95 (m, 2H, CH₂CH(OH)CH(OH)).

¹³C-NMR (D₂O 100 MHz): δ 6.3 (NH₃⁺C(CH₂OH)₂CH₂CH₃), δ 23.2 (NH₃⁺C(CH₂OH)₂CH₂CH₃), δ 37.3 (CH₂C(OH)(COO⁻)), δ 40.6 (CH₂C(OH)(COO⁻)), δ 60.5 (NH₃⁺C(CH₂OH)₂CH₂CH₃), δ 63.3 (NH₃⁺C(CH₂OH)₂CH₂CH₃), δ 66.9 (CH₂CH(OH)CH(OH)), δ 70.3 (CH₂CH(OH)CH(OH)), δ 75.1 (CH(OH)CH(OH)CH(OH)), δ 76.9 (CH₂C(OH)(COO⁻)), δ 181.3 (CH₂C(OH)(COO⁻)).

### <Working example 45> Synthesis of compound 45

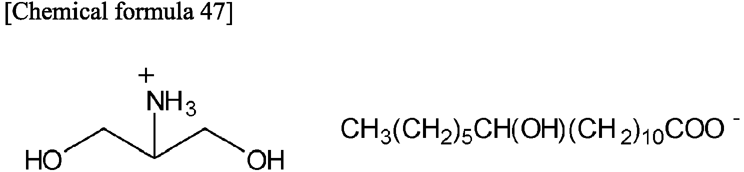

FT-IR(KBr): 3,375 cm⁻¹: O-H stretching vibration 2,955 cm⁻¹: C-H stretching vibration 1,576 cm⁻¹: COO⁻ stretching vibration

¹H-NMR (D₂O 400 MHz): δ 0.89 (t, 3H, CH₃CH₂), δ 1.27 (m, 22H, CH₃(CH₂)₉CH₂, (CH₂)₂ CH₂CH₂COO⁻), δ 1.53 (m, 2H, CH₂CH₂COO⁻), δ 1.54 (m, 4H, CH₂CH(OH)CH₂), δ 2.14 (t, 2H, CH₂CH₂COO⁻), δ 3.29 (m, 1H, CH(OH)), δ 3.32-3.38 (m, 1H, HOCH₂CH(N⁺H₃)CH₂OH), δ 3.61-3.77 (m, 4H, HOCH₂CH(N⁺H₃)CH₂OH).

¹³C-NMR (D₂O 100 MHz): δ 14.0 (CH₃CH₂), δ 22.6 (CH₃CH₂), δ 26.4 (CH₂CH₂COO⁻), δ 29.3 (CH₃CH₂CH₂(CH₂)₇, (CH₂)₂CH₂CH₂COO⁻), δ 31.7 (CH₂CH(OH)CH₂), δ 31.9 (CH₃CH₂CH₂), δ 37.8 (CH₂COO⁻), δ 54.1 (HOCH₂CH(N⁺H₃)CH₂OH), δ 58.6 (HOCH₂CH(N⁺H₃)CH₂OH), δ 77.2 (CH(OH)), δ 181.7 (COO⁻).

### <Working example 46> Synthesis of compound 46

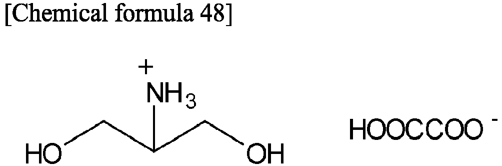

FT-IR(KBr): 3,388 cm⁻¹: O-H stretching vibration 2,958 cm⁻¹: C-H stretching vibration 1,714 cm⁻¹: COOH stretching vibration 1,558 cm⁻¹: COO⁻ stretching vibration

¹H-NMR (D₂O 400 MHz): δ 3.29-3.35 (m, 1H, HOCH₂CH(N⁺H₃)CH₂OH), δ 3.58-3.74 (m, 4H, HOCH₂CH(N⁺H₃)CH₂OH).

¹³C-NMR (D₂O 100 MHz): δ 54.1 (HOCH₂CH(N⁺H₃)CH₂OH), δ 58.6 (HOCH₂CH(N⁺H₃)CH₂OH), δ 179.7 (HOOCCOO⁻).

### <Working example 47> Synthesis of compound 47

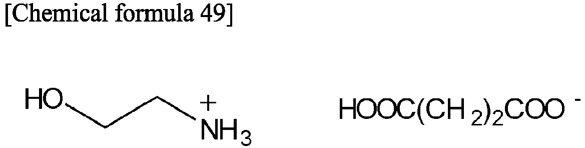

FT-IR(KBr): 3,162 m⁻¹: O-H stretching vibration 2,950 cm⁻¹: C-H stretching vibration 1,715 cm⁻¹: COOH stretching vibration 1,578 cm⁻¹: COO⁻ stretching vibration

¹H-NMR (D₂O 400 MHz): δ 2.51 (s, 4H, HOOCCH₂CH₂COO⁻), δ 3.08 (t, 2H, N⁺CH₂CH₂OH), δ 3.76 (t, 2H, NCH₂CH₂OH).

¹³C-NMR (D₂O 100 MHz): δ 31.4 (HOOCCH₂CH₂COO⁻), δ 41.3 (N⁺CH₂CH₂OH), δ 57.6 (N⁺CH₂CH₂OH), δ 179.7 (COOH, COO⁻).

### <Working example 48> Synthesis of compound 48

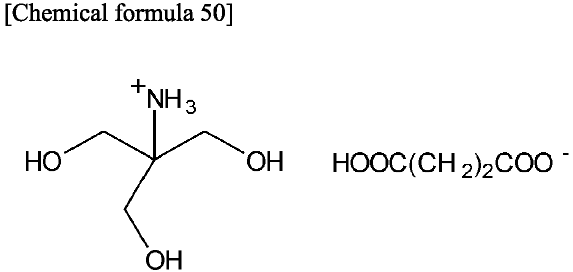

FT-IR(KBr): 3,145 cm⁻¹: O-H stretching vibration 2,946 cm⁻¹: C-H stretching vibration 1,711 cm⁻¹: COOH stretching vibration 1,572 cm⁻¹: COO⁻ stretching vibration

¹H-NMR (D₂O 400 MHz): δ 2.51 (s, 4H, HOOCCH₂CH₂COO⁻), δ 3.57 (s, 6H, NH₃⁺C(CH₂OH)₃).

¹³C-NMR (D₂O 100 MHz): δ 31.4 (HOOCCH₂CH₂COO⁻), δ 59.2 (NH₃⁺C(CH₂OH)₃), δ 61.4 (NH₃⁺C(CH₂OH)₃), δ 179.7 (COOH, COO⁻).

### <Working example 49> Synthesis of compound 49

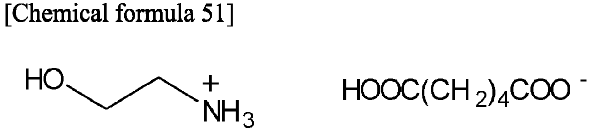

FT-IR(KBr): 3,162 m⁻¹: O-H stretching vibration 2,950 cm⁻¹: C-H stretching vibration 1,715 cm⁻¹: COOH stretching vibration 1,578 cm⁻¹: COO⁻ stretching vibration

¹H-NMR (D₂O 400 MHz): δ 1.56 (t, 4H, HOOCCH₂CH₂CH₂CH₂COO⁻), δ 2.27 (t, 4H, HOOCCH₂CH₂CH₂CH₂COO⁻), δ 3.08 (t, 2H, N⁺CH₂CH₂OH), δ 3.76 (t, 2H, N⁺CH₂CH₂OH).

¹³C-NMR (D₂O 100 MHz): δ 24.8 (HOOCCH₂CH₂CH₂CH₂COO⁻), δ 35.5 (HOOCCH₂CH₂CH₂CH₂COO⁻), δ 41.3 (N⁺CH₂CH₂OH), δ 57.6 (N⁺CH₂CH₂OH), δ 179.7 (COOH, COO⁻).

### <Working example 50> Synthesis of compound 50

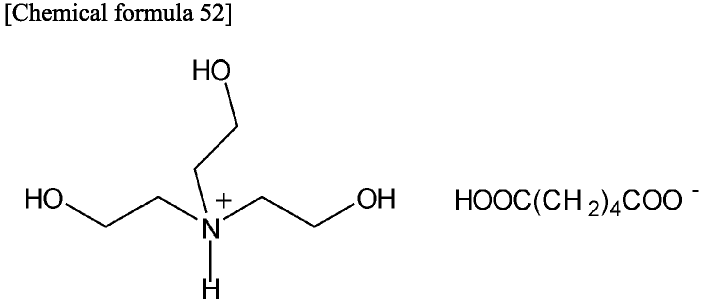

FT-IR(KBr): 3,312 m⁻¹: O-H stretching vibration 2,939 cm⁻¹: C-H stretching vibration 1,719 cm⁻¹: COOH stretching vibration 1,588 cm⁻¹: COO⁻ stretching vibration

¹H-NMR (D₂O 400 MHz): δ 1.56 (t, 4H, HOOCCH₂CH₂CH₂CH₂COO⁻), δ 2.27 (t, 4H, HOOCCH₂CH₂CH₂CH₂COO⁻), δ 3.31 (t, 6H, N⁺CH₂CH₂OH),δ 3.85 (t, 6H, N⁺CH₂CH₂OH).

¹³C-NMR (D₂O 100 MHz): δ 24.8 (HOOCCH₂CH₂CH₂CH₂COO⁻), δ 35.5 (HOOCCH₂CH₂CH₂CH₂COO⁻), δ 55.4 (N⁺CH₂CH₂OH), δ 55.6 (N⁺CH₂CH₂OH), δ 179.7 (COOH, COO⁻).

### <Working example 51> Synthesis of compound 51

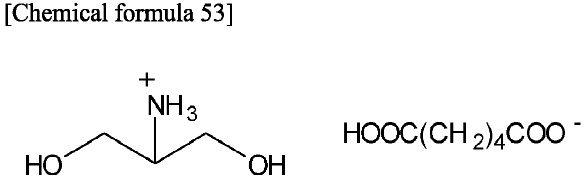

FT-IR(KBr): 3,388 cm⁻¹: O-H stretching vibration 2,958 cm⁻¹: C-H stretching vibration 1,714 cm⁻¹: COOH stretching vibration 1,558 cm⁻¹: COO⁻ stretching vibration

¹H-NMR (D₂O 400 MHz): δ 1.56 (t, 4H, HOOCCH₂CH₂CH₂CH₂COO⁻), δ 2.27 (t, 4H, HOOCCH₂CH₂CH₂CH₂COO⁻), δ 3.29-3.35 (m, 1H, HOCH₂CH(N⁺H₃)CH₂OH), δ 3.58-3.74 (m, 4H, HOCH₂CH(N⁺H₃)CH₂OH).

¹³C-NMR (D₂O 100 MHz): δ 24.8 (HOOCCH₂CH₂CH₂CH₂COO⁻), δ 35.5 (HOOCCH₂CH₂CH₂CH₂COO⁻), δ 54.1 (HOCH₂CH(N⁺H₃)CH₂OH), δ 58.6 (HOCH₂CH(N⁺H₃)CH₂OH), δ 179.7 (COOH, COO⁻).

### <Working example 52> Synthesis of compound 52

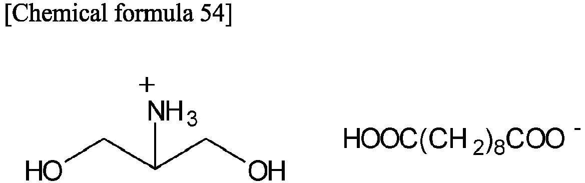

FT-IR(KBr): 3,388 cm⁻¹: O-H stretching vibration 2,958 cm⁻¹: C-H stretching vibration 1,714 cm⁻¹: COOH stretching vibration 1,558 cm⁻¹: COO⁻ stretching vibration

¹H-NMR (D₂O 400 MHz): δ 1.29 (t, 8H, HOOCCH₂CH₂(CH₂)₄CH₂CH₂COO⁻), δ 1.56 (t, 4H, HOOCCH₂CH₂(CH₂)₄CH₂CH₂COO⁻), δ 2.27 (t, 4H, HOOCCH₂CH₂(CH₂)₄CH₂CH₂COO⁻), δ 3.29-3.35 (m, 1H, HOCH₂CH(N⁺H₃)CH₂OH), δ 3.58-3.74 (m, 4H, HOCH₂CH(N⁺H₃)CH₂OH).

¹³C-NMR (D₂O 100 MHz): δ 24.8 (HOOCCH₂CH₂(CH₂)₄CH₂CH₂COO⁻), δ 29.7 (HOOCCH₂CH₂(CH₂)₄CH₂CH₂COO⁻), δ 35.5 (HOOCCH₂CH₂(CH₂)₄CH₂CH₂COO⁻), δ 54.1 (HOCH₂CH(N⁺H₃)CH₂OH), δ 58.6 (HOCH₂CH(N⁺H₃)CH₂OH), δ 179.7 (COOH, COO⁻).

### <Working example 53> Synthesis of compound 53

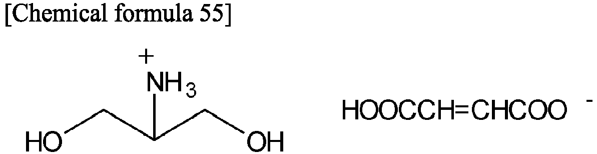

FT-IR(KBr): 3,388 cm⁻¹: O-H stretching vibration 2,958 cm⁻¹: C-H stretching vibration 1,714 cm⁻¹: COOH stretching vibration 1,558 cm⁻¹: COO⁻ stretching vibration

¹H-NMR (D₂O 400 MHz): δ 3.29-3.35 (m, 1H, HOCH₂CH(N⁺H₃)CH₂OH), δ 3.58-3.74 (m, 4H, HOCH₂CH(N⁺H₃)CH₂OH), δ 7.03 (t, 4H, HOOCCH=CHCOO⁻).

¹³C-NMR (D₂O 100 MHz): δ 54.1 (HOCH₂CH(N⁺H₃)CH₂OH), δ 58.6 (HOCH₂CH(N⁺H₃)CH₂OH), δ 137.7 (HOOCCH=CHCOO⁻), δ 179.7 (COOH, COO⁻).

### <Working example 54> Synthesis of compound 54

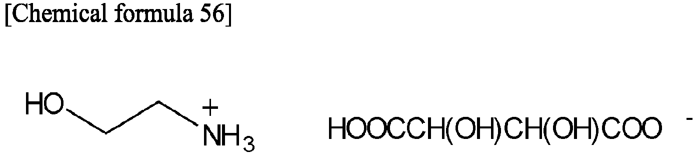

FT-IR(KBr): 3,162 m⁻¹: O-H stretching vibration 2,950 cm⁻¹: C-H stretching vibration 1,715 cm⁻¹: COOH stretching vibration 1,578 cm⁻¹: COO⁻ stretching vibration

¹H-NMR (D₂O 400 MHz): δ 2.27 (t, 4H, HOOCCH₂CH₂CH₂CH₂COO⁻), δ 3.08 (t, 2H, N⁺CH₂CH₂OH), δ 3.76 (t, 2H, N⁺CH₂CH₂OH), δ 4.49 (s, 2H, HOOCCH(OH)CH(OH)COO⁻).

¹³C-NMR (D₂O 100 MHz): δ 41.3 (N⁺CH₂CH₂OH), δ 57.6 (N⁺CH₂CH₂OH), δ 72.8 (HOOCCH(OH)CH(OH)COO⁻), δ 176.3 (HOOCCH(OH)CH(OH)COO⁻).

### <Working example 55> Synthesis of compound 55

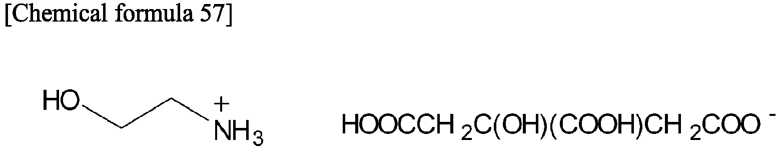

FT-IR(KBr): 3,162 m⁻¹: O-H stretching vibration 2,950 cm⁻¹: C-H stretching vibration 1,715 cm⁻¹: COOH stretching vibration 1,578 cm⁻¹: COO⁻ stretching vibration

¹H-NMR (D₂O 400 MHz): δ 2.65 (m, 4H, HOOCCH₂C(OH)(COOH)CH₂COO⁻), δ 3.08 (t, 2H, N⁺CH₂CH₂OH), δ 3.76 (t, 2H, N⁺CH₂CH₂OH).

¹³C-NMR (D₂O 100 MHz): δ 41.3 (N⁺CH₂CH₂OH), δ 43.7 (HOOCCH₂C(OH)(COOH)CH₂COO⁻), δ 57.6 (N⁺CH₂CH₂OH), δ 73.9 (HOOCCH₂C(OH)(COOH)CH₂COO⁻), δ 174.8 (HOOCCH₂C(OH)(COOH)CH₂COO⁻), δ 178.7 (HOOCCH₂C(OH)(COOH)CH₂COO⁻).

### <Working example 56> Synthesis of compound 56

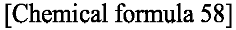

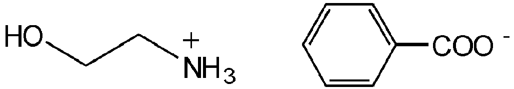

FT-IR(KBr): 3,162 m⁻¹: O-H stretching vibration 2,950 cm⁻¹: C-H stretching vibration 1,578 cm⁻¹: COO⁻ stretching vibration

¹H-NMR (D₂O 400 MHz): δ 3.08 (t, 2H, N⁺CH₂CH₂OH), δ 3.76 (t, 2H, N⁺CH₂CH₂OH), δ 7.40-7.50 (m, 3H, CHCHCHCHCCOO⁻), δ 7.51-7.80 (m, 2H, CHCCOOH).

¹³C-NMR (D₂O 100 MHz): δ 41.3 (N⁺CH₂CH₂OH), δ 57.6 (N⁺CH₂CH₂OH), δ 128.3 (CHCHCHCCOO⁻), δ 128.8 (CHCHCCOO⁻), δ 131.2 (CCOO), δ 136.3 (CHCHCHCCOO⁻), δ 175.7 (COO⁻).

### <Working example 57> Synthesis of compound 57

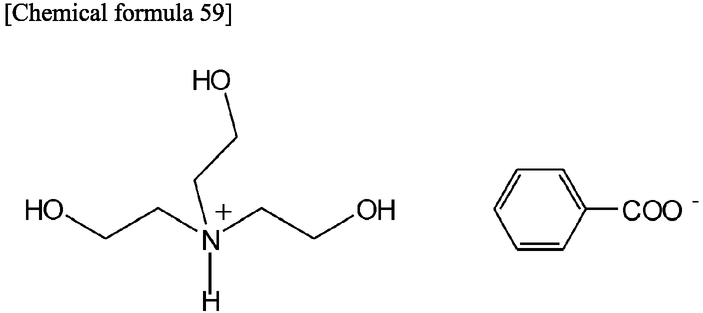

FT-IR(KBr): 3,360 cm⁻¹: O-H stretching vibration 2,950 cm⁻¹: C-H stretching vibration 1,558 cm⁻¹: COO⁻ stretching vibration

¹H-NMR (D₂O 400 MHz): δ 3.31(t, 6H, N⁺CH₂CH₂OH), δ 3.85 (t, 6H, N⁺CH₂CH₂OH), δ 7.40-7.50 (m, 3H, CHCHCHCHCCOO-), δ 7.51-7.80 (m, 2H, CHCCOOH).

¹³C-NMR (D₂O 100 MHz): δ 25.5 (CH₂CH₂CHCOO⁻), δ 55.4 (N⁺CH₂CH₂OH), δ 55.6 (N⁺CH₂CH₂OH), δ 128.3 (CHCHCHCCOO⁻), δ 128.8 (CHCHCCOO⁻), δ 131.2 (CCOO-), δ 136.3 (CHCHCHCCOO⁻), δ 175.7 (COO-).

### <Working example 58> Synthesis of compound 58

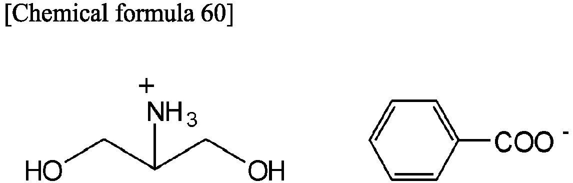

FT-IR(KBr): 3,388 cm⁻¹: O-H stretching vibration 2,958 cm⁻¹: C-H stretching vibration 1,558 cm⁻¹: COO⁻ stretching vibration

¹H-NMR (D₂O 400 MHz): δ 3.29-3.35 (m, 1H, HOCH₂CH(N⁺H₃)CH₂OH), δ 3.58-3.74 (m, 4H, HOCH₂CH(N⁺H₃)CH₂OH), δ 7.40-7.50 (m, 3H, CHCHCHCHCCOO⁻), δ 7.51-7.80 (m, 2H, CHCCOOH).

¹³C-NMR (D₂O 100 MHz): δ 54.1 (HOCH₂CH(N⁺H₃)CH₂OH), δ 58.6 (HOCH₂CH(N⁺H₃)CH₂OH), δ 128.3 (CHCHCHCCOO⁻), δ 128.8 (CHCHCCOO⁻), δ 131.2 (CCOO), δ 136.3 (CHCHCHCCOO⁻), δ 175.7 (COO-).

### <Working example 59> Synthesis of compound 59

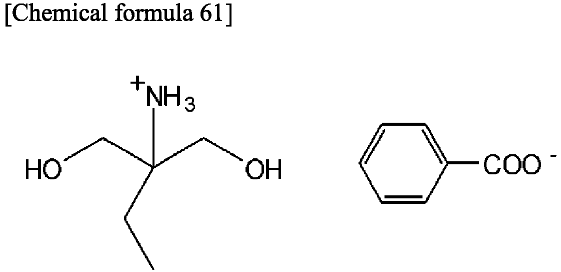

FT-IR(KBr): 3,388 cm⁻¹: O-H stretching vibration 2,922 cm⁻¹: C-H stretching vibration 1,543 cm⁻¹: COO⁻ stretching vibration

¹H-NMR (D₂O 400 MHz): δ 0.73-0.77 (m, 3H, NH₃⁺C(CH₂OH)₂CH₂CH₃), δ 1.50-1.56 (m, 2H, NH₃⁺C(CH₂OH)₂CH₂CH₃), δ 3.49-3.50 (m, 4H, NH₃⁺C(CH₂OH)₂CH₂CH₃), δ 7.40-7.50 (m, 3H, CHCHCHCHCCOO⁻), δ 7.51-7.80 (m, 2H, CHCCOOH).

¹³C-NMR (D₂O 100 MHz): δ 6.3 (NH₃⁺C(CH₂OH)₂CH₂CH₃), δ 23.2 (NH₃⁺C(CH₂OH)₂CH₂CH₃), δ 60.5 (NH₃⁺C(CH₂OH)₂CH₂CH₃), δ 63.3 (NH₃⁺C(CH₂OH)₂CH₂CH₃), δ 128.3 (CHCHCHCCOO⁻), δ 128.8 (CHCHCCOO-), δ 131.2 (CCOO-), δ 136.3 (CHCHCHCCOO-), δ 175.7 (COO⁻).

### <Working example 60> Synthesis of compound 60

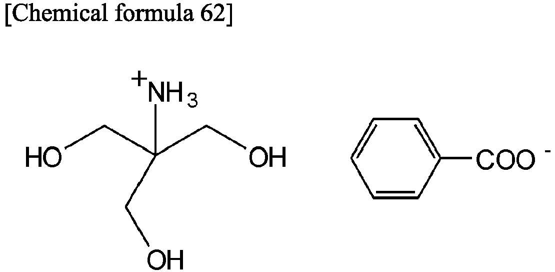

FT-IR(KBr): 3,145 cm⁻¹: O-H stretching vibration 2,946 cm⁻¹: C-H stretching vibration 1,572 cm⁻¹: COO⁻ stretching vibration

¹H-NMR (D₂O 400 MHz): δ 3.57 (s, 6H, NH₃⁺C(CH₂OH)₃), δ 7.40-7.50 (m, 3H, CHCHCHCHCCOO⁻), δ 7.51-7.80 (m, 2H, CHCCOOH).

¹³C-NMR (D₂O 100 MHz): δ 59.2 (NH₃⁺C(CH₂OH)₃), δ 61.4 (NH₃⁺C(CH₂OH)₃), δ 128.3 (CHCHCHCCOO⁻), δ 128.8 (CHCHCCOO⁻), δ 131.2 (CCOO), δ 136.3 (CHCHCHCCOO⁻), δ 175.7 (COO⁻).

### <Working example 61> Synthesis of compound 61

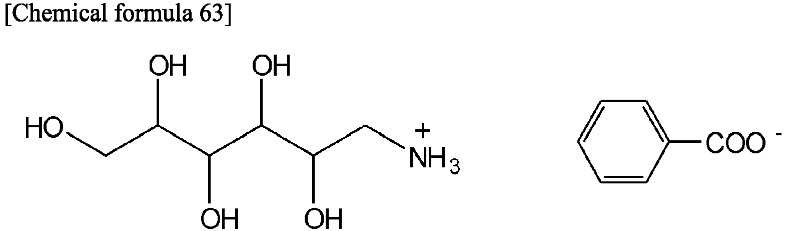

FT-IR(KBr): 3,167 cm⁻¹: O-H stretching vibration 2,920 cm⁻¹: C-H stretching vibration 1,573 cm⁻¹: COO⁻ stretching vibration

¹H-NMR (D₂O 400 MHz): δ 2.94-3.13 (m, 2H, HOCH₂(CH(OH))₃CH(OH)CH₂NH₃⁺), δ 3.51-3.72 (m, 5H, HOCH₂(CH(OH))₃CH(OH)CH₂NH₃⁺), δ 3.89-3.96 (m, 1H, HOCH₂(CH(OH))₃CH(OH)CH₂NH₃⁺), δ 7.40-7.50 (m, 3H, CHCHCHCHCCOO-), δ 7.51-7.80 (m, 2H, CHCCOOH).

¹³C-NMR (D₂O 100 MHz): δ 41.7 (HOCH₂(CH(OH))₃CH(OH)CH₂NH₃⁺), δ 62.6 (HOCH₂(CH(OH))₃CH(OH)CH₂NH₃⁺), δ 68.9-70.9 (HOCH₂(CH(OH))₃CH(OH)CH₂NH₃⁺), δ 128.3 (CHCHCHCCOO⁻), δ 128.8 (CHCHCCOO⁻), δ 131.2 (CCOO⁻), δ 136.3 (CHCHCHCCOO⁻), δ 175.7 (COO⁻).

### <Working example 62> Synthesis of compound 62

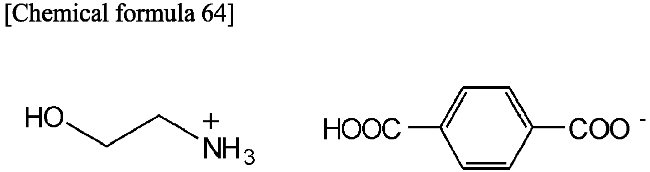

FT-IR(KBr): 3,162 m⁻¹: O-H stretching vibration 2,950 cm⁻¹: C-H stretching vibration 1,715 cm⁻¹: COOH stretching vibration 1,578 cm⁻¹: COO⁻ stretching vibration

¹H-NMR (D₂O 400 MHz): δ 3.08 (t, 2H, N⁺CH₂CH₂OH), δ 3.76 (t, 2H, N⁺CH₂CH₂OH), δ 8.34 (m, 4H, CHCCOO⁻).

¹³C-NMR (D₂O 100 MHz): δ 41.3 (N⁺CH₂CH₂OH), δ 57.6 (N⁺CH₂CH₂OH), δ 128.8 (CHCHCCOO⁻), δ 131.2 (CCOO), δ 175.7 (COO⁻).

### <Working example 63> Synthesis of compound 63

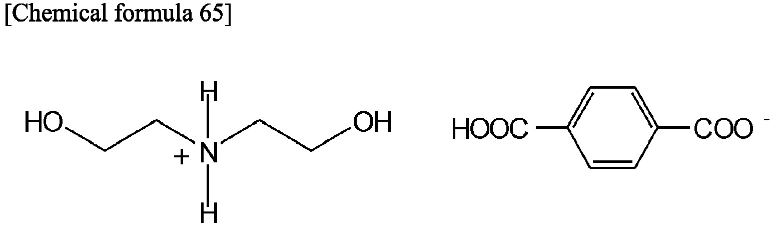

FT-IR(KBr): 3,306 cm⁻¹: O-H stretching vibration 2,923 cm⁻¹: C-H stretching vibration 1,715 cm⁻¹: COOH stretching vibration 1,559 cm⁻¹: COO⁻ stretching vibration

¹H-NMR (D₂O 400 MHz): δ 3.15 (t, 4H, N⁺CH₂CH₂OH), δ 3.79 (t, 4H, N⁺CH₂CH₂OH), δ 8.34 (m, 4H, CHCCOO⁻).

¹³C-NMR (D₂O 100 MHz): δ 48.9 (N⁺CH₂CH₂OH), δ 56.6 (N⁺CH₂CH₂OH), δ 128.8 (CHCHCCOO⁻), δ 131.2 (CCOO⁻), δ 175.7 (COO⁻).

### <Working example 64> Synthesis of compound 64

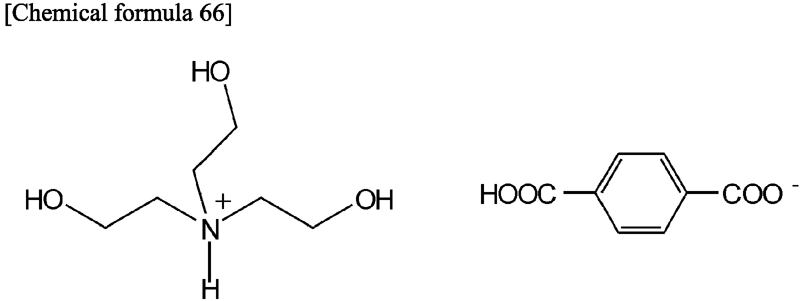

FT-IR(KBr): 3,360 cm⁻¹: O-H stretching vibration 2,950 cm⁻¹: C-H stretching vibration 1,715 cm⁻¹: COOH stretching vibration 1,558 cm⁻¹: COO⁻ stretching vibration

¹H-NMR (D₂O 400 MHz): δ 3.31(t, 6H, N⁺CH₂CH₂OH),δ 3.85 (t, 6H, N⁺CH₂CH₂OH), δ 8.34 (m, 4H, CHCCOO⁻).

¹³C-NMR (D₂O 100 MHz): δ 55.4 (N⁺CH₂CH₂OH), δ 55.6 (N⁺CH₂CH₂OH), δ 128.8 (CHCHCCOO⁻), δ 131.2 (CCOO⁻),δ 175.7 (COO-).

### <Working example 65> Synthesis of compound 65

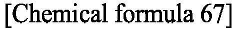

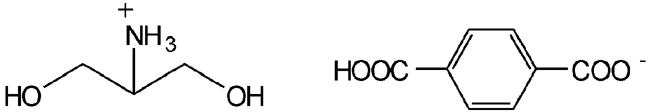

FT-IR(KBr): 3,388 cm⁻¹: O-H stretching vibration 2,958 cm⁻¹: C-H stretching vibration 1,714 cm⁻¹: COOH stretching vibration 1,558 cm⁻¹: COO⁻ stretching vibration

¹H-NMR (D₂O 400 MHz): δ 3.29-3.35 (m, 1H, HOCH₂CH(N⁺H₃)CH₂OH), δ 3.58-3.74 (m, 4H, HOCH₂CH(N⁺H₃)CH₂OH), δ 8.34 (m, 4H, CHCCOO⁻).

¹³C-NMR (D₂O 100 MHz): δ 54.1 (HOCH₂CH(N⁺H₃)CH₂OH), δ 58.6 (HOCH₂CH(N⁺H₃)CH₂OH), δ 128.8 (CHCHCCOO⁻), δ 131.2 (CCOO), δ 175.7 (COO⁻).

### <Working example 66> Synthesis of compound 66

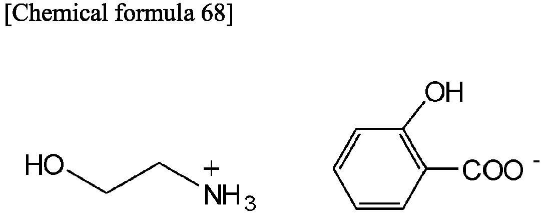

FT-IR(KBr): 3,162 m⁻¹: O-H stretching vibration 2,950 cm⁻¹: C-H stretching vibration 1,578 cm⁻¹: COO⁻ stretching vibration

¹H-NMR (D₂O 400 MHz): δ 3.08 (t, 2H, N⁺CH₂CH₂OH), δ 3.76 (t, 2H, N⁺CH₂CH₂OH), δ 6.85 (m, 2H, C(OH)CHCH, C(COO⁻)CHCHCH), δ 7.35 (m, 1H, CHCHC(OH)), δ 7.71 (m, 1H, C(COOH)CHCH).

¹³C-NMR (D₂O 100 MHz): δ 41.3 (N⁺CH₂CH₂OH), δ 57.6 (N⁺CH₂CH₂OH), δ 116.3 (C(OH)CHCH), δ 118.0 (CHC(COO⁻)C(OH)), δ 119.4 (C(COO⁻)CHCHCH), δ 130.5 (C(COO⁻)CHCH), δ 134.0 (CHCHC(OH)), δ 159.6 (CC(OH)C), δ 175.5 (CCOO⁻).

### <Working example 67> Synthesis of compound 67

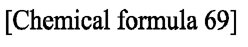

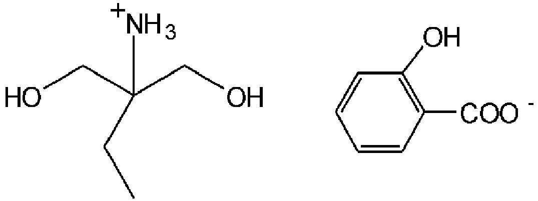

FT-IR(KBr): 3,388 cm⁻¹: O-H stretching vibration 2,922 cm⁻¹: C-H stretching vibration 1,543 cm⁻¹: COO⁻ stretching vibration

¹H-NMR (D₂O 400 MHz): δ 0.73-0.77 (m, 3H, NH₃⁺C(CH₂OH)₂CH₂CH₃), δ 1.50-1.56 (m, 2H, NH₃⁺C(CH₂OH)₂CH₂CH₃), δ 3.49-3.50 (m, 4H, NH₃⁺C(CH₂OH)₂CH₂CH₃), δ 6.85 (m, 2H, C(OH)CHCH, C(COO⁻)CHCHCH), δ 7.35 (m, 1H, CHCHC(OH)), δ 7.71 (m, 1H, C(COOH)CHCH).

¹³C-NMR (D₂O 100 MHz): δ 6.3 (NH₃⁺C(CH₂OH)₂CH₂CH₃), δ 23.2 (NH₃⁺C(CH₂OH)₂CH₂CH₃), δ 60.5 (NH₃⁺C(CH₂OH)₂CH₂CH₃), δ 63.3 (NH₃⁺C(CH₂OH)₂CH₂CH₃), δ 116.3 (C(OH)CHCH), δ 118.0 (CHC(COO⁻)C(OH)), δ 119.4 (C(COO⁻)CHCHCH), δ 130.5 (C(COO⁻)CHCH), δ 134.0 (CHCHC(OH)), δ 159.6 (CC(OH)C), δ 175.5 (CCOO⁻).

### <Working example 68> Synthesis of compound 68

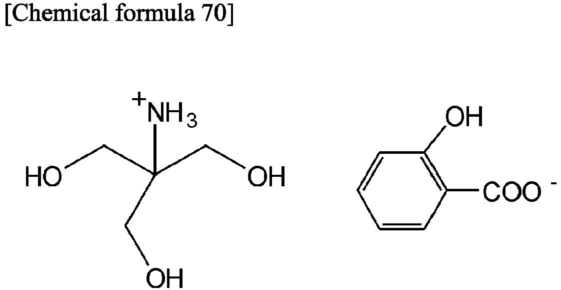

FT-IR(KBr): 3,145 cm⁻¹: O-H stretching vibration 2,946 cm⁻¹: C-H stretching vibration 1,572 cm⁻¹: COO⁻ stretching vibration

¹H-NMR (D₂O 400 MHz): δ 3.57 (s, 6H, NH₃⁺C(CH₂OH)₃), δ 6.85 (m, 2H, C(OH)CHCH, C(COO-)CHCHCH), δ 7.35 (m, 1H, CHCHC(OH)), δ 7.71 (m, 1H, C(COOH)CHCH).

¹³C-NMR (D₂O 100 MHz): δ 59.2 (NH₃⁺C(CH₂OH)₃), δ 61.4 (NH₃⁺C(CH₂OH)₃), δ 116.3 (C(OH)CHCH), δ 118.0 (CHC(COO⁻)C(OH)), δ 119.4 (C(COO⁻)CHCHCH), δ 130.5 (C(COO⁻)CHCH), δ 134.0 (CHCHC(OH)), δ 159.6 (CC(OH)C), δ 175.5 (CCOO⁻).

### <Working example 69> Synthesis of compound 69

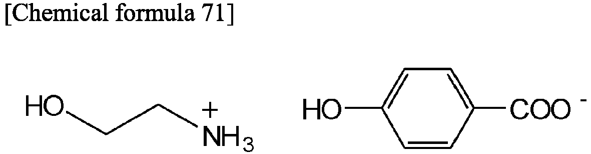

FT-IR(KBr): 3,162 m⁻¹: O-H stretching vibration 2,950 cm⁻¹: C-H stretching vibration 1,578 cm⁻¹: COO⁻ stretching vibration

¹H-NMR (D₂O 400 MHz): δ 3.08 (t, 2H, N⁺CH₂CH₂OH), δ 3.76 (t, 2H, N⁺CH₂CH₂OH), δ 6.83 (d, 2H, C(COO-)CHCH), δ 7.73 (d, 2H, C(OH)CHCH).

¹³C-NMR (D₂O 100 MHz): δ 41.3 (N⁺CH₂CH₂OH), δ 57.6 (N⁺CH₂CH₂OH), δ 114.9 (C(OH)CHCH), δ 128.2 (C(COO⁻)), δ 131.2 (C(COO⁻)CHCH), δ 158.4 (C(OH)), δ 175.3 (C(COO⁻)).

### <Working example 70> Synthesis of compound 70

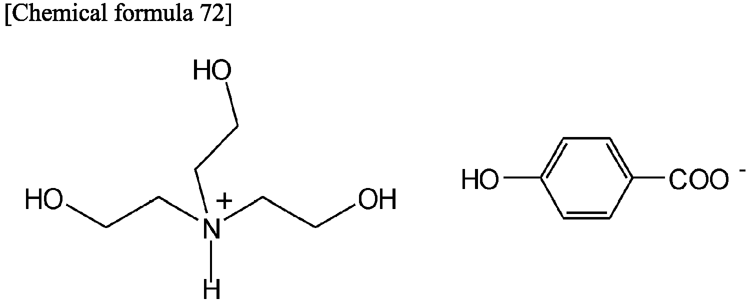

FT-IR(KBr): 3,360 cm⁻¹: O-H stretching vibration 2,950 cm⁻¹: C-H stretching vibration 1,558 cm⁻¹: COO⁻ stretching vibration

¹H-NMR (D₂O 400 MHz): δ 3.31(t, 6H, N⁺CH₂CH₂OH),δ 3.85 (t, 6H, N⁺CH₂CH₂OH), δ 6.83 (d, 2H, C(COO⁻)CHCH), δ 7.73 (d, 2H, C(OH)CHCH).

¹³C-NMR (D₂O 100 MHz): δ 55.4 (N⁺CH₂CH₂OH), δ 55.6 (N⁺CH₂CH₂OH), δ 114.9 (C(OH)CHCH), δ 128.2 (C(COO-)), δ 131.2 (C(COO⁻)CHCH), δ 158.4 (C(OH)), δ 175.3 (C(COO⁻)).

### <Working example 71> Synthesis of compound 71

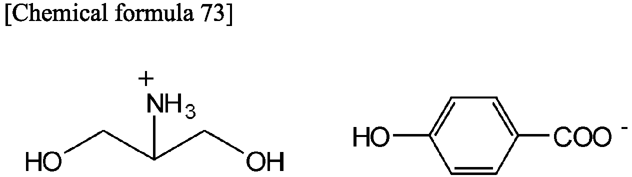

FT-IR(KBr): 3,388 cm⁻¹: O-H stretching vibration 2,958 cm⁻¹: C-H stretching vibration 1,558 cm⁻¹: COO⁻ stretching vibration

¹H-NMR (D₂O 400 MHz): δ 3.29-3.35 (m, 1H, HOCH₂CH(N⁺H₃)CH₂OH), δ 3.58-3.74 (m, 4H, HOCH₂CH(N⁺H₃)CH₂OH), δ 6.83 (d, 2H, C(COO⁻)CHCH), δ 7.73 (d, 2H, C(OH)CHCH).

¹³C-NMR (D₂O 100 MHz): δ 54.1 (HOCH₂CH(N⁺H₃)CH₂OH), δ 58.6 (HOCH₂CH(N⁺H₃)CH₂OH), δ 114.9 (C(OH)CHCH) , δ 128.2 (C(COO⁻)) , δ 131.2 (C(COO⁻)CHCH) , δ 158.4 (C(OH)) , δ 175.3 (C(COO⁻)).

### <Working example 72> Synthesis of compound 72

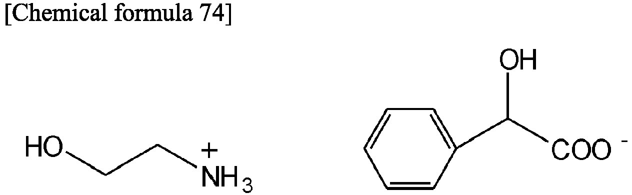

FT-IR(KBr): 3,162 m⁻¹: O-H stretching vibration 2,950 cm⁻¹: C-H stretching vibration 1,578 cm⁻¹: COO⁻ stretching vibration

¹H-NMR (D₂O 400 MHz): δ 3.08 (t, 2H, N⁺CH₂CH₂OH), δ 3.76 (t, 2H, N⁺CH₂CH₂OH), δ 4.94 (s, 1H, CH(OH)(COO⁻)), δ 7.34 (m, 5H, (CH)₅).

¹³C-NMR (D₂O 100 MHz): δ 41.3 (N⁺CH₂CH₂OH), δ 57.6 (N⁺CH₂CH₂OH), δ 75.0 (CH(OH)(COO⁻)) , δ 127.1 ((CH)₂CH(CH)₂) , δ 128.2 (CHCHCCH(OH)(COO⁻)) , δ 128.8 (CHCCH(OH)(COO⁻)) , δ 140.6 (CCH(OH)(COO⁻)) , δ 179.4 (COO⁻).

### <Working example 73> Synthesis of compound 73

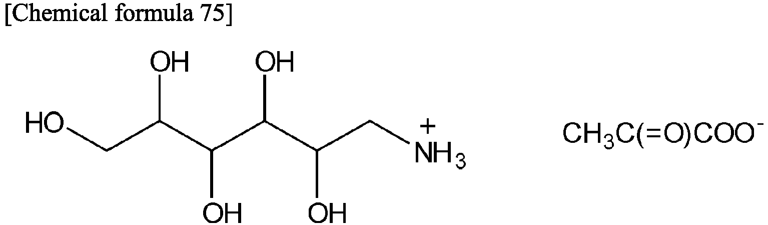

FT-IR(KBr): 3,167 cm⁻¹: O-H stretching vibration 2,920 cm⁻¹: C-H stretching vibration 1,573 cm⁻¹: COO⁻ stretching vibration

¹H-NMR (D₂O 400 MHz): δ 2.53 (d, 3H, CH₃), δ 2.94-3.13 (m, 2H, HOCH₂(CH(OH))₃CH(OH)CH₂NH₃⁺), δ 3.51-3.72 (m, 5H, HOCH₂(CH(OH))₃CH(OH)CH₂NH₃⁺), δ 3.89-3.96 (m, 1H, HOCH₂(CH(OH))₃CH(OH)CH₂NH₃⁺).

¹³C-NMR (D₂O 100 MHz): δ 25.5 (CH₃), δ 41.7 (HOCH₂(CH(OH))₃CH(OH)CH₂NH₃⁺), δ 62.6 (HOCH₂(CH(OH))₃CH(OH)CH₂NH₃⁺), δ 68.9-70.9 (HOCH₂(CH(OH))₃CH(OH)CH₂NH₃⁺), δ 161.4 (COO⁻), δ 193.7 (CH₃C=O).

### <Working example 74> Synthesis of compound 74

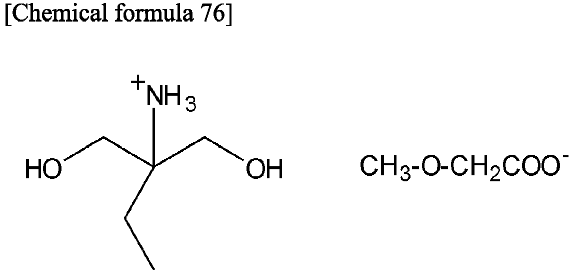

FT-IR(KBr): 3,388 cm⁻¹: O-H stretching vibration 2,922 cm⁻¹: C-H stretching vibration 1,543 cm⁻¹: COO⁻ stretching vibration

¹H-NMR (D₂O 400 MHz): δ 0.73-0.77 (m, 3H, NH₃⁺C(CH₂OH)₂CH₂CH₃), δ 1.50-1.56 (m, 2H, NH₃⁺C(CH₂OH)₂CH₂CH₃), δ 3.27 (s, 3H, CH₃OCH₂COO⁻), δ 3.49-3.50 (m, 4H, NH₃⁺C(CH₂OH)₂CH₂CH₃), δ 3.77 (s, 2H, CH₃OCH₂COO⁻).

¹³C-NMR (D₂O 100 MHz): δ 6.3 (NH₃⁺C(CH₂OH)₂CH₂CH₃), δ 23.2 (NH₃⁺C(CH₂OH)₂CH₂CH₃), δ 57.6 (CH₃OCH₂COO⁻), δ 60.5 (NH₃⁺C(CH₂OH)₂CH₂CH₃), δ 63.3 (NH₃⁺C(CH₂OH)₂CH₂CH₃), δ 71.2 (CH₃OCH₂COO⁻), δ 178.0 (CH₃OCH₂COO⁻).

### <Working example 75> Synthesis of compound 75

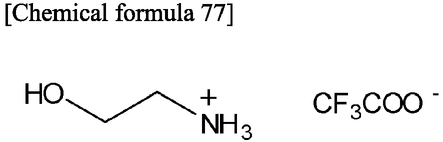

FT-IR(KBr): 3,313 m⁻¹: O-H stretching vibration 2,950 cm⁻¹: C-H stretching vibration 1,679 cm⁻¹: COO⁻ stretching vibration

¹H-NMR (D₂O 400 MHz): δ 3.08 (t, 2H, N⁺CH₂CH₂OH),δ 3.76 (t, 2H, NCH₂CH₂OH).

¹³C-NMR (D₂O 100 MHz):δ 41.3 (N⁺CH₂CH₂OH), δ 57.6 (N⁺CH₂CH₂OH), δ 114.6 (CF₃COO⁻), δ 165.1 (CF₃COO⁻).

### <Working example 76> Synthesis of compound 76

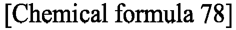

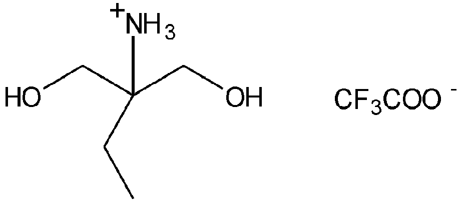

FT-IR(KBr): 3,332 m⁻¹: O-H stretching vibration 2,950 cm⁻¹: C-H stretching vibration 1,665 cm⁻¹: COO⁻ stretching vibration

¹H-NMR (D₂O 400 MHz): δ 0.73-0.77 (m, 3H, NH₃⁺C(CH₂OH)₂CH₂CH₃), δ 1.50-1.56 (m, 2H, NH₃⁺C(CH₂OH)₂CH₂CH₃), δ 3.49-3.50 (m, 4H, NH₃⁺C(CH₂OH)₂CH₂CH₃).

¹³C-NMR (D₂O 100 MHz): δ 6.3 (NH₃⁺C(CH₂OH)₂CH₂CH₃), δ 23.2 (NH₃⁺C(CH₂OH)₂CH₂CH₃), δ 60.5 (NH₃⁺C(CH₂OH)₂CH₂CH₃), δ 63.3 (NH₃⁺C(CH₂OH)₂CH₂CH₃), δ 114.6 (CF₃COO⁻), δ 165.1 (CF₃COO⁻).

### <Working example 77> Synthesis of compound 77

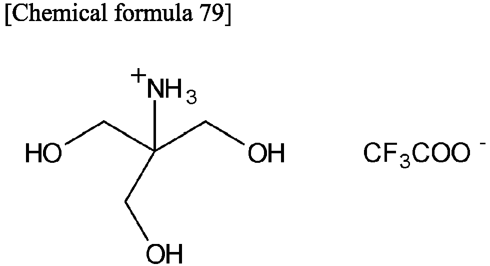

FT-IR(KBr): 3,332 m⁻¹: O-H stretching vibration 2,950 cm⁻¹: C-H stretching vibration 1,665 cm⁻¹: COO⁻ stretching vibration

¹H-NMR (D₂O 400 MHz): δ 3.57 (s, 6H, NH₃⁺C(CH₂OH)₃).

¹³C-NMR (D₂O 100 MHz): δ 59.2 (NH₃⁺C(CH₂OH)₃), δ 61.4 (NH₃⁺C(CH₂OH)₃), δ 114.6 (CF₃COO⁻), δ 165.1 (CF₃COO⁻).

### <Working example 78> Synthesis of compound 78

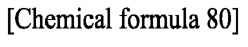

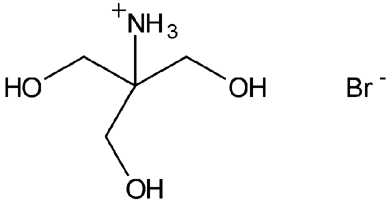

FT-IR(KBr): 3,388 cm⁻¹: O-H stretching vibration 2,959 cm⁻¹: C-H stretching vibration ¹H-NMR (D₂O 400 MHz): δ 3.65 (s, 6H, NH₃⁺C(CH₂OH)₃).

¹³C-NMR (D₂O 100 MHz): δ 59.4 (NH₃⁺C(CH₂OH)₃), δ 61.4 (NH₃⁺C(CH₂OH)₃).

### <Working example 79> Synthesis of compound 79

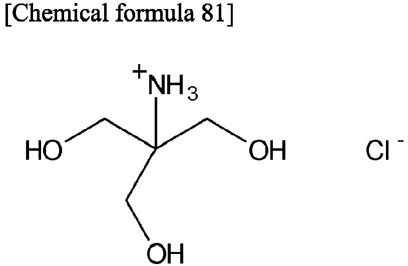

FT-IR(KBr): 3,388 cm⁻¹: O-H stretching vibration 2,959 cm⁻¹: C-H stretching vibration ¹H-NMR (D₂O 400 MHz): δ 3.65 (s, 6H, NH₃⁺C(CH₂OH)₃).

¹³C-NMR (D₂O 100 MHz): δ 59.4 (NH₃⁺C(CH₂OH)₃), δ 61.4 (NH₃⁺C(CH₂OH)₃).

### <Working example 80> Synthesis of compound 80

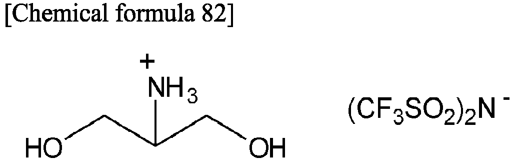

FT-IR(KBr): 3,388 cm⁻¹: O-H stretching vibration 2,959 cm⁻¹: C-H stretching vibration ¹H-NMR (D₂O 400 MHz): δ 3.35-3.41 (m, 1H, HOCH₂CH(N⁺H₃)CH₂OH), δ 3.64-3.80 (m, 4H, HOCH₂CH(N⁺H₃)CH₂OH).

¹³C-NMR (D₂O 100 MHz): δ 54.2 (HOCH₂CH(N⁺H₃)CH₂OH), δ 58.7 (HOCH₂CH(N⁺H₃)CH₂OH).

### <Working example 81> Synthesis of compound 81

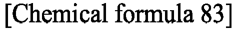

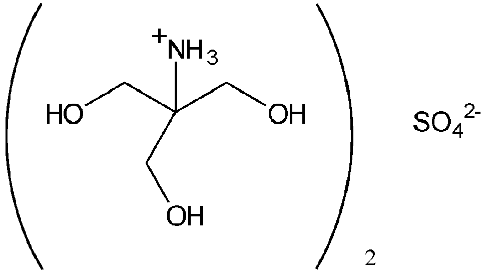

FT-IR(KBr): 3,388 cm⁻¹: O-H stretching vibration 2,959 cm⁻¹: C-H stretching vibration ¹H-NMR (D₂O 400 MHz): δ 3.65 (s, 6H, NH₃⁺C(CH₂OH)₃).

¹³C-NMR (D₂O 100 MHz): δ 59.4 (NH₃⁺C(CH₂OH)₃), δ 61.4 (NH₃⁺C(CH₂OH)₃).

### <Working example 82> Synthesis of compound 82

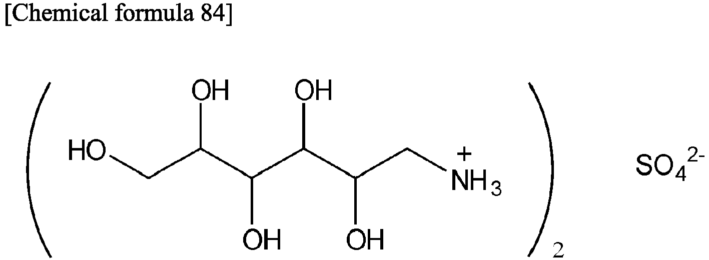

FT-IR(KBr): 3,388 cm⁻¹: O-H stretching vibration 2,959 cm⁻¹: C-H stretching vibration ¹H-NMR (D₂O 400 MHz): δ 2.86-3.05 (m, 2H, HOCH₂(CH(OH))₃CH(OH)CH₂NH₃⁺), δ 3.41-3.68 (m, 5H, HOCH₂(CH(OH))₃CH(OH)CH₂NH₃⁺), δ 3.83-3.88 (m, 1H, HOCH₂(CH(OH))₃CH(OH)CH₂NH₃⁺).

¹³C-NMR (D₂O 100 MHz): δ 41.7 (HOCH₂(CH(OH))₃CH(OH)CH₂NH₃⁺), δ 62.6 (HOCH₂(CH(OH))₃CH(OH)CH₂NH₃⁺), δ 69.2-70.8 (HOCH₂(CH(OH))₃CH(OH)CH₂NH₃⁺).

### <Working example 83> Synthesis of compound 83

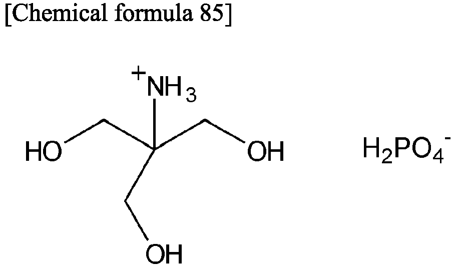

FT-IR(KBr): 3,388 cm⁻¹: O-H stretching vibration 2,959 cm⁻¹: C-H stretching vibration ¹H-NMR (D₂O 400 MHz): δ 3.65 (s, 6H, NH₃⁺C(CH₂OH)₃).

¹³C-NMR (D₂O 100 MHz): δ 59.4 (NH₃⁺C(CH₂OH)₃), δ 61.4 (NH₃⁺C(CH₂OH)₃).

### <Working example 84> Synthesis of compound 84

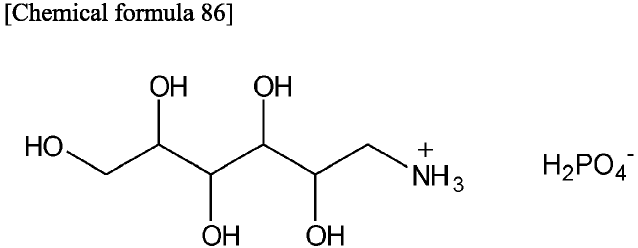

FT-IR(KBr): 3,388 cm⁻¹: O-H stretching vibration 2,959 cm⁻¹: C-H stretching vibration ¹H-NMR (D₂O 400 MHz): δ 2.86-3.05 (m, 2H, HOCH₂(CH(OH))₃CH(OH)CH₂NH₃⁺), δ 3.41-3.68 (m, 5H, HOCH₂(CH(OH))₃CH(OH)CH₂NH₃⁺), δ 3.83-3.88 (m, 1H, HOCH₂(CH(OH))₃CH(OH)CH₂NH₃⁺).

¹³C-NMR (D₂O 100 MHz): δ 41.7 (HOCH₂(CH(OH))₃CH(OH)CH₂NH₃⁺), δ 62.6 (HOCH₂(CH(OH))₃CH(OH)CH₂NH₃⁺), δ 69.2-70.8 (HOCH₂(CH(OH))₃CH(OH)CH₂NH₃⁺).

### <Working example 85> Synthesis of compound 85

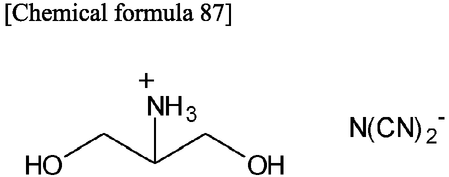

FT-IR(KBr): 3,388 cm⁻¹: O-H stretching vibration 2,959 cm⁻¹: C-H stretching vibration ¹H-NMR (D₂O 400 MHz): δ 3.35-3.41 (m, 1H, HOCH₂CH(N⁺H₃)CH₂OH), δ 3.64-3.80 (m, 4H, HOCH₂CH(N⁺H₃)CH₂OH).

¹³C-NMR (D₂O 100 MHz): δ 54.2 (HOCH₂CH(N⁺H₃)CH₂OH), δ 58.7 (HOCH₂CH(N⁺H₃)CH₂OH).

### <Working example 86> Synthesis of compound 85

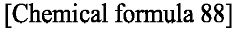

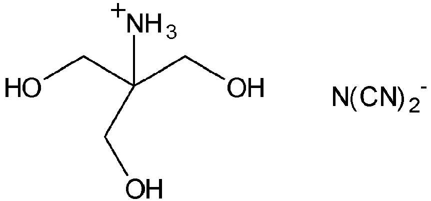

FT-IR(KBr): 3,388 cm⁻¹: O-H stretching vibration 2,959 cm⁻¹: C-H stretching vibration ¹H-NMR (D₂O 400 MHz): δ 3.65 (s, 6H, NH₃⁺C(CH₂OH)₃).

¹³C-NMR (D₂O 100 MHz): δ 59.4 (NH₃⁺C(CH₂OH)₃), δ 61.4 (NH₃⁺C(CH₂OH)₃).

### <Working example 87> Synthesis of compound 87

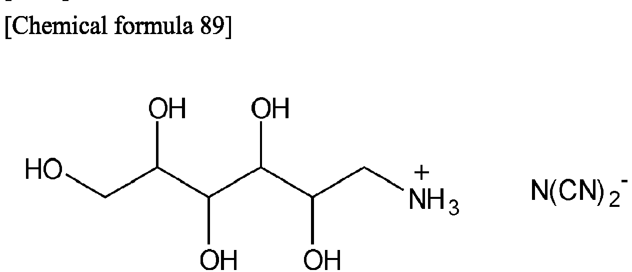

FT-IR(KBr): 3,388 cm⁻¹: O-H stretching vibration 2,959 cm⁻¹: C-H stretching vibration ¹H-NMR (D₂O 400 MHz): δ 2.86-3.05 (m, 2H, HOCH₂(CH(OH))₃CH(OH)CH₂NH₃⁺), δ 3.41-3.68 (m, 5H, HOCH₂(CH(OH))₃CH(OH)CH₂NH₃⁺), δ 3.83-3.88 (m, 1H, HOCH₂(CH(OH))₃CH(OH)CH₂NH₃⁺).

¹³C-NMR (D₂O 100 MHz): δ 41.7 (HOCH₂(CH(OH))₃CH(OH)CH₂NH₃⁺), δ 62.6 (HOCH₂(CH(OH))₃CH(OH)CH₂NH₃⁺), δ 69.2-70.8 (HOCH₂(CH(OH))₃CH(OH)CH₂NH₃⁺).

### <Working example 88> Synthesis of compound 88

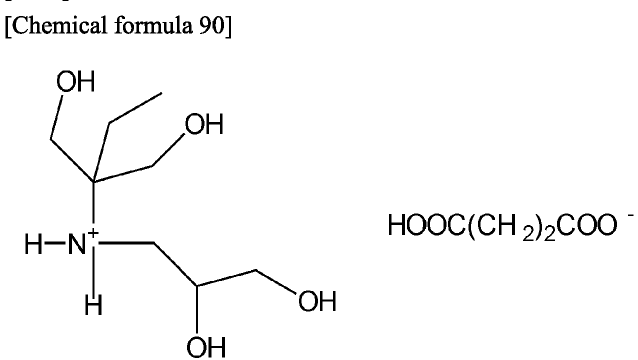

Here, 2-amino-2-ethyl-1,3-propanediol (25.00 g, 0.210 mol) and 3-chloro-1,2-propanediol (116.07 g, 1.050 mol) were reacted in 1,000 mL of 1-propanol under reflux for 48 hours, followed by distilling away 1-propanol under a reduced pressure. THF was then added to a liquid thus obtained before performing washing with heating, thus obtaining a white powder. Sodium hydroxide was added to the white powder thus obtained so as to then stir them under room temperature for two hours. Next, ethanol was added thereto, followed by filtering away a crystal precipitated, and then distilling away the filtrate under a reduced pressure. A liquid thus obtained was then purified by column chromatography to obtain an amine-based compound 1 shown in the working example 88 in Table 1.

The amine-based compound 1 (2.50 g, 0.013 mol) and succinic acid (1.53 g, 0.013 mmol) were reacted in 50 mmL of water under room temperature for three hours, followed by distilling away water under a reduced pressure to obtain a white solid. By washing the solid thus obtained, a compound 88 as a white solid (ammonium succinate) was obtained. FT-IR(KBr): 3,162 m⁻¹: O-H stretching vibration 2,950 cm⁻¹: C-H stretching vibration 1,715 cm⁻¹: COOH stretching vibration 1,578 cm⁻¹: COO⁻ stretching vibration

¹H-NMR (D₂O 400 MHz): δ 0.86-0.92 (m, 3H, NH₂⁺C(CH₂OH)₂CH₂CH₃), δ 1.44 (m, 2H, NH₂⁺C(CH₂OH)₂CH₂CH₃), δ 2.51 (s, 4H, HOOCCH₂CH₂COO⁻), δ 2.78-2.96 (m, 2H, NH₂⁺CH₂CH(OH)), δ 3.61-3.78 (m, 6H, NH₂⁺C(CH₂OH)₂CH₂CH₃, NH₂⁺CH₂CH(OH)CH₂OH), δ 3.83-3.88 (m, 1H, NH₂⁺CH₂CH(OH))

¹³C-NMR (D₂O 100 MHz): δ 9.6 (NH₂⁺C(CH₂OH)₂CH₂CH₃), δ 22.7 (CH₃CH₂CH₂), δ 24.6 (NH₂⁺C(CH₂OH)₂CH₂CH₃), δ 31.4 (HOOCCH₂CH₂COO⁻), δ 43.6 (NH₂⁺C(CH₂OH)₂CH₂CH₃), δ 45.8 (NH₂⁺CH₂CH(OH)), δ 60.2 (NH₂⁺C(CH₂OH)₂CH₂CH₃), δ 62.8 (NH₂⁺CH₂CH(OH)CH₂OH), δ 71.1 (NH₂⁺CH₂CH(OH)CH₂OH), δ 179.7 (COOH, COO⁻).

### <Working example 89> Synthesis of compound 89

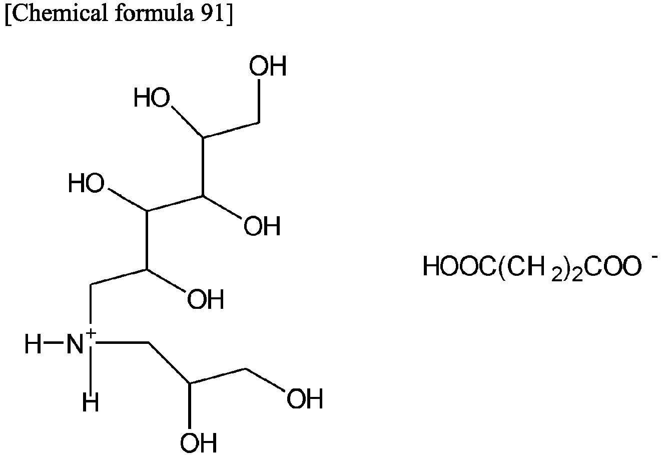

D-glucamine (25.00 g, 0.138 mol) and 3-chloro-1,2-propanediol (76.26 g, 0.690 mol) were reacted in 1,000 mL of 1-propanol under reflux for 48 hours, followed by distilling away 1-propanol under a reduced pressure. THF was then added to a liquid thus obtained before performing washing with heating, thus obtaining a white powder. Sodium hydroxide was added to the white powder thus obtained so as to then stir them under room temperature for two hours. Next, ethanol was added thereto, followed by filtering away a crystal precipitated, and then distilling away the filtrate under a reduced pressure. A liquid thus obtained was then purified by column chromatography to obtain an amine-based compound 2 shown in the working example 89 in Table 10.

The amine-based compound 2 (2.50 g, 0.010 mol) and succinic acid (1.16 g, 0.010 mol) were reacted in 50 mL of water under room temperature for three hours, followed by distilling away water under a reduced pressure to obtain a yellow solid. By washing the solid thus obtained, a compound 89 as a yellow solid (ammonium succinate) was obtained.

FT-IR(KBr): 3,162 m⁻¹: O-H stretching vibration 2,950 cm⁻¹: C-H stretching vibration 1,715 cm⁻¹: COOH stretching vibration 1,578 cm⁻¹: COO⁻ stretching vibration ¹H-NMR (D₂O 400 MHz): δ 2.51 (s, 4H, HOOCCH₂CH₂COO⁻), δ 3.00-3.23 (m, 4H, HOCH₂(CH(OH))₃CH(OH)CH₂NH₂⁺, NH₂⁺CH₂CH(OH)), δ 3.51-3.74 (m, 7H, HOCH₂(CH(OH))₃CH(OH)CH₂NH₂⁺, NH₂⁺CH₂CH(OH)CH₂OH), δ 3.94-3.98 (m, 1H, NH₂⁺CH₂CH(OH), δ 4.03-4.09 (m, 1H, HOCH₂(CH(OH))₃CH(OH)CH₂NH₂⁺).

¹³C-NMR (D₂O 100 MHz): δ 31.4 (HOOCCH₂CH₂COO⁻), δ 43.2 (HOCH₂(CH(OH))₃CH(OH)CH₂NH₂⁺), δ 45.8 (NH₂⁺CH₂CH(OH)), δ 62.8 (NH₂⁺CH₂CH(OH)CH₂OH), δ 64.7 (HOCH₂(CH(OH))₃CH(OH)CH₂NH₂⁺), δ 70.7-72.9 (HOCH₂(CH(OH))₃CH(OH)CH₂NH₃⁺, NH₂⁺CH₂CH(OH)CH₂OH), δ 179.7 (COOH, COO⁻).

### <Working example 90> Synthesis of compound 90

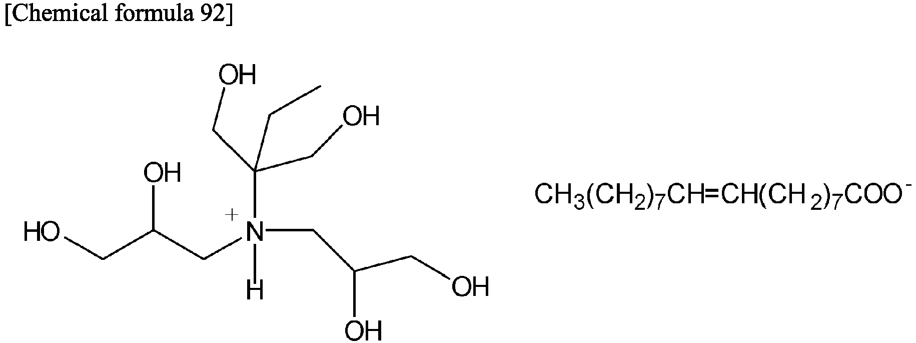

The amine-based compound 1 (10.00 g, 0.052 mol) and 3-chloro-1,2-propanediol (28.74 g, 0.260 mol) were reacted in 500 mL of 1-propanol under reflux for 48 hours, followed by distilling away 1-propanol under a reduced pressure. THF was then added to a liquid thus obtained before performing washing with heating, thus obtaining a white powder. Sodium hydroxide was added to the white powder thus obtained so as to then stir them under room temperature for two hours. Next, ethanol was added thereto, followed by filtering away a crystal precipitated, and then distilling away the filtrate under a reduced pressure. A liquid thus obtained was then purified by column chromatography to obtain an amine-based compound 3 shown in the working example 90 in Table 10.

The amine-based compound 3 (2.50 g, 0.013 mol) and oleic acid (1.53 g, 0.013 mmol) were reacted in 50 mL of water under room temperature for three hours, followed by distilling away water under a reduced pressure to obtain a white solid. By washing the solid thus obtained, a compound 90 as a white solid (ammonium oleate) was obtained.

FT-IR(KBr): 3,162 m⁻¹: O-H stretching vibration 2,950 cm⁻¹: C-H stretching vibration 1,578 cm⁻¹: COO⁻ stretching vibration ¹H-NMR (D₂O 400 MHz): δ 2.51 (s, 4H, HOOCCH₂CH₂COO⁻), δ 3.00-3.23 (m, 4H, HOCH₂(CH(OH))₃CH(OH)CH₂NH₂⁺, NH₂⁺CH₂CH(OH)), δ 3.51-3.74 (m, 7H, HOCH₂(CH(OH))₃CH(OH)CH₂NH₂⁺, NH₂⁺CH₂CH(OH)CH₂OH), δ 3.94-3.98 (m, 1H, NH₂⁺CH₂CH(OH), δ 4.03-4.09 (m, 1H, HOCH₂(CH(OH))₃CH(OH)CH₂NH₂⁺). δ 0.89 (t, 3H, CH₃CH₂), δ 1.27 (m, 20H, CH₃(CH₂)₆CH₂, (CH₂)₄ CH₂CH₂COO⁻), δ 1.53 (m, 2H, CH₂CH₂COO⁻), δ 2.00 (m, 4H, CH₂CH=CHCH₂), δ 2.14 (t, 2H, CH₂CH₂COO⁻), δ 5.32 (m, 2H, CH=CH).

¹³C-NMR (D₂O 100 MHz): δ 31.4 (HOOCCH₂CH₂COO⁻), δ 43.2 (HOCH₂(CH(OH))₃CH(OH)CH₂NH₂⁺), δ 45.8 (NH₂⁺CH₂CH(OH)), δ 62.8 (NH₂⁺CH₂CH(OH)CH₂OH), δ 64.7 (HOCH₂(CH(OH))₃CH(OH)CH₂NH₂⁺), δ 70.7-72.9 (HOCH₂(CH(OH))₃CH(OH)CH₂NH₃⁺, NH₂⁺CH₂CH(OH)CH₂OH). δ 14.0 (CH₃CH₂), δ 22.6 (CH₃CH₂), δ 26.4 (CH₂CH₂COO⁻), δ 27.2 (CH₂CH=CHCH₂), δ 29.3 (CH₃CH₂CH₂(CH₂)₄, (CH₂)₄CH₂CH₂COO⁻), δ 31.9 (CH₃CH₂CH₂), δ 37.8 (CH₂CH₂COO⁻), δ 129.7 (CH=CH), δ 181.7 (COO⁻).

### <Working example 91> Synthesis of compound 91

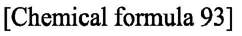

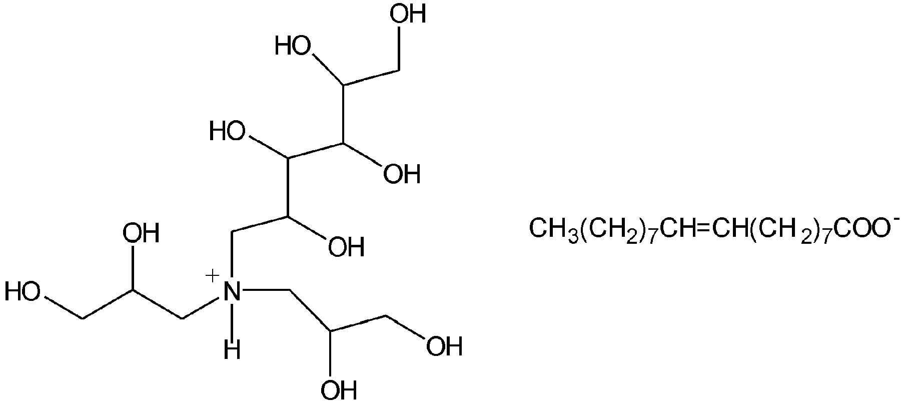

The amine-based compound 2 (10.00 g, 0.039 mol) and 3-chloro-1,2-propanediol (21.56 g, 0.195 mol) were reacted in 500 mL of 1-propanol under reflux for 48 hours, followed by distilling away 1-propanol under a reduced pressure. THF was then added to a liquid thus obtained before performing washing with heating, thus obtaining a white powder. Sodium hydroxide was added to the white powder thus obtained so as to then stir them under room temperature for two hours. Next, ethanol was added thereto, followed by filtering away a crystal precipitated, and then distilling away the filtrate under a reduced pressure. A liquid thus obtained was then purified by column chromatography to obtain an amine-based compound 4 shown in the working example 91 in Table 10.

The amine-based compound 4 (2.50 g, 0.009 mol) and oleic acid (2.64 g, 0.009 mol) were reacted in 50 mL of water under room temperature for three hours, followed by distilling away water under a reduced pressure to obtain a yellow solid. By washing the solid thus obtained, a compound 91 as a yellow solid (ammonium oleate) was obtained.

FT-IR(KBr): 3,162 m⁻¹: O-H stretching vibration 2,950 cm⁻¹: C-H stretching vibration 1,578 cm⁻¹: COO⁻ stretching vibration ¹H-NMR (D₂O 400 MHz): δ 3.00-3.23 (m, 6H, HOCH₂(CH(OH))₃CH(OH)CH₂NH⁺, NH⁺CH₂CH(OH)), δ 3.51-3.74 (m, 9H, HOCH₂(CH(OH))₃CH(OH)CH₂NH⁺, NH⁺CH₂CH(OH)CH₂OH), δ 3.94-3.98 (m, 2H, NH⁺CH₂CH(OH), δ 4.03-4.09 (m, 2H, HOCH₂(CH(OH))₃CH(OH)CH₂NH⁺). δ 0.89 (t, 3H, CH₃CH₂), δ 1.27 (m, 20H, CH₃(CH₂)₆CH₂, (CH₂)₄ CH₂CH₂COO⁻), δ 1.53 (m, 2H, CH₂CH₂COO⁻), δ 2.00 (m, 4H, CH₂CH=CHCH₂), δ 2.14 (t, 2H, CH₂CH₂COO⁻), δ 5.32 (m, 2H, CH=CH).

¹³C-NMR (D₂O 100 MHz): δ 43.2 (HOCH₂(CH(OH))₃CH(OH)CH₂NH⁺), δ 45.8 (NH⁺CH₂CH(OH)), δ 62.8 (NH⁺CH₂CH(OH)CH₂OH), δ 64.7 (HOCH₂(CH(OH))₃CH(OH)CH₂NH⁺), δ 70.7-72.9 (HOCH₂(CH(OH))₃CH(OH)CH₂NH⁺, NH⁺CH₂CH(OH)CH₂OH). δ 14.0 (CH₃CH₂), δ 22.6 (CH₃CH₂), δ 26.4 (CH₂CH₂COO⁻), δ 27.2 (CH₂CH=CHCH₂), δ 29.3 (CH₃CH₂CH₂(CH₂)₄, (CH₂)₄CH₂CH₂COO⁻), δ 31.9 (CH₃CH₂CH₂), δ 37.8 (CH₂CH₂COO⁻), δ 129.7 (CH=CH), δ 181.7 (COO⁻).

### <Working example 92> Synthesis of compound 92

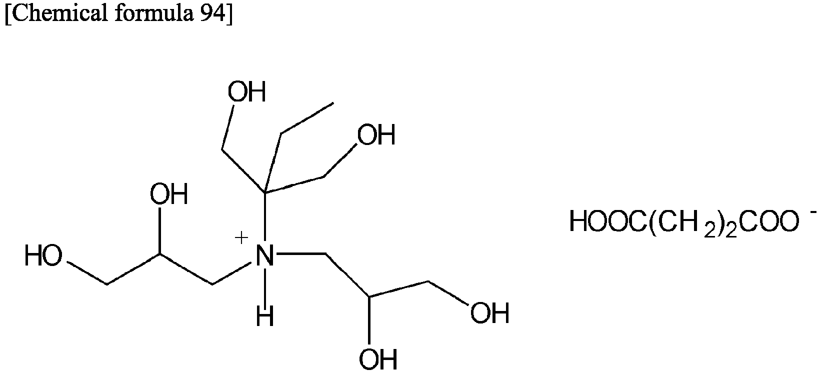

The compound was synthesized by a synthesis method similar to that of the working example 90 and at the compounding molar ratio shown in Table 10. The property values are shown below.

FT-IR(KBr): 3,162 m⁻¹: O-H stretching vibration 2,950 cm⁻¹: C-H stretching vibration 1,715 cm⁻¹: COOH stretching vibration 1,578 cm⁻¹: COO⁻ stretching vibration ¹H-NMR (D₂O 400 MHz): δ 2.51 (s, 4H, HOOCCH₂CH₂COO⁻), δ 3.00-3.23 (m, 4H, HOCH₂(CH(OH))₃CH(OH)CH₂NH₂⁺, NH₂⁺CH₂CH(OH)), δ 3.51-3.74 (m, 7H, HOCH₂(CH(OH))₃CH(OH)CH₂NH₂⁺, NH₂⁺CH₂CH(OH)CH₂OH), δ 3.94-3.98 (m, 1H, NH₂⁺CH₂CH(OH), δ 4.03-4.09 (m, 1H, HOCH₂(CH(OH))₃CH(OH)CH₂NH₂⁺). δ 2.51 (s, 4H, HOOCCH₂CH₂COO⁻).

¹³C-NMR (D₂O 100 MHz): δ 31.4 (HOOCCH₂CH₂COO⁻), δ 43.2 (HOCH₂(CH(OH))₃CH(OH)CH₂NH₂⁺), δ 45.8 (NH₂⁺CH₂CH(OH)), δ 62.8 (NH₂⁺CH₂CH(OH)CH₂OH), δ 64.7 (HOCH₂(CH(OH))₃CH(OH)CH₂NH₂⁺), δ 70.7-72.9 (HOCH₂(CH(OH))₃CH(OH)CH₂NH₃⁺, NH₂⁺CH₂CH(OH)CH₂OH). δ 31.4 (HOOCCH₂CH₂COO⁻), δ 179.7 (COOH, COO⁻).

### <Working example 93> Synthesis of compound 93

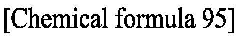

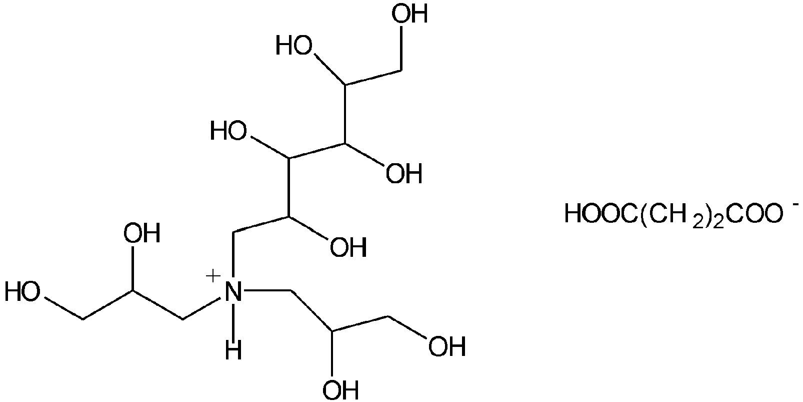

The compound was synthesized by a synthesis method similar to that of the working example 91 and at the compounding molar ratio shown in Table 10. The property values are shown below.

FT-IR(KBr): 3,162 m⁻¹: O-H stretching vibration 2,950 cm⁻¹: C-H stretching vibration 1,715 cm⁻¹: COOH stretching vibration 1,578 cm⁻¹: COO⁻ stretching vibration ¹H-NMR (D₂O 400 MHz): δ 3.00-3.23 (m, 6H, HOCH₂(CH(OH))₃CH(OH)CH₂NH⁺, NH⁺CH₂CH(OH)), δ 3.51-3.74 (m, 9H, HOCH₂(CH(OH))₃CH(OH)CH₂NH⁺, NH⁺CH₂CH(OH)CH₂OH), δ 3.94-3.98 (m, 2H, NH⁺CH₂CH(OH), δ 4.03-4.09 (m, 2H, HOCH₂(CH(OH))₃CH(OH)CH₂NH⁺). δ 2.51 (s, 4H, HOOCCH₂CH₂COO⁻).

¹³C-NMR (D₂O 100 MHz): δ 43.2 (HOCH₂(CH(OH))₃CH(OH)CH₂NH⁺), δ 45.8 (NH⁺CH₂CH(OH)), δ 62.8 (NH⁺CH₂CH(OH)CH₂OH), δ 64.7 (HOCH₂(CH(OH))₃CH(OH)CH₂NH⁺), δ 70.7-72.9 (HOCH₂(CH(OH))₃CH(OH)CH₂NH⁺, NH⁺CH₂CH(OH)CH₂OH). δ 31.4 (HOOCCH₂CH₂COO⁻), δ 179.7 (COOH, COO⁻).

### <Working example 94> Synthesis of compound 94

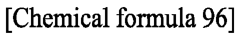

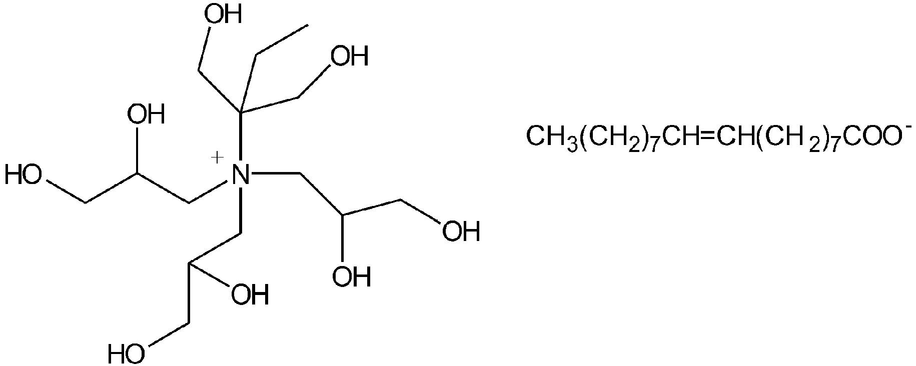

The amine-based compound 3 (10.00 g, 0.037 mol) and 3-chloro-1,2-propanediol (40.90 g, 0.370 mol) were reacted in 200 mL of acetonitrile under a pressurized condition and a temperature of 130°C for four hours, followed by distilling away acetonitrile under a reduced pressure. THF was then added to a solid thus obtained before performing washing with heating, thus obtaining a light yellow powder. Water was then added to such light yellow powder, followed by passing it through an anion-exchange resin to obtain an amine-based compound 5 shown in the working example 94 in Table 10.

The amine-based compound 5 (5.00 g, 0.014 mol) and isostearic acid (3.98 g, 0.0140 mol) were reacted in 50 mL of water under room temperature for three hours, followed by distilling away water under a reduced pressure to obtain a yellow solid. By washing the solid thus obtained, a compound 94 as a yellow solid (quaternary ammonium isostearate) was obtained.

FT-IR(KBr): 3,350 cm⁻¹: O-H stretching vibration 2,940 cm⁻¹: C-H stretching vibration 1,560 cm⁻¹: COO⁻ stretching vibration ¹H-NMR (D₂O 400 MHz): δ 0.86-0.92 (m, 6H, N⁺C(CH₂OH)₂CH₂CH₃, CH₃CH₂CH₂), δ 1.44 (m, 2H, N⁺C(CH₂OH)₂CH₂CH₃), δ 2.78-2.96 (m, 6H, N⁺CH₂CH(OH)), δ 3.61-3.78 (m, 10H, N⁺C(CH₂OH)₂CH₂CH₃, N⁺CH₂CH(OH)CH₂OH), δ 3.83-3.88 (m, 3H, N⁺CH₂CH(OH)). δ 0.83-0.90 (m, 6H, CH₃(CH₂)₈CH((CH₂)₆CH₃)COO⁻), δ1.08-1.58 (m, 28H, CH₃(CH₂)₈CH((CH₂)₆CH₃)COO⁻), δ 2.24-2.28 (m, 1H, CH₃(CH₂)₈CH((CH₂)₆CH₃)COO⁻).

¹³C-NMR (D₂O 100 MHz): δ 9.6 (N⁺C(CH₂OH)₂CH₂CH₃), δ 24.6 (N⁺C(CH₂OH)₂CH₂CH₃), δ 43.6 (N⁺C(CH₂OH)₂CH₂CH₃), δ 45.8 (N⁺CH₂CH(OH)), δ 60.2 (N⁺C(CH₂OH)₂CH₂CH₃), δ 62.8 (N⁺CH₂CH(OH)CH₂OH), δ 71.1 (N⁺CH₂CH(OH)CH₂OH). δ 14.1 (CH₃CH₂), δ 22.7 (CH₃CH--₂CH₂), δ 27.1 (CH₂CH₂CHCOO⁻), δ 30.0 (CH₃CH₂CH₂(CH₂)₄CH₂CH₂CH(CH₂CH₂(CH₂)₂CH₂CH₂CH₃)COO⁻,CH₂CH₂CHCOO⁻), δ 31.9 (CH₃CH₂CH₂), δ 37.2 (CHCOO-), δ 182.1 (CHCOO⁻).

### <Working example 95> Synthesis of compound 95

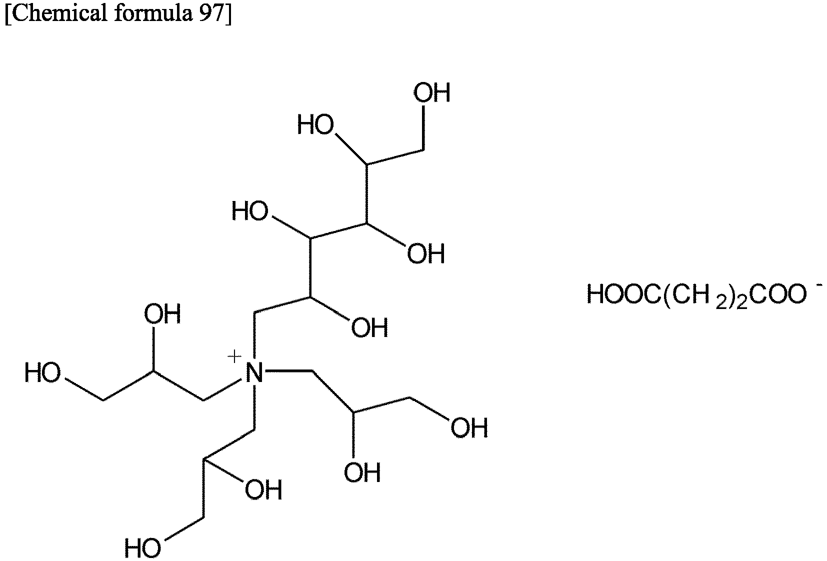

The amine-based compound 4 (10.00 g, 0.030 mol) and 3-chloro-1,2-propanediol (33.16 g, 0.300 mol) were reacted in 200 mL of acetonitrile under a pressurized condition and a temperature of 130°C for four hours, followed by distilling away acetonitrile under a reduced pressure. THF was then added to a solid thus obtained before performing washing with heating, thus obtaining a light yellow powder. Water was then added to such light yellow powder, followed by passing it through an anion-exchange resin to obtain an amine-based compound 6 shown in the working example 95 in Table 10.

The amine-based compound 6 (5.00 g, 0.012 mol) and succinic acid (1.42 g, 0.012 mol) were reacted in 50 mL of water under room temperature for three hours, followed by distilling away water under a reduced pressure to obtain a yellow solid. By washing the solid thus obtained, a compound 95 as a yellow solid (quaternary ammonium succinate) was obtained. FT-IR(KBr): 3,344 cm⁻¹: O-H stretching vibration 2,920 cm⁻¹: C-H stretching vibration 1,715 cm⁻¹: COOH stretching vibration 1,554 cm⁻¹: COO⁻ stretching vibration ¹H-NMR (D₂O 400 MHz): δ 3.00-3.23 (m, 8H, HOCH₂(CH(OH))₃CH(OH)CH₂N⁺, N⁺CH₂CH(OH)), δ 3.51-3.74 (m, 11H, HOCH₂(CH(OH))₃CH(OH)CH₂N⁺, N⁺CH₂CH(OH)CH₂OH), δ 3.94-3.98 (m, 3H, N⁺CH₂CH(OH)), δ 4.03-4.09 (m 1H, HOCH₂(CH(OH))₃CH(OH)CH₂N⁺). δ 2.51 (s, 4H, HOOCCH₂CH₂COO⁻).

¹³C-NMR (D₂O 100 MHz): δ 43.2 (HOCH₂(CH(OH))₃CH(OH)CH₂N⁺), δ 45.8 (N⁺CH₂CH(OH)), δ 62.8 (N⁺CH₂CH(OH)CH₂OH), δ 64.7 (HOCH₂(CH(OH))₃CH(OH)CH₂N⁺), δ 70.7-72.9 (HOCH₂(CH(OH))₃CH(OH)CH₂N⁺, N⁺CH₂CH(OH)CH₂OH). δ 31.4 (HOOCCH₂CH₂COO⁻), δ 179.7 (COOH, COO⁻).

### <Working examples 96 to 108>

Compounds 96 to 101, 103, 105 and 107 of the working examples 96 to 101, 103, 105 and 107 shown in Table 6 were synthesized by a synthesis method similar to that of the working example 94 and at the compounding molar ratios shown in Table 10. Further, compounds 102, 104, 106 and 108 of the working examples 102, 104, 106 and 108 were synthesized by a synthesis method similar to that of the working example 95 and at the compounding molar ratios shown in Table 10. The property values are shown below.

### <Working example 96> Synthesis of compound 96

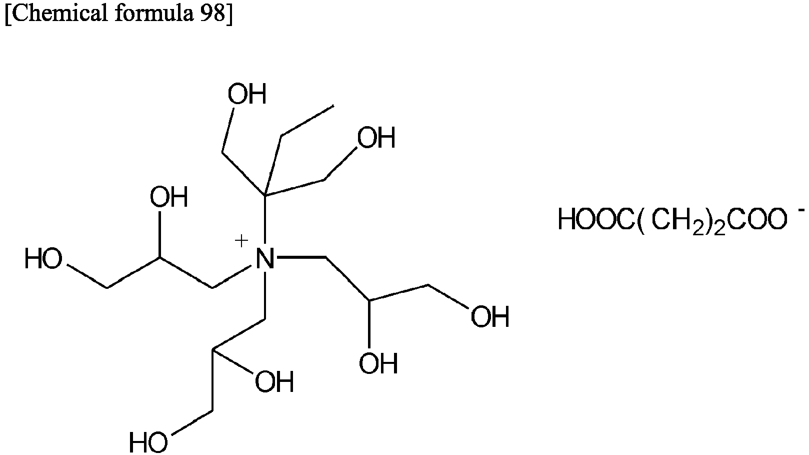

FT-IR(KBr): 3,398 cm⁻¹: O-H stretching vibration 2,922 cm⁻¹: C-H stretching vibration 1,715 cm⁻¹: COOH stretching vibration 1,560 cm⁻¹: COO⁻ stretching vibration ¹H-NMR (D₂O 400 MHz): δ 0.86-0.92 (m, 6H, N⁺C(CH₂OH)₂CH₂CH₃, CH₃CH₂CH₂), δ 1.44 (m, 2H, N⁺C(CH₂OH)₂CH₂CH₃), δ 2.78-2.96 (m, 6H, N⁺CH₂CH(OH)), δ 3.61-3.78 (m, 10H, N⁺C(CH₂OH)₂CH₂CH₃, N⁺CH₂CH(OH)CH₂OH), δ 3.83-3.88 (m, 3H, N⁺CH₂CH(OH)). δ 2.51 (s, 4H, HOOCCH₂CH₂COO⁻).

¹³C-NMR (D₂O 100 MHz): δ 9.6 (N⁺C(CH₂OH)₂CH₂CH₃), δ 24.6 (N⁺C(CH₂OH)₂CH₂CH₃), δ 43.6 (N⁺C(CH₂OH)₂CH₂CH₃), δ 45.8 (N⁺CH₂CH(OH)), δ 60.2 (N⁺C(CH₂OH)₂CH₂CH₃), δ 62.8 (N⁺CH₂CH(OH)CH₂OH), δ 71.1 (N⁺CH₂CH(OH)CH₂OH). δ 31.4 (HOOCCH₂CH₂COO⁻), δ 179.7 (COOH, COO⁻).

### <Working example 97> Synthesis of compound 97

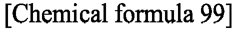

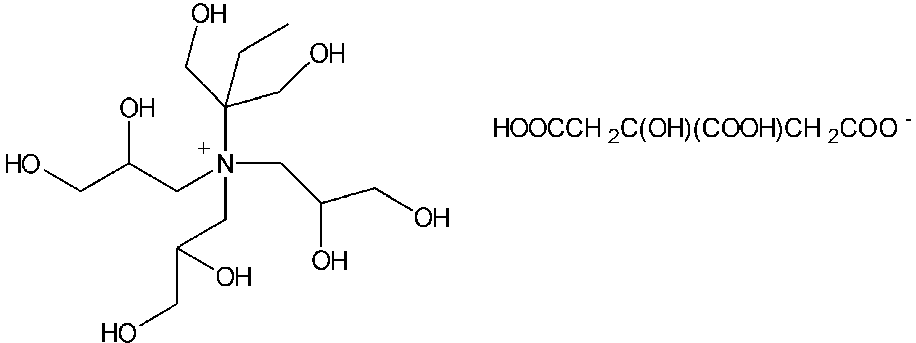

FT-IR(KBr): 3,398 cm⁻¹: O-H stretching vibration 2,922 cm⁻¹: C-H stretching vibration 1,715 cm⁻¹: COOH stretching vibration 1,560 cm⁻¹: COO⁻ stretching vibration ¹H-NMR (D₂O 400 MHz): δ 0.86-0.92 (m, 6H, N⁺C(CH₂OH)₂CH₂CH₃, CH₃CH₂CH₂), δ 1.44 (m, 2H, N⁺C(CH₂OH)₂CH₂CH₃), δ 2.78-2.96 (m, 6H, N⁺CH₂CH(OH)), δ 3.61-3.78 (m, 10H, N⁺C(CH₂OH)₂CH₂CH₃, N⁺CH₂CH(OH)CH₂OH), δ 3.83-3.88 (m, 3H, N⁺CH₂CH(OH)). δ 2.66-2.83 (m, 4H, HOOCCH₂C(OH)(COOH)CH₂COO⁻).

¹³C-NMR (D₂O 100 MHz): δ 9.6 (N⁺C(CH₂OH)₂CH₂CH₃), δ 24.6 (N⁺C(CH₂OH)₂CH₂CH₃), δ 43.6 (N⁺C(CH₂OH)₂CH₂CH₃), δ 45.8 (N⁺CH₂CH(OH)), δ 60.2 (N⁺C(CH₂OH)₂CH₂CH₃), δ 62.8 (N⁺CH₂CH(OH)CH₂OH), δ 71.1 (N⁺CH₂CH(OH)CH₂OH). δ 43.7 (HOOCCH₂C(OH)(COOH)CH₂COO⁻), δ 73.8 (HOOCCH₂C(OH)(COOH)CH₂COO⁻), δ 174.7 (HOOCCH₂C(OH)(COOH)CH₂COO⁻), δ 178.6 (HOOCCH₂C(OH)(COOH)CH₂COO⁻).

### <Working example 98> Synthesis of compound 98

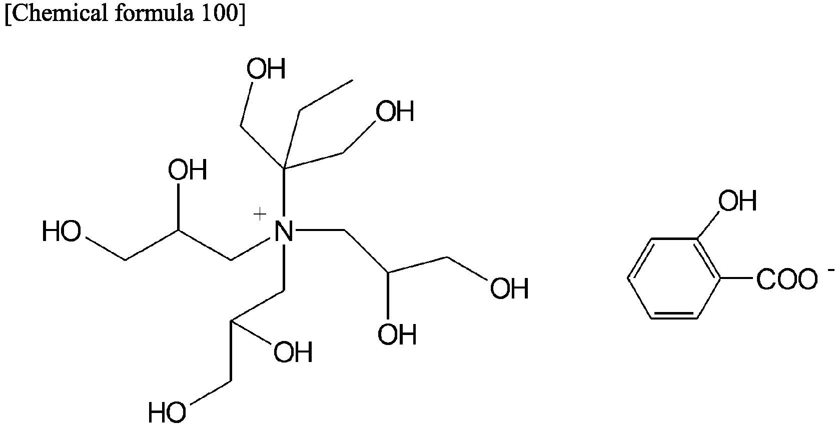

FT-IR(KBr): 3,398 cm⁻¹: O-H stretching vibration 2,922 cm⁻¹: C-H stretching vibration 1,560 cm⁻¹: COO⁻ stretching vibration ¹H-NMR (D₂O 400 MHz): δ 0.86-0.92 (m, 6H, N⁺C(CH₂OH)₂CH₂CH₃, CH₃CH₂CH₂), δ 1.44 (m, 2H, N⁺C(CH₂OH)₂CH₂CH₃), δ 2.78-2.96 (m, 6H, N⁺CH₂CH(OH)), δ 3.61-3.78 (m, 10H, N⁺C(CH₂OH)₂CH₂CH₃, N⁺CH₂CH(OH)CH₂OH), δ 3.83-3.88 (m, 3H, N⁺CH₂CH(OH)). δ 6.80-6.86 (m, 2H, C(OH)CHCH, C(COO⁻)CHCHCH), δ 7.35-7.39 (m, 1H, CHCHC(OH)), δ 7.68-7.73 (m, 1H, C(COOH)CHCH).

¹³C-NMR (D₂O 100 MHz): δ 9.6 (N⁺C(CH₂OH)₂CH₂CH₃), δ 24.6 (N⁺C(CH₂OH)₂CH₂CH₃), δ 43.6 (N⁺C(CH₂OH)₂CH₂CH₃), δ 45.8 (N⁺CH₂CH(OH)), δ 60.2 (N⁺C(CH₂OH)₂CH₂CH₃), δ 62.8 (N⁺CH₂CH(OH)CH₂OH), δ 71.1 (N⁺CH₂CH(OH)CH₂OH). δ 116.2 (C(OH)CHCH), δ 118.0 (CHC(COO⁻)C(OH)), δ 119.3 (C(COO⁻)CHCHCH), δ 130.5 (C(COO⁻)CHCH), δ 133.9 (CHCHC(OH)), δ 159.6 (CC(OH)C), δ 175.5 (CCOO⁻).

### <Working example 99> Synthesis of compound 99

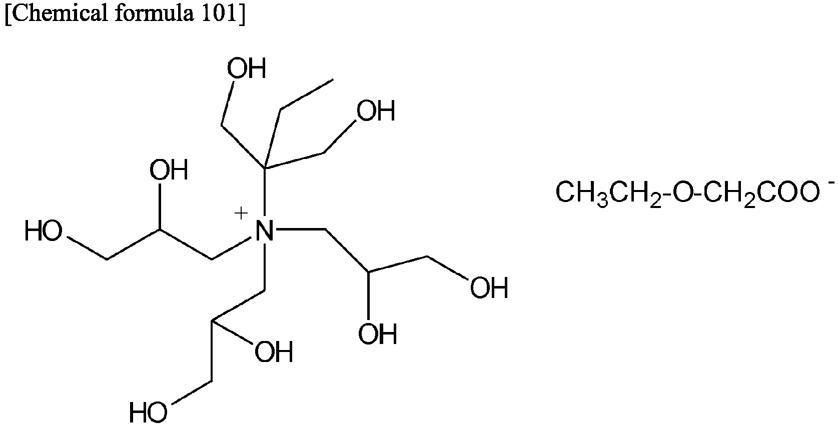

FT-IR(KBr): 3,398 cm⁻¹: O-H stretching vibration 2,922 cm⁻¹: C-H stretching vibration 1,560 cm⁻¹: COO⁻ stretching vibration ¹H-NMR (D₂O 400 MHz): δ 0.86-0.92 (m, 6H, N⁺C(CH₂OH)₂CH₂CH₃, CH₃CH₂CH₂), δ 1.44 (m, 2H, N⁺C(CH₂OH)₂CH₂CH₃), δ 2.78-2.96 (m, 6H, N⁺CH₂CH(OH)), δ 3.61-3.78 (m, 10H, N⁺C(CH₂OH)₂CH₂CH₃, N⁺CH₂CH(OH)CH₂OH), δ 3.83-3.88 (m, 3H, N⁺CH₂CH(OH)). δ 0.99 (s, 3H, CH₃CH₂OCH₂COO⁻), δ 3.34-3.39 (q, 2H, CH₃CH₂OCH₂COO⁻), δ 3.49-3.64 (m, 2H, CH₃CH₂OCH₂COO⁻).

¹³C-NMR (D₂O 100 MHz): δ 9.6 (N⁺C(CH₂OH)₂CH₂CH₃), δ 24.6 (N⁺C(CH₂OH)₂CH₂CH₃), δ 43.6 (N⁺C(CH₂OH)₂CH₂CH₃), δ 45.8 (N⁺CH₂CH(OH)), δ 60.2 (N⁺C(CH₂OH)₂CH₂CH₃), δ 62.8 (N⁺CH₂CH(OH)CH₂OH), δ 71.1 (N⁺CH₂CH(OH)CH₂OH). δ 14.0 (CH₃CH₂O), δ 66.4 (CH₃CH₂O), δ 69.1 (CH₃CH₂OCH₂COO⁻), δ 178.1 (CH₃CH₂OCH₂COO⁻).

### <Working example 100> Synthesis of compound 100

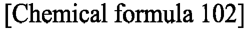

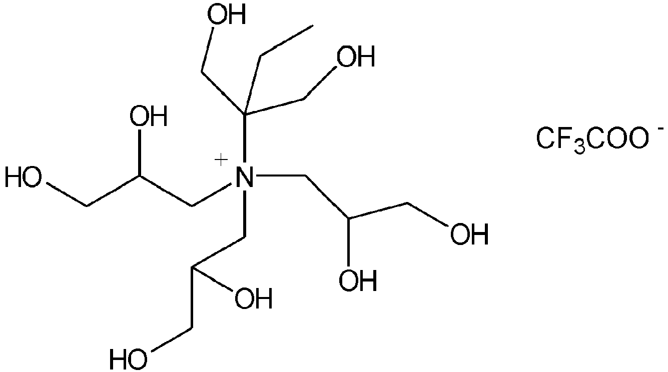

FT-IR(KBr): 3,398 cm⁻¹: O-H stretching vibration 2,922 cm⁻¹: C-H stretching vibration 1,665 cm⁻¹: COO⁻ stretching vibration ¹H-NMR (D₂O 400 MHz): δ 0.86-0.92 (m, 6H, N⁺C(CH₂OH)₂CH₂CH₃, CH₃CH₂CH₂), δ 1.44 (m, 2H, N⁺C(CH₂OH)₂CH₂CH₃), δ 2.78-2.96 (m, 6H, N⁺CH₂CH(OH)), δ 3.61-3.78 (m, 10H, N⁺C(CH₂OH)₂CH₂CH₃, N⁺CH₂CH(OH)CH₂OH), δ 3.83-3.88 (m, 3H, N⁺CH₂CH(OH)).

¹³C-NMR (D₂O 100 MHz): δ 9.6 (N⁺C(CH₂OH)₂CH₂CH₃), δ 24.6 (N⁺C(CH₂OH)₂CH₂CH₃), δ 43.6 (N⁺C(CH₂OH)₂CH₂CH₃), δ 45.8 (N⁺CH₂CH(OH)), δ 60.2 (N⁺C(CH₂OH)₂CH₂CH₃), δ 62.8 (N⁺CH₂CH(OH)CH₂OH), δ 71.1 (N⁺CH₂CH(OH)CH₂OH). δ 114.6 (CF₃COO⁻), δ 165.1 (CF₃COO⁻).

### <Working example 101> Synthesis of compound 101

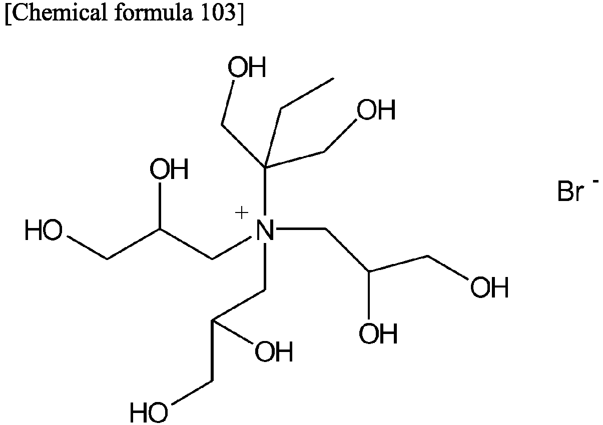

FT-IR(KBr): 3,398 cm⁻¹: O-H stretching vibration 2,922 cm⁻¹: C-H stretching vibration ¹H-NMR (D₂O 400 MHz): δ 0.86-0.92 (m, 6H, N⁺C(CH₂OH)₂CH₂CH₃, CH₃CH₂CH₂), δ 1.44 (m, 2H, N⁺C(CH₂OH)₂CH₂CH₃), δ 2.78-2.96 (m, 6H, N⁺CH₂CH(OH)), δ 3.61-3.78 (m, 10H, N⁺C(CH₂OH)₂CH₂CH₃, N⁺CH₂CH(OH)CH₂OH), δ 3.83-3.88 (m, 3H, N⁺CH₂CH(OH)). ¹³C-NMR (D₂O 100 MHz): δ 9.6 (N⁺C(CH₂OH)₂CH₂CH₃), δ 24.6 (N⁺C(CH₂OH)₂CH₂CH₃), δ 43.6 (N⁺C(CH₂OH)₂CH₂CH₃), δ 45.8 (N⁺CH₂CH(OH)), δ 60.2 (N⁺C(CH₂OH)₂CH₂CH₃), δ 62.8 (N⁺CH₂CH(OH)CH₂OH), δ 71.1 (N⁺CH₂CH(OH)CH₂OH).

### <Working example 102> Synthesis of compound 102

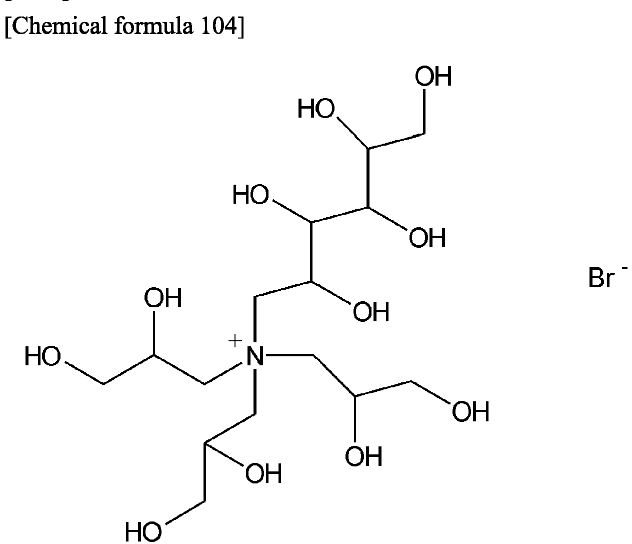

FT-IR(KBr): 3,344 cm⁻¹: O-H stretching vibration 2,920 cm⁻¹: C-H stretching vibration ¹H-NMR (D₂O 400 MHz): δ 3.00-3.23 (m, 8H, HOCH₂(CH(OH))₃CH(OH)CH₂N⁺, N⁺CH₂CH(OH)), δ 3.51-3.74 (m, 11H, HOCH₂(CH(OH))₃CH(OH)CH₂N⁺, N⁺CH₂CH(OH)CH₂OH), δ 3.94-3.98 (m, 3H, N⁺CH₂CH(OH)), δ 4.03-4.09 (m 1H, HOCH₂(CH(OH))₃CH(OH)CH₂N⁺).

¹³C-NMR (D₂O 100 MHz): δ 43.2 (HOCH₂(CH(OH))₃CH(OH)CH₂N⁺), δ 45.8 (N⁺CH₂CH(OH)), δ 62.8 (N⁺CH₂CH(OH)CH₂OH), δ 64.7 (HOCH₂(CH(OH))₃CH(OH)CH₂N⁺), δ 70.7-72.9 (HOCH₂(CH(OH))₃CH(OH)CH₂N⁺, N⁺CH₂CH(OH)CH₂OH).

### <Working example 103> Synthesis of compound 103

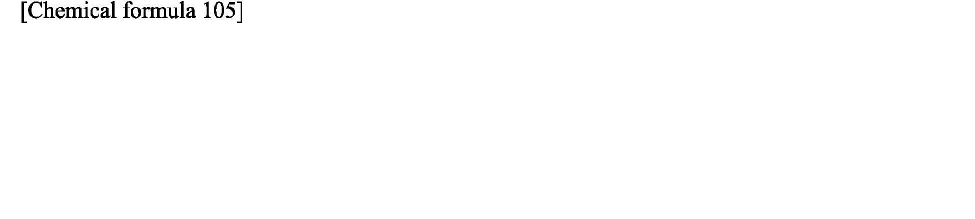

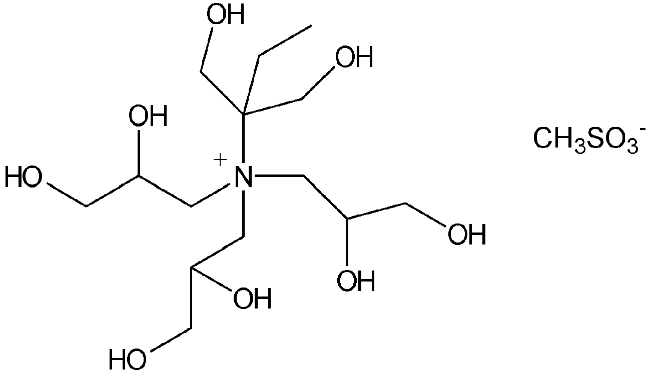

FT-IR(KBr): 3,398 cm⁻¹: O-H stretching vibration 2,922 cm⁻¹: C-H stretching vibration ¹H-NMR (D₂O 400 MHz): δ 0.86-0.92 (m, 6H, N⁺C(CH₂OH)₂CH₂CH₃, CH₃CH₂CH₂), δ 1.44 (m, 2H, N⁺C(CH₂OH)₂CH₂CH₃), δ 2.78-2.96 (m, 6H, N⁺CH₂CH(OH)), δ 3.61-3.78 (m, 10H, N⁺C(CH₂OH)₂CH₂CH₃, N⁺CH₂CH(OH)CH₂OH), δ 3.83-3.88 (m, 3H, N⁺CH₂CH(OH)). δ 2.75 (s, 3H, CH₃SO₃⁻).

¹³C-NMR (D₂O 100 MHz): δ 9.6 (N⁺C(CH₂OH)₂CH₂CH₃), δ 24.6 (N⁺C(CH₂OH)₂CH₂CH₃), δ 43.6 (N⁺C(CH₂OH)₂CH₂CH₃), δ 45.8 (N⁺CH₂CH(OH)), δ 60.2 (N⁺C(CH₂OH)₂CH₂CH₃), δ 62.8 (N⁺CH₂CH(OH)CH₂OH), δ 71.1 (N⁺CH₂CH(OH)CH₂OH). δ 38.5 (CH₃SO₃⁻).

### <Working example 104> Synthesis of compound 104

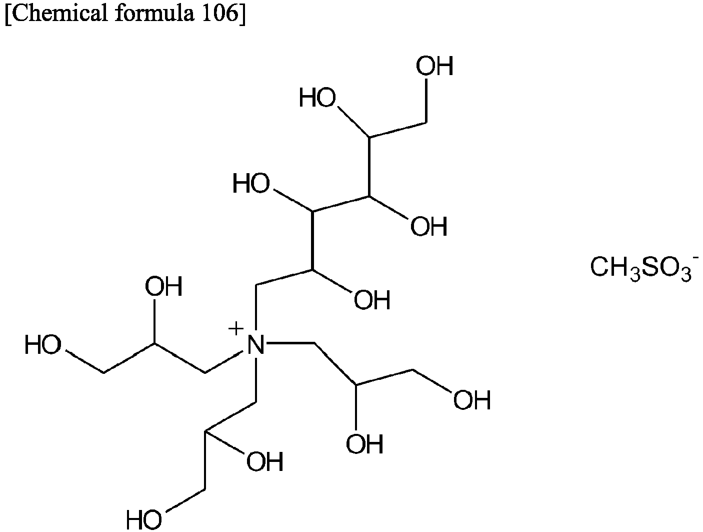

FT-IR(KBr): 3,344 cm⁻¹: O-H stretching vibration 2,920 cm⁻¹: C-H stretching vibration ¹H-NMR (D₂O 400 MHz): δ 3.00-3.23 (m, 8H, HOCH₂(CH(OH))₃CH(OH)CH₂N⁺, N⁺CH₂CH(OH)), δ 3.51-3.74 (m, 11H, HOCH₂(CH(OH))₃CH(OH)CH₂N⁺, N⁺CH₂CH(OH)CH₂OH), δ 3.94-3.98 (m, 3H, N⁺CH₂CH(OH)), δ 4.03-4.09 (m 1H, HOCH₂(CH(OH))₃CH(OH)CH₂N⁺). δ 2.75 (s, 3H, CH₃SO₃⁻).

¹³C-NMR (D₂O 100 MHz): δ 43.2 (HOCH₂(CH(OH))₃CH(OH)CH₂N⁺), δ 45.8 (N⁺CH₂CH(OH)), δ 62.8 (N⁺CH₂CH(OH)CH₂OH), δ 64.7 (HOCH₂(CH(OH))₃CH(OH)CH₂N⁺), δ 70.7-72.9 (HOCH₂(CH(OH))₃CH(OH)CH₂N⁺, N⁺CH₂CH(OH)CH₂OH). δ 38.5 (CH₃SO₃⁻).

### <Working example 105> Synthesis of compound 105

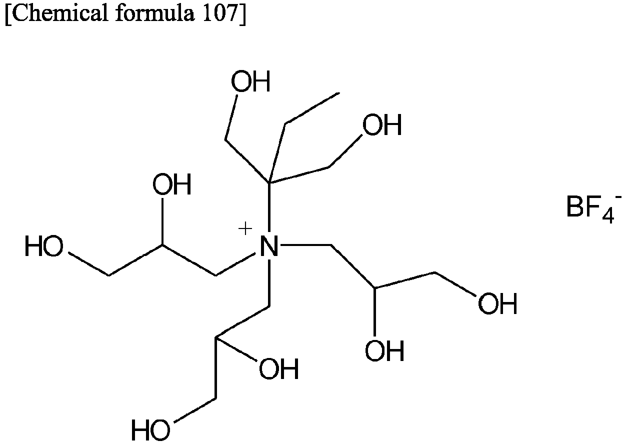

FT-IR(KBr): 3,398 cm⁻¹: O-H stretching vibration 2,922 cm⁻¹: C-H stretching vibration ¹H-NMR (D₂O 400 MHz): δ 0.86-0.92 (m, 6H, N⁺C(CH₂OH)₂CH₂CH₃, CH₃CH₂CH₂), δ 1.44 (m, 2H, N⁺C(CH₂OH)₂CH₂CH₃), δ 2.78-2.96 (m, 6H, N⁺CH₂CH(OH)), δ 3.61-3.78 (m, 10H, N⁺C(CH₂OH)₂CH₂CH₃, N⁺CH₂CH(OH)CH₂OH), δ 3.83-3.88 (m, 3H, N⁺CH₂CH(OH)). ¹³C-NMR (D₂O 100 MHz): δ 9.6 (N⁺C(CH₂OH)₂CH₂CH₃), δ 24.6 (N⁺C(CH₂OH)₂CH₂CH₃), δ 43.6 (N⁺C(CH₂OH)₂CH₂CH₃), δ 45.8 (N⁺CH₂CH(OH)), δ 60.2 (N⁺C(CH₂OH)₂CH₂CH₃), δ 62.8 (N⁺CH₂CH(OH)CH₂OH), δ 71.1 (N⁺CH₂CH(OH)CH₂OH).

### <Working example 106> Synthesis of compound 106

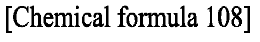

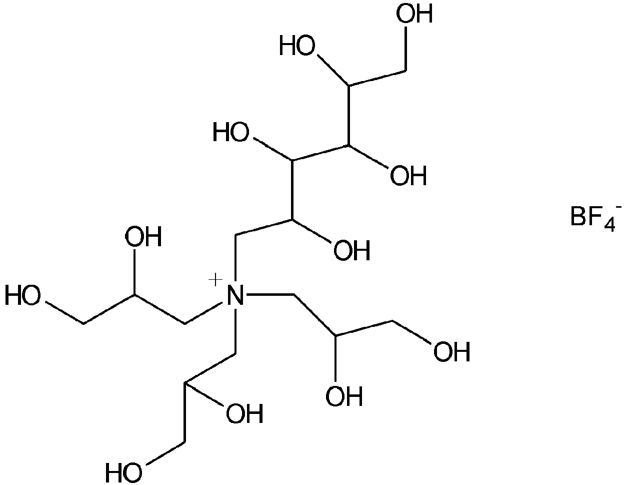

FT-IR(KBr): 3,344 cm⁻¹: O-H stretching vibration 2,920 cm⁻¹: C-H stretching vibration ¹H-NMR (D₂O 400 MHz): δ 3.00-3.23 (m, 8H, HOCH₂(CH(OH))₃CH(OH)CH₂N⁺, N⁺CH₂CH(OH)), δ 3.51-3.74 (m, 11H, HOCH₂(CH(OH))₃CH(OH)CH₂N⁺, N⁺CH₂CH(OH)CH₂OH), δ 3.94-3.98 (m, 3H, N⁺CH₂CH(OH)), δ 4.03-4.09 (m 1H, HOCH₂(CH(OH))₃CH(OH)CH₂N⁺).

¹³C-NMR (D₂O 100 MHz): δ 43.2 (HOCH₂(CH(OH))₃CH(OH)CH₂N⁺), δ 45.8 (N⁺CH₂CH(OH)), δ 62.8 (N⁺CH₂CH(OH)CH₂OH), δ 64.7 (HOCH₂(CH(OH))₃CH(OH)CH₂N⁺), δ 70.7-72.9 (HOCH₂(CH(OH))₃CH(OH)CH₂N⁺, N⁺CH₂CH(OH)CH₂OH).

### <Working example 107> Synthesis of compound 107

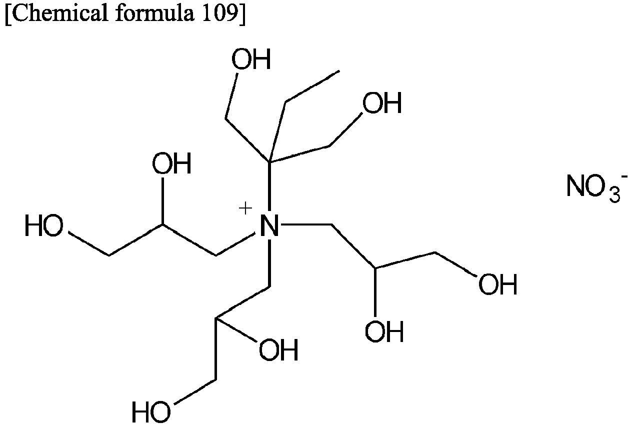

FT-IR(KBr): 3,398 cm⁻¹: O-H stretching vibration 2,922 cm⁻¹: C-H stretching vibration ¹H-NMR (D₂O 400 MHz): δ 0.86-0.92 (m, 6H, N⁺C(CH₂OH)₂CH₂CH₃, CH₃CH₂CH₂), δ 1.44 (m, 2H, N⁺C(CH₂OH)₂CH₂CH₃), δ 2.78-2.96 (m, 6H, N⁺CH₂CH(OH)), δ 3.61-3.78 (m, 10H, N⁺C(CH₂OH)₂CH₂CH₃, N⁺CH₂CH(OH)CH₂OH), δ 3.83-3.88 (m, 3H, N⁺CH₂CH(OH)). ¹³C-NMR (D₂O 100 MHz): δ 9.6 (N⁺C(CH₂OH)₂CH₂CH₃), δ 24.6 (N⁺C(CH₂OH)₂CH₂CH₃), δ 43.6 (N⁺C(CH₂OH)₂CH₂CH₃), δ 45.8 (N⁺CH₂CH(OH)), δ 60.2 (N⁺C(CH₂OH)₂CH₂CH₃), δ 62.8 (N⁺CH₂CH(OH)CH₂OH), δ 71.1 (N⁺CH₂CH(OH)CH₂OH).

### <Working example 108> Synthesis of compound 108

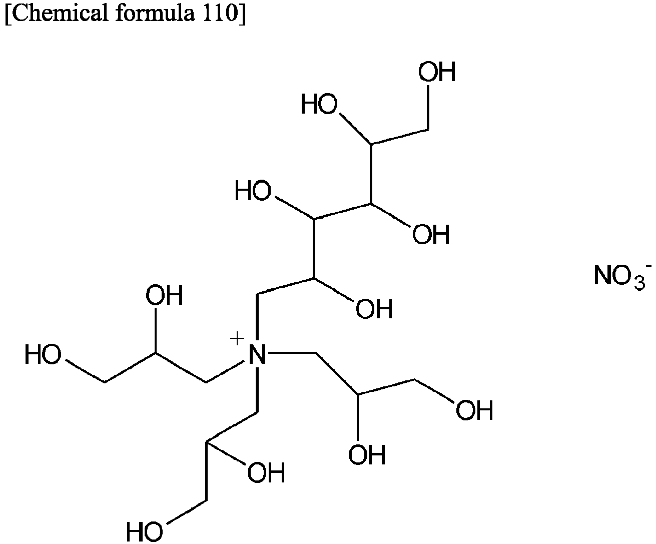

FT-IR(KBr):3,344 cm⁻¹: O-H stretching vibration 2,920 cm⁻¹: C-H stretching vibration ¹H-NMR (D₂O 400 MHz): δ 3.00-3.23 (m, 8H, HOCH₂(CH(OH))₃CH(OH)CH₂N⁺, N⁺CH₂CH(OH)), δ 3.51-3.74 (m, 11H, HOCH₂(CH(OH))₃CH(OH)CH₂N⁺, N⁺CH₂CH(OH)CH₂OH), δ 3.94-3.98 (m, 3H, N⁺CH₂CH(OH)), δ 4.03-4.09 (m 1H, HOCH₂(CH(OH))₃CH(OH)CH₂N⁺).

¹³C-NMR (D₂O 100 MHz): δ 43.2 (HOCH₂(CH(OH))₃CH(OH)CH₂N⁺), δ 45.8 (N⁺CH₂CH(OH)), δ 62.8 (N⁺CH₂CH(OH)CH₂OH), δ 64.7 (HOCH₂(CH(OH))₃CH(OH)CH₂N⁺), δ 70.7-72.9 (HOCH₂(CH(OH))₃CH(OH)CH₂N⁺, N⁺CH₂CH(OH)CH₂OH).

### <Working example 109> Synthesis of compound 109

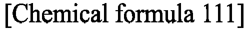

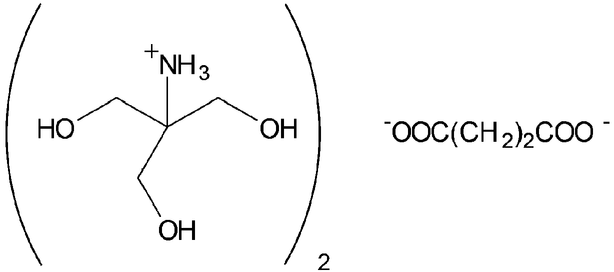

A compound 109 of a working example 109 shown in Table 6 was synthesized by a synthesis method similar to that of the working example 1 and at the compounding molar ratio shown in Table 10. The property values are shown below.

FT-IR(KBr): 3,145 cm⁻¹: O-H stretching vibration 2,946 cm⁻¹: C-H stretching vibration 1,572 cm⁻¹: COO⁻ stretching vibration ¹H-NMR (D₂O 400 MHz): δ 2.28 (s, 4H, ⁻OOCCH₂CH₂COO⁻), δ 3.61 (s, 12H, NH₃⁺C(CH₂OH)₃).

¹³C-NMR (D₂O 100 MHz): δ 34.0 (⁻OOCCH₂CH₂COO⁻), δ 59.4 (NH₃⁺C(CH₂OH)₃), δ 61.3 (NH₃⁺C(CH₂OH)₃), δ 182.3 (COO⁻).

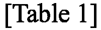

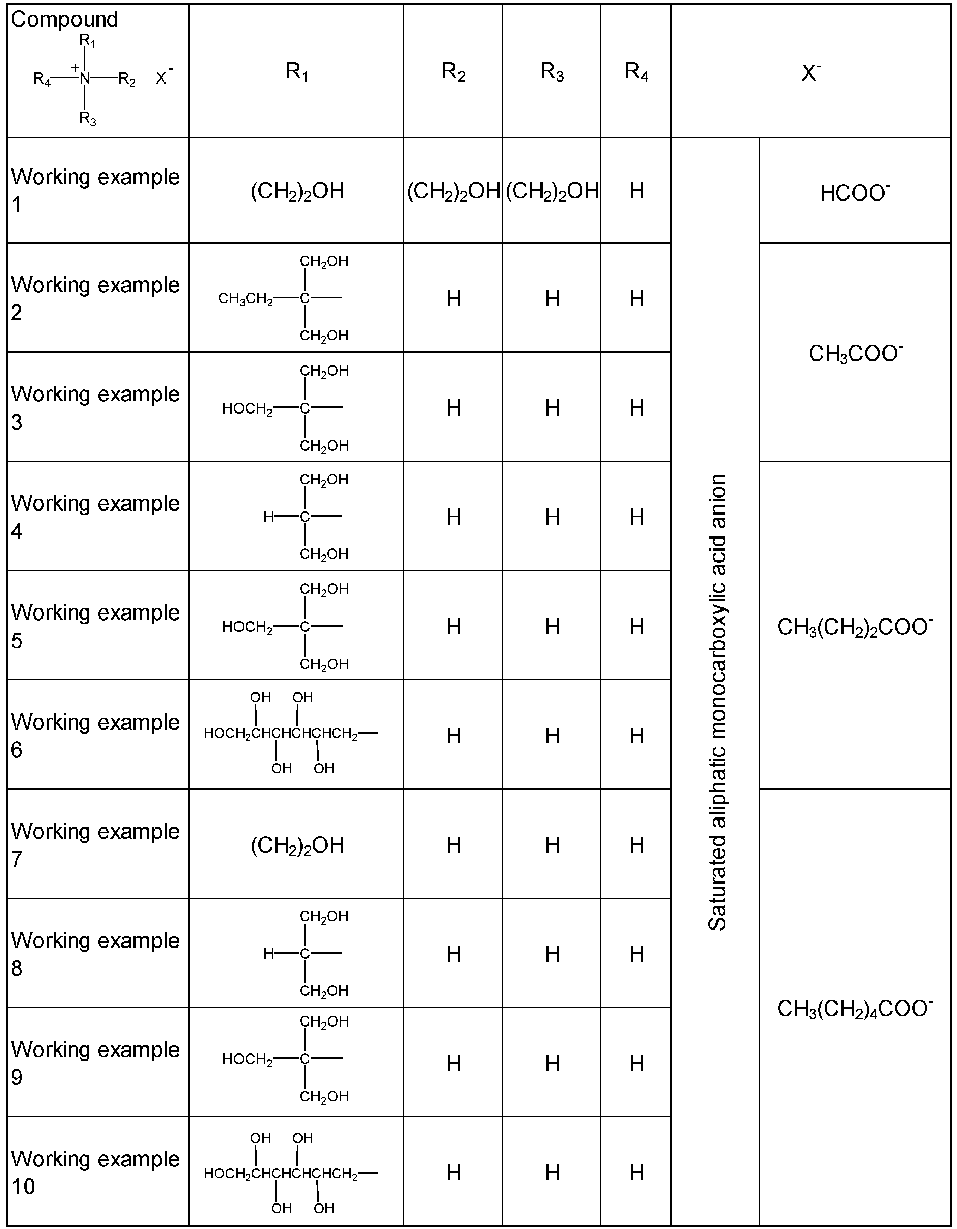

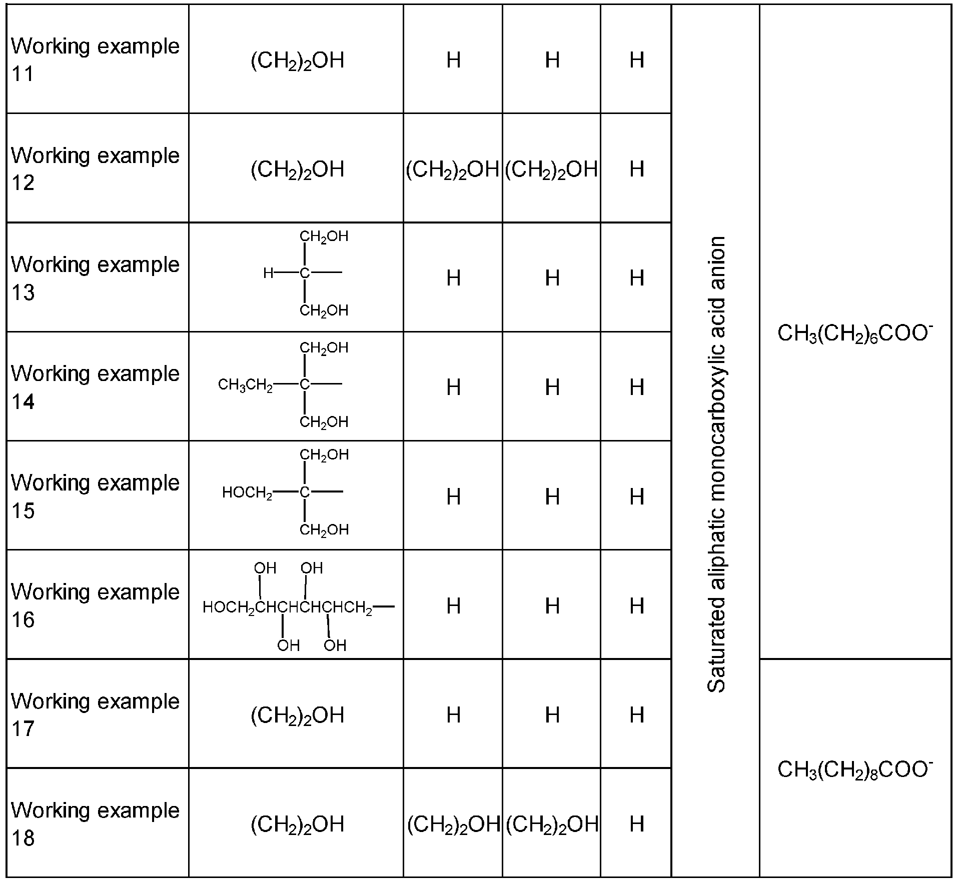

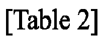

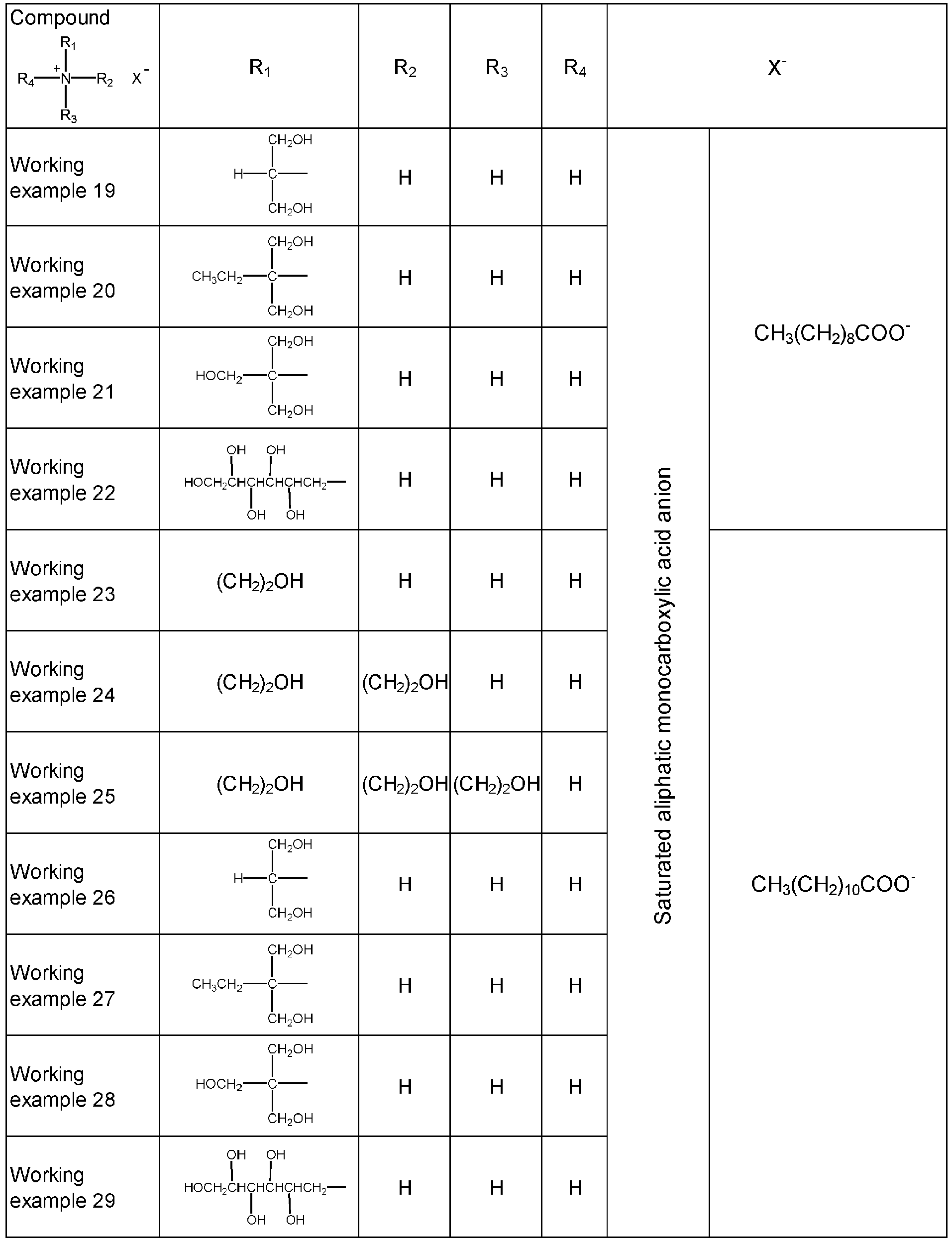

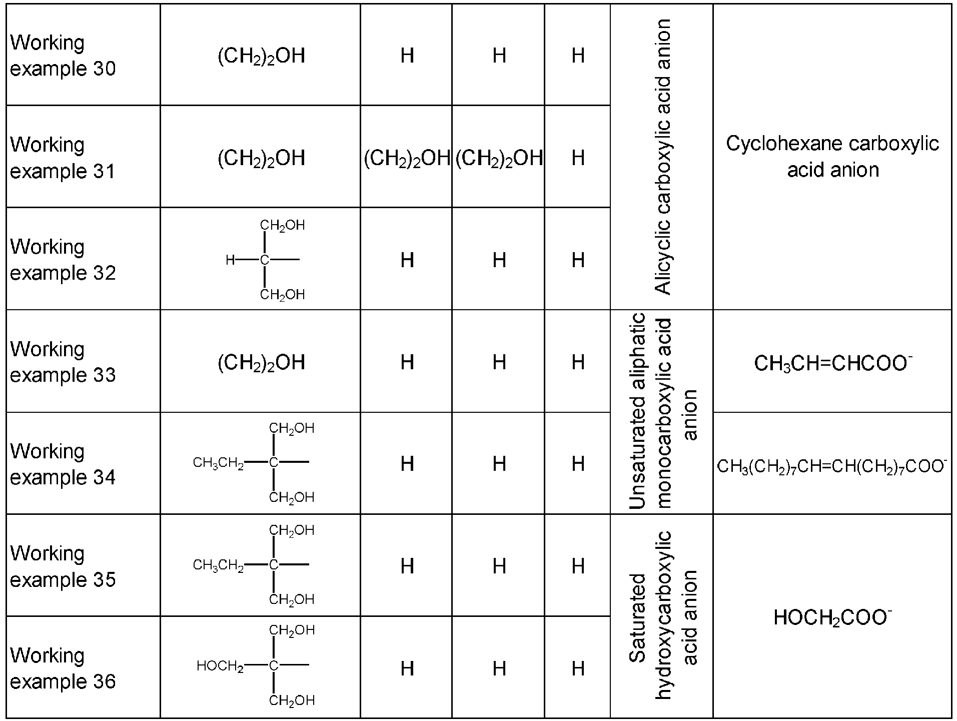

**[Table 3]**

| Compound | R₁ | R₂ | R₃ | R₄ | X⁻ | |
|---|---|---|---|---|---|---|
| 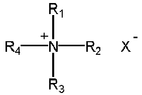 | | | | | | |
| Working example 37 | 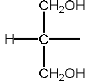 | H | H | H | Saturated hydroxycarboxylic acid anion | CH₃(CH₂)₇CH(OH)C H(OH)(CH₂)₇COO⁻ |
| Working example 38 | 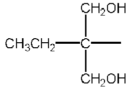 | H | H | H | | |
| Working example 39 | 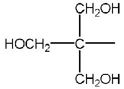 | H | H | H | | |
| Working example 40 | 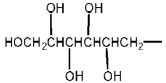 | H | H | H | | |
| Working example 41 | (CH₂)₂OH | H | H | H | | Quinic acid anion |
| Working example 42 | (CH₂)₂OH | (CH₂)₂OH | H | H | | |
| Working example 43 | 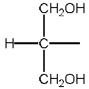 | H | H | H | | |
| Working example 44 | 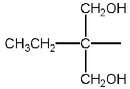 | H | H | H | | |
| Working example 45 | 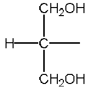 | H | H | H | | 12-hydroxystearic acid anion |
| Working example 46 | 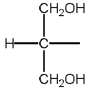 | H | H | H | Saturated dicarboxylic acid anion | HOOCCOO⁻ |
| Working example 47 | (CH₂)₂OH | H | H | H | | HOOC(CH₂)₂COO⁻ |
| Working example 48 | 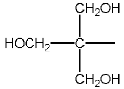 | H | H | H | | |
| Working example 49 | (CH₂)₂OH | H | H | H | Saturated dicarboxylic acid anion | HOOC(CH₂)₄COO⁻ |
| Working example 50 | (CH₂)₂OH | (CH₂)₂OH | (CH₂)₂OH | H | | |
| Working example 51 | 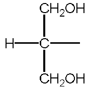 | H | H | H | | |
| Working example 52 | 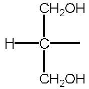 | H | H | H | | HOOC(CH₂)₈COO⁻ |
| Working example 53 | 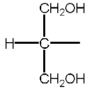 | H | H | H | Unsaturated dicarboxylic acid anion | HOOCCH=CHCOO⁻ |
| Working example 54 | (CH₂)₂OH | H | H | H | Saturated hydroxydi- or tricarboxylic acid anion | HOOCCH(OH)CH(O H)COO⁻ |

**[Table 4]**

| Compound | R₁ | R₂ | R₃ | R₄ | X⁻ | |
|---|---|---|---|---|---|---|
| 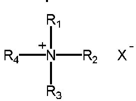 | | | | | | |
| Working example 55 | (CH₂)₂OH | H | H | H | Saturated hydroxydi- or tricarboxylic acid anion | HOOCCH₂C(OH)(COOH )CH₂COO⁻ |
| Working example 56 | (CH₂)₂OH | H | H | H | Aromatic carboxylic acid anion | Benzoic acid anion |
| Working example 57 | (CH₂)₂OH | (CH₂)₂OH | (CH₂)₂OH | H | | |
| Working example 58 | 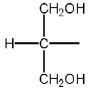 | H | H | H | | |
| Working example 59 | 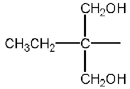 | H | H | H | | |
| Working example 60 | 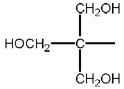 | H | H | H | | |
| Working example 61 | 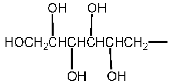 | H | H | H | | |
| Working example 62 | (CH₂)₂OH | H | H | H | | Terephthalic acid anion |
| Working example 63 | (CH₂)₂OH | (CH₂)₂OH | H | H | | |
| Working example 64 | (CH₂)₂OH | (CH₂)₂OH | (CH₂)₂OH | H | | |
| Working example 65 | 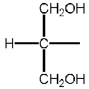 | H | H | H | | |
| Working example 66 | (CH₂)₂OH | H | H | H | Aromatic carboxylic acid anion | Salicylic acid anion |
| Working example 67 | 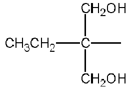 | H | H | H | | |
| Working example 68 | 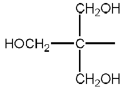 | H | H | H | | |
| Working example 69 | (CH₂)₂OH | H | H | H | | p-hydroxybenzoic acid anion |
| Working example 70 | (CH₂)₂OH | (CH₂)₂OH | (CH₂)₂OH | H | | |
| Working example 71 | 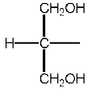 | H | H | H | | |
| Working example 72 | (CH₂)₂OH | H | H | H | | Mandelic acid anion |

**[Table 5]**

| Compound | R₁ | R₂ | R₃ | R₄ | X⁻ | |
|---|---|---|---|---|---|---|
| 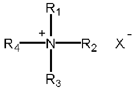 | | | | | | |
| Working example 73 | 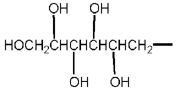 | H | H | H | Saturated carbonyl carboxylic acid anion | CH₃C(=O)COO⁻ |
| Working example 74 | 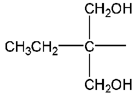 | H | H | H | Alkylether carboxylic acid anion | CH₃-O-CH₂COO⁻ |
| Working example 75 | (CH₂)₂OH | H | H | H | Halogen carboxylic acid anion | CF₃COO⁻ |
| Working example 76 | 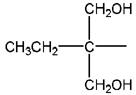 | H | H | H | | |
| Working example 77 | 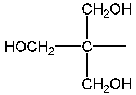 | H | H | H | | |
| Working example 78 | 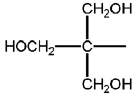 | H | H | H | Halide anion | Br⁻ |
| Working example 79 | 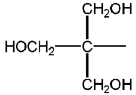 | H | H | H | | Cl⁻ |
| Working example 80 | 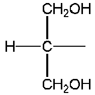 | H | H | H | Fluorine-based anion | (CF₃SO₂)₂N⁻ |
| Working example 81 | 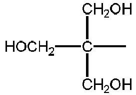 | H | H | H | Sulfur-based anion | SO₄²⁻ |
| Working example 82 | 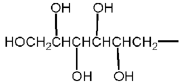 | H | H | H | | |
| Working example 83 | 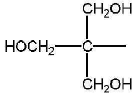 | H | H | H | Phosphorusbased anion | H₂PO₄⁻ |
| Working example 84 | 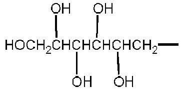 | H | H | H | | |
| Working example 85 | 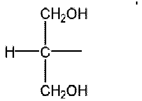 | H | H | H | Cyano-based anion | N(CN)₂⁻ |
| Working example 86 | 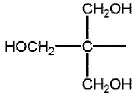 | H | H | H | | |
| Working example 87 | 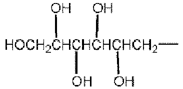 | H | H | H | | |

**[Table 6]**

| Compound | R₁ | R₂ | R₃ | R₄ | X" | |
|---|---|---|---|---|---|---|
| 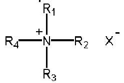 | | | | | | |
| Working example 88 | 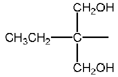 | 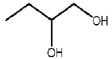 | H | H | Saturated dicarboxylic acid anion | HOOC(CH₂)₂COO⁻ |
| Working example 89 | 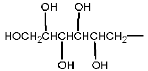 | 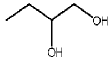 | H | H | | |
| Working example 90 | 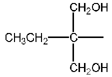 | 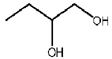 | 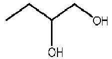 | H | Unsaturated aliphatic monocarboxylic acid anion | CH₃(CH₂)₇CH=CH(C H₂)₇COO⁻ |
| Working example 91 | 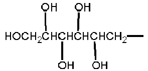 | 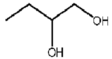 | 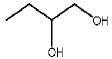 | H | | |
| Working example 92 | 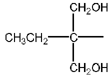 | 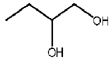 | 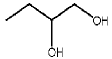 | H | Saturated dicarboxylic acid anion | HOOC(CH₂)₂COO⁻ |
| Working example 93 | 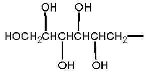 | 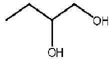 | 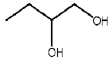 | H | | |
| Working example 94 | 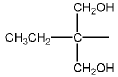 | 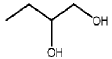 | 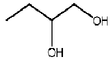 | 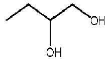 | Saturated aliphatic monocarboxylic | CH₃(CH₂)₈CH((CH₂)₆ CH₃)COO⁻ |
| Working example 95 | 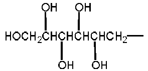 | 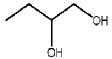 | 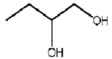 | 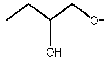 | Saturated dicarboxylic acid anion | HOOC(CH₂)₂COO⁻ |
| Working example 96 | 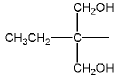 | 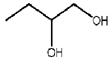 | 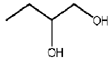 | 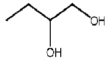 | | |
| Working example 97 | 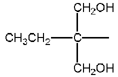 | 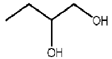 | 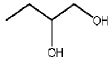 | 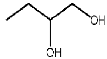 | Saturated hydroxydi- or tricarboxylic acid anion | HOOCCH₂C(OH)(CO OH)CH₂COO⁻ |
| Working example 98 | 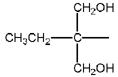 | 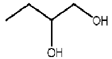 | 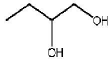 | 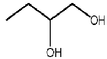 | Aromatic monocarboxylic acid anion | Salicylic acid |
| Working example 99 | 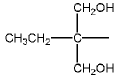 | 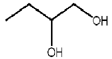 | 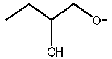 | 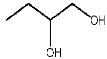 | Alkylether carboxylic acid anion | CH₃CH₂-O-CH₂COO⁻ |
| Working example 100 | 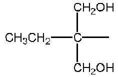 | 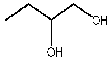 | 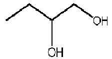 | 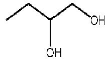 | Halogen carboxylic acid anion | CF₃COO⁻ |
| Working example 101 | 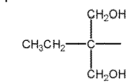 | 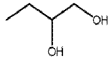 | 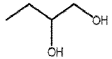 | 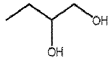 | Halide anion | Br⁻ |
| Working example 102 | 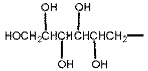 | 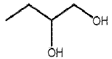 | 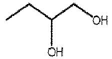 | 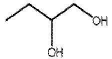 | | |
| Working example 103 | 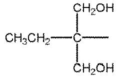 | 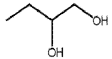 | 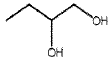 | 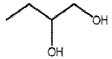 | Sulfur-based anion | CH₃SO₃⁻ |
| Working example 104 | 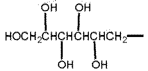 | 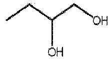 | 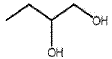 | 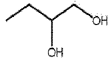 | | |
| Working example 105 | 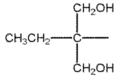 | 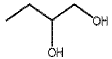 | 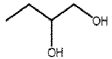 | 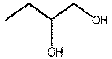 | Fluorine-based anion | BF₄⁻ |
| Working example 106 | 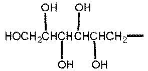 | 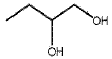 | 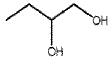 | 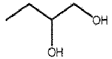 | | |
| Working example 107 | 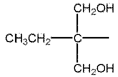 | 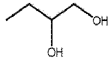 | 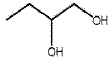 | 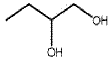 | Nitrogen oxide-based anion | NO₃⁻ |
| Working example 108 | 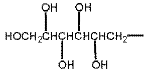 | 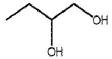 | 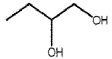 | 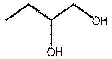 | | |
| Working example 109 | 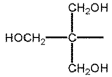 | H | H | H | Saturated dicarboxylic acid anion | ⁻OOC(CH₂)₂COO⁻ |

### <Comparative example 1> Compound 110

### Tributyl ammonium lactate

The compound was synthesized by a method described in JP-A-2014-131974.

### <Comparative example 2> Compound 111

### Choline acetate

The compound was synthesized using choline hydroxide and acetic acid, with reference to the method described in JP-A-2014-131974.

### <Comparative example 3> Compound 112

1-butyl-3-methylimidazolium bromide

The compound was synthesized using 1-butyl-3-methylimidazolium tetrafluoroborate, an ion-exchange resin and hydrobromic acid, with reference to a method described in JP-A-2016-041682.

### <Comparative example 4> Compound 113

As D(+)-glucose, a reagent produced by KANTO CHEMICAL CO., INC. was used.

### <Comparative example 5> Compound 114

As gelatin, nippi peptide FCP-AS-L (by Nippi. Inc.) was used.

### <Comparative example 6> Compound 115

As albumin, albumin produced by NACALAI TESQUE, INC. (bovine origin, general grade, pH 5.2) was used.

### <Comparative example 7> Compound 116

As urease, a reagent produced by Wako Pure Chemical Industries, Ltd. (sword bean origin) was used.

### <Comparative example 8> Compound 117

As a buffer for urease, there was used a 10 mM aqueous solution of potassium dihydrogen phosphate whose pH level had been adjusted to 7.5 with sodium hydroxide.

### <Comparative example 9> Compound 118

As cytochrome C, a reagent produced by NACALAI TESQUE, INC. (Horse Heart, molecular weight 12384) was used.

### <Comparative example 10> Compound 119

As a buffer for cytochrome C and DNA, there was used a 50 mM phosphate buffer of pH 7.4 prepared using 50 mM potassium dihydrogen phosphate and 50 mM dipotassium hydrogen phosphate.

### <Comparative example 11> Compound 120

DNA produced by the method described in JP-A-2014-131974 was used.

### <Comparative example 12> Compound 121

As tetra-n-butylammonium bromide, a reagent produced by KANTO CHEMICAL CO., INC. was used.

### <Reference example 1> Compound 122

The compound was synthesized by the method described in JP-A-2014-131974.

### <Reference example 2> Compound 123

The compound was synthesized by the method described in JP-A-2014-131974.

The following measurements and evaluations were performed on the compounds of the above working and comparative examples.

### 1. Condition at room temperature (25°C)

Each of the compounds of the working examples 1 to 109 was added into a screw cap tube and then turned into an anhydride when dried under a reduced pressure; the condition (liquid, solid) of each compound as an anhydride at room temperature (25°C) was observed. The results are shown in Tables 7 to 10. Here, in Tables 7 to 10, "solid" refers to a state where the compound is solid at room temperature (25°C). As a result of performing the drying treatment under a reduced pressure, the compounds of the working examples 1 to 109 were all solid at room temperature (25°C).

Thus, as compared to a conventional organic ammonium salt, it became clear that in the case of the organic ammonium salt of the present invention, by employing a polyhydroxyalkyl group, the hydrogen bonds in the molecule shall establish a strong cation structure, which makes it easier for crystallization to take place, and thereby allows the melting point to be impacted by selecting functional groups and characteristic groups in structural design and a wide range of various anions to be applied.

**[Table 7]**

| Compound | Raw material | | Molar ratio | | Property of product at room temperature m.p(°C) |
|---|---|---|---|---|---|
| | Amine-based compound | Acid | Am ine-based compound | Acid | |
| Working example1 | N(CH₂CH₂OH)₃ | Formic acid | 1 | 1 | solid 25°C< |
| Working example2 | NH₂C(CH₂CH₃)(CH₂OH)₂ | Acetic acid | 1 | 1 | solid 25°C< |
| Working example3 | NH₂C(CH₂OH)₃ | Acetic acid | 1 | 1 | solid 25°C< |
| Working example4 | NH₂CH(CH₂OH)₂ | Butyric acid | 1 | 1 | solid 25°C< |
| Working example5 | NH₂C(CH₂OH)₃ | Butyric acid | 1 | 1 | solid 25°C< |
| Working example6 | NH₂(CH₂)(CHOH)₄CH₂OH | Butyric acid | 1 | 1 | solid 25°C< |
| Working example7 | NH₂(CH₂CH₂OH) | Caproic acid | 1 | 1 | solid 25°C< |
| Working example8 | NH₂CH(CH₂OH)₂ | Caproic acid | 1 | 1 | solid 25°C< |
| Working example9 | NH₂C(CH₂OH)₃ | Caproic acid | 1 | 1 | solid 25°C< |
| Working example10 | NH₂(CH₂)(CHOH)₄CH₂OH | Caproic acid | 1 | 1 | solid 25°C< |
| Working example11 | NH₂(CH₂CH₂OH) | Caprylic acid | 1 | 1 | solid 25°C< |
| Working example12 | N(CH₂CH₂OH)₃ | Caprylic acid | 1 | 1 | solid 25°C< |
| Working example13 | NH₂CH(CH₂OH)₂ | Caprylic acid | 1 | 1 | solid 25°C< |
| Working example14 | NH₂C(CH₂CH₃)(CH₂OH)₂ | Caprylic acid | 1 | 1 | solid 25°C< |
| Working example15 | NH₂C(CH₂OH)₃ | Caprylic acid | 1 | 1 | solid 25°C< |
| Working example16 | NH₂(CH₂)(CHOH)₄CH₂OH | Caprylic acid | 1 | 1 | solid 25°C< |
| Working example17 | NH₂(CH₂CH₂OH) | Capric acid | 1 | 1 | solid 25°C< |
| Working example18 | N(CH₂CH₂OH)₃ | Capric acid | 1 | 1 | solid 25°C< |
| Working example19 | NH₂CH(CH₂OH)₂ | Capric acid | 1 | 1 | solid 25°C< |
| Working example20 | NH₂C(CH₂CH₃)(CH₂OH)₂ | Capric acid | 1 | 1 | solid 25°C< |
| Working example21 | NH₂C(CH₂OH)₃ | Capric acid | 1 | 1 | solid 25°C< |
| Working example22 | NH₂(CH₂)(CHOH)₄CH₂OH | Capric acid | 1 | 1 | solid 25°C< |
| Working example23 | NH₂(CH₂CH₂OH) | Lauric acid | 1 | 1 | solid 25°C< |
| Working example24 | NH(CH₂CH₂OH)₂ | Lauric acid | 1 | 1 | solid 25°C< |
| Working example25 | N(CH₂CH₂OH)₃ | Lauric acid | 1 | 1 | solid 25°C< |
| Working example26 | NH₂CH(CH₂OH)₂ | Lauric acid | 1 | 1 | solid 25°C< |
| Working example27 | NH₂C(CH₂CH₃)(CH₂OH)₂ | Lauric acid | 1 | 1 | solid 25°C< |
| Working example28 | NH₂C(CH₂OH)₃ | Lauric acid | 1 | 1 | solid 25°C< |
| Working example29 | NH₂(CH₂)(CHOH)₄CH₂OH | Lauric acid | 1 | 1 | solid 25°C< |
| Working example30 | NH₂(CH₂CH₂OH) | Cyclohexane carboxylic acid | 1 | 1 | solid 25°C< |

**[Table 8]**

| Compound | Raw material | | Molar ratio | | Property of product at room temperature m.p(°C) |
|---|---|---|---|---|---|
| | Am ine-based compound | Acid | Am ine-based com pound | Acid | |
| Working example31 | N(CH₂CH₂OH)₃ | Cyclohexane carboxylic acid | 1 | 1 | solid 25°C< |
| Working example32 | NH₂CH(CH₂OH)₂ | Cyclohexane carboxylic acid | 1 | 1 | solid 25°C< |
| Working example33 | NH₂(CH₂CH₂OH) | Crotonic acid | 1 | 1 | solid 25°C< |
| Working example34 | NH₂C(CH₂CH₃)(CH₂OH)₂ | Oleic acid | 1 | 1 | solid 25°C< |
| Working example35 | NH₂C(CH₂CH₃)(CH₂OH)₂ | Glycolic acid | 1 | 1 | solid 25°C< |
| Working example36 | NH₂C(CH₂OH)₃ | Glycolic acid | 1 | 1 | solid 25°C< |
| Working example37 | NH₂CH(CH₂OH)₂ | 9,10-dihydroxystearic acid | 1 | 1 | solid 25°C< |
| Working example38 | NH₂C(CH₂CH₃)(CH₂OH)₂ | 9,10-dihydroxystearic acid | 1 | 1 | solid 25°C< |
| Working example39 | NH₂C(CH₂OH)₃ | 9,10-dihydroxystearic acid | 1 | 1 | solid 25°C< |
| Working example40 | NH₂(CH₂)(CHOH)₄CH₂OH | 9,10-dihydroxystearic acid | 1 | 1 | solid 25°C< |
| Working example41 | NH₂(CH₂CH₂OH) | Quinic acid | 1 | 1 | solid 25°C< |
| Working example42 | NH(CH₂CH₂OH)₂ | Quinic acid | 1 | 1 | solid 25°C< |
| Working example43 | NH₂CH(CH₂OH)₂ | Quinic acid | 1 | 1 | solid 25°C< |
| Working example44 | NH₂C(CH₂CH₃)(CH₂OH)₂ | Quinic acid | 1 | 1 | solid 25°C< |
| Working example45 | NH₂CH(CH₂OH)₂ | 12-hydroxystearic acid | 1 | 1 | solid 25°C< |
| Working example46 | NH₂CH(CH₂OH)₂ | Oxalic acid | 1 | 1 | solid 25°C< |
| Working example47 | NH₂(CH₂CH₂OH) | Succinic acid | 1 | 1 | solid 25°C< |
| Working example48 | NH₂C(CH₂OH)₃ | Succinic acid | 1 | 1 | solid 25°C< |
| Working example49 | NH₂(CH₂CH₂OH) | Adipic acid | 1 | 1 | solid 25°C< |
| Working example50 | N(CH₂CH₂OH)₃ | Adipic acid | 1 | 1 | solid 25°C< |
| Working example51 | NH₂CH(CH₂OH)₂ | Adipic acid | 1 | 1 | solid 25°C< |
| Working example52 | NH₂CH(CH₂OH)₂ | Sebacic acid | 1 | 1 | solid 25°C< |
| Working example53 | NH₂CH(CH₂OH)₂ | Fumaric acid | 1 | 1 | solid 25°C< |
| Working example54 | NH₂(CH₂CH₂OH) | Tartaric acid | 1 | 1 | solid 25°C< |
| Working example55 | NH₂(CH₂CH₂OH) | Citric acid | 1 | 1 | solid 25°C< |
| Working example56 | NH₂(CH₂CH₂OH) | Benzoic acid | 1 | 1 | solid 25°C< |
| Working example57 | N(CH₂CH₂OH)₃ | Benzoic acid | 1 | 1 | solid 25°C< |
| Working example58 | NH₂CH(CH₂OH)₂ | Benzoic acid | 1 | 1 | solid 25°C< |
| Working example59 | NH₂C(CH₂CH₃)(CH₂OH)₂ | Benzoic acid | 1 | 1 | solid 25°C< |
| Working example60 | NH₂C(CH₂OH)₃ | Benzoic acid | 1 | 1 | solid 25°C< |

**[Table 9]**

| Compound | Raw material | | Molar ratio | | Property of product at room temperature m.p(°C) |
|---|---|---|---|---|---|
| | Amine-based compound | Carboxylic acid | Am ine-based com pound | Acid | |
| Working example61 | NH₂(CH₂)(CHOH)₄CH₂OH | Benzoic acid | 1 | 1 | solid 25°C< |
| Working example62 | NH₂(CH₂CH₂OH) | Terephthalic acid | 1 | 1 | solid 25°C< |
| Working example63 | NH(CH₂CH₂OH)₂ | Terephthalic acid | 1 | 1 | solid 25°C< |
| Working example64 | N(CH₂CH₂OH)₃ | Terephthalic acid | 1 | 1 | solid 25°C< |
| Working example65 | NH₂CH(CH₂OH)₂ | Terephthalic acid | 1 | 1 | solid 25°C< |
| Working example66 | NH₂(CH₂CH₂OH) | Salicylic acid | 1 | 1 | solid 25°C< |
| Working example67 | NH₂C(CH₂CH₃)(CH₂OH)₂ | Salicylic acid | 1 | 1 | solid 25°C< |
| Working example68 | NH₂C(CH₂OH)₃ | Salicylic acid | 1 | 1 | solid 25°C< |
| Working example69 | NH₂(CH₂CH₂OH) | p-hydroxybenzoic acid | 1 | 1 | solid 25°C< |
| Working example70 | N(CH₂CH₂OH)₃ | p-hydroxybenzoic acid | 1 | 1 | solid 25°C< |
| Working example71 | NH₂CH(CH₂OH)₂ | p-hydroxybenzoic acid | 1 | 1 | solid 25°C< |
| Working example72 | NH₂(CH₂CH₂OH) | Mandelic acid | 1 | 1 | solid 25°C< |
| Working example73 | NH₂(CH₂)(CHOH)₄CH₂OH | Pyruvic acid | 1 | 1 | solid 25°C< |
| Working example74 | NH₂C(CH₂CH₃)(CH₂OH)₂ | Methoxyacetic acid | 1 | 1 | solid 25°C< |
| Working example75 | NH₂(CH₂CH₂OH) | Trifluoroacetic acid | 1 | 1 | solid 25°C< |
| Working example76 | NH₂C(CH₂CH₃)(CH₂OH)₂ | Trifluoroacetic acid | 1 | 1 | solid 25°C< |
| Working example77 | NH₂C(CH₂OH)₃ | Trifluoroacetic acid | 1 | 1 | solid 25°C< |
| Working example78 | NH₂C(CH₂OH)₃ | Hydrobromic acid | 1 | 1 | solid 25°C< |
| Working example79 | NH₂C(CH₂OH)₃ | Hydrochloric acid | 1 | 1 | solid 25°C< |
| Working example80 | NH₂CH(CH₂OH)₂ | Trifluoromethanesulfonyl imide | 1 | 1 | solid 25°C< |
| Working example81 | NH₂C(CH₂OH)₃ | Sulfuric acid | 2 | 1 | solid 25°C< |
| Working example82 | NH₂(CH₂)(CHOH)₄CH₂OH | Sulfuric acid | 2 | 1 | solid 25°C< |
| Working example83 | NH₂C(CH₂OH)₃ | Phosphoric acid | 1 | 1 | solid 25°C< |
| Working example84 | NH₂(CH₂)(CHOH)₄CH₂OH | Phosphoric acid | 1 | 1 | solid 25°C< |
| Working example85 | NH₂CH(CH₂OH)₂ | Sodium dicyanamide | 1 | 1 | solid 25°C< |
| Working example86 | NH₂C(CH₂OH)₃ | Sodium dicyanamide | 1 | 1 | solid 25°C< |
| Working example87 | NH₂(CH₂)(CHOH)₄CH₂OH | Sodium dicyanamide | 1 | 1 | solid 25°C< |

**[Table 10]**

| Compound | Raw material | | Molar ratio | | Property of product at room tem perature m.p(°C) |
|---|---|---|---|---|---|
| | Am ine-based compound | Carboxylic acid | Am ine-based com pound | Acid | |
| Working example88 | NH(CH₂CHOHCH₂OH)(C(CH₂CH₃)(CH₂OH)₂) | Succinic acid | 1 | 1 | solid 25°C < |
| Working example89 | NH(CH₂CHOHCH₂OH)(CH₂(CHOH)₄CH₂OH) | Succinic acid | 1 | 1 | solid 2°C < |
| Working example90 | N(CH₂CHOHCH₂OH)2(C(CH₂CH₃)(CH₂OH)₂) | Oleic acid | 1 | 1 | solid 25°C< |
| Working example91 | N(CH₂CHOHCH₂OH)₂(CH₂(CHOH)₄CH₂OH) | Oleic acid | 1 | 1 | solid 2°C< |
| Working example92 | N(CH₂CHOHCH₂OH)₂(C(CH₂CH₃(CH₂OH)₂) | Succinic acid | 1 | 1 | solid 25°C < |
| Working example93 | N(CH₂CHOHCH₂OH)₂(CH₂(CHOH)₄CH₂OH) | Succinic acid | 1 | 1 | solid 25°C < |
| Working example94 | N⁺(CH₂CHOHCH₂OH)₃(C(CH₂CH₃)(CH₂OH)₂) OH⁻ | Isostearic acid | 1 | 1 | solid 25°C < |
| Working example95 | N⁺(CH₂CHOHCH₂OH)₃(CH₂(CHOH)₄CH₂OH) OH⁻ | Succinic acid | 1 | 1 | solid 25°C< |
| Working example96 | N⁺(CH₂CHOHCH₂OH)₃(C(CH₂CH₃)(CH₂OH)₂) OH⁻ | Succinic acid | 1 | 1 | solid 25°C < |
| Working example97 | N⁺(CH₂CHOHCH₂OH)₃(C(CH₂CH₃)(CH₂OH)₂) OH⁻ | Citric acid | 1 | 1 | solid 25°C< |
| Working example98 | N⁺(CH₂CHOHCH₂OH)₃(C(CH₂CH₃)(CH₂OH)₂) OH⁻ | Salicylic acid | 1 | 1 | solid 25°C< |
| Working example99 | N⁺(CH₂CHOHCH₂OH)₃(C(CH₂CH₃)(CH₂OH)₂) OH⁻ | Ethoxyacetic acid | 1 | 1 | solid 25°C < |
| Working example100 | N⁺(CH₂CHOHCH₂OH)₃(C(CH₂CH₃)(CH₂OH)₂) OH⁻ | Trifluoroacetic acid | 1 | 1 | solid 25°C < |
| Working example101 | N⁺(CH₂CHOHCH₂OH)₃(C(CH₂CH₃)(CH₂OH)₂) OH⁻ | Hydrobromic acid | 1 | 1 | solid 25°C < |
| Working example102 | N⁺(CH₂CHOHCH₂OH)₃(CH₂(CHOH)₄CH₂OH) OH⁻ | Hydrobromic acid | 1 | 1 | solid 25°C < |
| Working example103 | N⁺(CH₂CHOHCH₂OH)₃(C(CH₂CH₃)(CH₂OH)₂) OH⁻ | Methanesulfonic acid | 1 | 1 | solid 25°C< |
| Working example104 | N⁺(CH₂CHOHCH₂OH)₃(CH₂(CHOH)₄CH₂OH) OH⁻ | Methanesulfonic acid | 1 | 1 | solid 25°C < |
| Working example105 | N⁺(CH₂CHOHCH₂OH)₃(C(CH₂CH₃)(CH₂OH)₂) OH⁻ | Tetrafluoroboric acid | 1 | 1 | solid 25°C < |
| Working example106 | N⁺(CH₂CHOHCH₂OH)₃(CH₂(CHOH)₄CH₂OH) OH⁻ | Tetrafluoroboric acid | 1 | 1 | solid 25°C < |
| Working example107 | N⁺(CH₂CHOHCH₂OH)₃(C(CH₂CH₃)(CH₂OH)₂) OH⁻ | Nitric acid | 1 | 1 | solid 25°C < |
| Working example108 | N⁺(CH₂CHOHCH₂OH)₃(CH₂(CHOH)₄CH₂OH) OH⁻ | Nitric acid | 1 | 1 | solid 2°C< |
| Working example109 | NH₂C(CH₂OH)₃ | Succinic acid | 2 | 1 | solid 25°C < |

### 2. Enzyme long-term stability (1)

The compounds shown in Table 11 were each used as a stabilizer, and a 50% aqueous solution of the compound was prepared. Urease was then added and dissolved therein so that the enzyme concertation(s) shown in Table 11 would be achieved, followed by distilling away water under a reduced pressure. After distilling away water at a reduced pressure, a solid sample obtained was placed in a thermo-hygrostat set to a condition of 40°C, 80%RH which were higher than a temperature and humidity generally employed to preserve enzymes. After leaving the sample therein for a given period of time, each sample was then collected so as to measure an activity retention rate of the enzyme preserved in each compound by a method described below, thereby making it possible to confirm the retainability of the steric structure of the enzyme in each compound and a stabilization effect.

### <Hydrolase: measurement of urease activity>

The activity of urease was measured in such a way that the ammonium ions produced from urea by decomposition owing to the enzyme reaction of urease were quantified by the indophenol method.

At first, 100 mL of a 1 mM substrate solution (prepared by dissolving urea as a substrate into a 10 mM phosphate buffer solution of pH 7.5) was put into an Erlenmeyer flask, and then preliminarily warmed at 30°C for about 30 min.

Next, the sample left at the given concentration and temperature shown in Table 11 for a given period of time was added to the abovementioned substrate solution so that an enzyme content would be 0.5 mg, and was then left to react at 30°C for 60 min.

After the reaction was over, 0.1 mL of the reaction solution was collected, followed by immediately adding thereto 2 mL of a phenol solution (prepared by dissolving 10 g of phenol and 50 mg of sodium pentacyanonitrosylferrate (III) into an ion-exchange water, and then using the ion-exchange water to dilute them in a measuring cylinder so as to achieve a volume of 1,000 mL) and 2 mL of a sodium hypochlorite solution (prepared by dissolving 5 g of sodium hydroxide and 8.4 mL of a 5% sodium hypochlorite solution into an ion-exchange water, and then using the ion-exchange water to dilute them in a measuring cylinder so as to achieve a volume of 1,000 mL), and then reacting them in a thermostat bath of 37°C for 20 min.

An absorbance of this reaction solution at a wavelength of 635 nm was measured by an ultraviolet-visible spectrophotometer (V-550 by JASCO Corporation), followed by obtaining a production amount of ammonium ions from an indophenol content achieved, and then calculating the urease activity. The quantification of the ammonium ions was performed in a way such that there was prepared an ammonium ion solution at a concertation of 0.1 to 3.0 mM, and there was used a calibration curve obtained by conducting quantification via the indophenol method as above.

Here, a value of enzyme activity as a reference of the enzyme activity retention rate was calculated as follows. An enzyme solution having an enzyme concentration of 50 mg/mL was prepared by dissolving a urease powder stored at a proper temperature into a buffer (10 mM phosphate buffer solution of pH 7.5). After preparation, the solution was then immediately added to the substrate solution so that the enzyme content would be 0.5 mg as above; after the enzyme reaction was over, the enzyme activity retention rate was calculated on the basis of the ammonium ion content quantified by the indophenol method.

The results in Table 11 under the condition of 40°C show that while the activity retention rate had dropped to 0 to 3% after 30 days in the cases of an enzyme preserved in an imidazolium-based organic ammonium salt, an enzyme preserved in a general additive, and an enzyme to which no additive was added, the activity retention rates after 30 days in the cases of the compounds of the present invention were higher than those of the comparative examples at any of the ratios of stabilizer : enzyme.

Further, in the cases of the reference examples 1 and 2 where the compounds were liquid at 25°C, preservation was difficult when the ratio of stabilizer: enzyme was 1:1 and 0.1:1 as the enzyme was unable to be dissolved therein; in the cases of the compounds of the present invention, since mixed were both solids, the enzyme was able to be preserved at a high concentration.

That is, it was indicated that under a high-concentration, high-temperature and long-term condition, the compounds of the present invention are capable of retaining the activity of an enzyme, and ensuring a high retainability of the steric structure of the enzyme.

**[Table 11]**

| Compound | 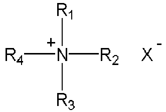 | | | | | Enzyme activity retention rate(%) (after 30 days) | | |
|---|---|---|---|---|---|---|---|---|
| | | | | | | Stabilizer: Enzyme = 10 : 1 | Stabilizer: Enzyme = 1 : 1 | Stabilizer: Enzyme = 0.1 : 1 |
| | R₁ | R₂ | R₃ | R₄ | X⁻ | | | |
| Working example23 | (CH₂)₂OH | H | H | H | CH₃(CH₂)₁₀COO⁻ | 41 | 5 | 8 |
| Working example24 | (CH₂)₂OH | (CH₂)₂OH | H | H | CH₃(CH₂)₁₀COO⁻ | 25 | 25 | 9 |
| Working example25 | (CH₂)₂OH | (OH₂)₂OH | (OH₂)₂OH | H | CH₃(CH₂)₁₀COO⁻ | 32 | 41 | 14 |
| Working example26 | 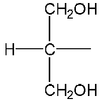 | H | H | H | CH₃(CH₂)₁₀COO⁻ | 25 | 81 | 10 |
| Working example27 | 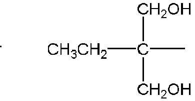 | H | H | H | CH₃(CH₂)₁₀COO⁻ | 20 | 74 | 8 |
| Working example35 | | | | | HOCH₂COO⁻ | 22 | 16 | 9 |
| Working example3 | 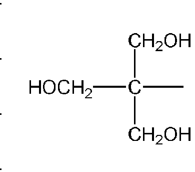 | H | H | H | CH₃COO⁻ | 53 | 50 | 20 |
| Working example28 | | | | | CH₃(CH₂)₁₀COO⁻ | 35 | 65 | 15 |
| Working example36 | | | | | HOCH₂COO⁻ | 45 | 41 | 27 |
| Working example48 | | | | | HOOC(CH₂)₂COO⁻ | 52 | 48 | 18 |
| Working example60 | | | | | Ph-COO⁻ | 29 | 20 | 7 |
| Working example78 | | | | | Br⁻ | 18 | 4 | 3 |
| Working example83 | | | | | H₂PO₄⁻ | 38 | 24 | 16 |
| Working example29 | 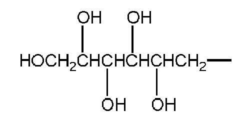 | H | H | H | CH₃(CH₂)₁₀COO⁻ | 21 | 18 | 5 |
| Comparative example1 | CH₃(CH₂)₃ | CH₃(CH₂)₃ | CH₃(CH₂)₃ | CH₃(CH₂)₃ | CH₃CH(OH)COO⁻ | 0 | 0 | 0 |
| Comparative example2 | CH₃ | CH₃ | CH₃ | (CH₂)₂OH | CH₃COO⁻ | 0 | 3 | 2 |
| Comparative example3 | BMI-Br | | | | | 0 | 0 | 0 |
| Comparative example4 | Glucose | | | | | 0 | 0 | 0 |
| Comparative example5 | Gelatin | | | | | 0 | 0 | 0 |
| Comparative example6 | Albumin | | | | | 0 | 0 | 0 |
| Comparative example7 | No additive (enzyme only) | | | | | 0 | 0 | 0 |
| Reference example1 | (CH₂)₂OH | (CH₂)₂OH | (CH₂)₂OH | H | CH₃COO⁻ | 52 | - (Enzyme insoluble) | - (Enzyme insoluble) |
| Reference example2 | 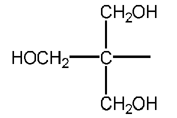 | H | H | H | CH₃CH(OH)COO⁻ | 58 | - (Enzyme insoluble) | - (Enzyme insoluble) |

A 50% aqueous solution of each compound shown in Table 12 was prepared, followed by adding and dissolving urease thereinto so that the enzyme concentration shown in Table 12 would be achieved. The solution was then left in a thermo-hygrostat set to a condition of 40°C, 80%RH which were higher than a temperature and humidity generally employed to preserve enzymes. After leaving the solution therein for a given period of time, each sample was then collected so as to measure the activity retention rate of the enzyme preserved in each compound by a method described below, thereby making it possible to confirm the retainability of the steric structure of the enzyme in each compound and the stabilization effect.

The results in Table 12 concerning the state of an aqueous solution and under the condition of 40°C show that while the activity retention rate had dropped to 0 to 2% after 30 days in the cases of an enzyme preserved in an imidazolium-based organic ammonium salt, an enzyme preserved in a 50% aqueous solution of a general additive, and an enzyme preserved in a buffer, the activity retention rates after 30 days in the cases of the 50% aqueous solutions containing the compounds of the present invention were higher than those of the comparative examples.

That is, it was indicated that under a high-concentration, high-temperature and long-term condition, the compounds of the present invention, regardless of whether in the state of a solid or an aqueous solution, are capable of retaining the activity of an enzyme, and ensuring a high retainability of the steric structure of the enzyme.

**[Table 12]**

| Compound | 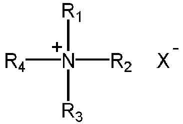 | | | | | Aqueous solution concentration (wt%) | Enzyme activity retention rate (%) (after 30 days) |
|---|---|---|---|---|---|---|---|
| | | | | | | | Stabilizer: Enzyme = 1 : 1 |
| | R₁ | R₂ | R₃ | R₄ | X⁻ | | |
| Working example23 | (CH₂)₂OH | H | H | H | CH₃(CH₂)₁₀COO⁻ | 50 | 3 |
| Working example24 | (CH₂)₂OH | (CH₂)₂OH | H | H | CH₃(CH₂)₁₀COO⁻ | 50 | 10 |
| Working example25 | (CH₂)₂OH | (CH₂)₂OH | (CH₂)₂OH | H | CH₃(CH₂)₁₀COO⁻ | 50 | 11 |
| Working example26 | 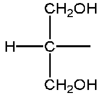 | H | H | H | CH₃(CH₂)₁₀COO⁻ | 50 | 18 |
| Working example27 | 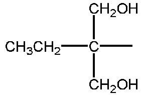 | H | H | H | CH₃(CH₂)₁₀COO⁻ | 50 | 11 |
| Working example35 | | | | | HOCH₂COO⁻ | 50 | 10 |
| Working example3 | 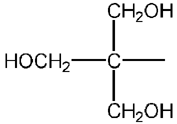 | H | H | H | CH₃COO⁻ | 50 | 32 |
| Working example28 | | | | | CH₃(CH₂)₁₀COO⁻ | 50 | 15 |
| Working example36 | | | | | HOCH₂COO⁻ | 50 | 39 |
| Working example48 | | | | | HOOC(CH₂)₂COO⁻ | 50 | 30 |
| Working example60 | | | | | Ph-COO⁻ | 50 | 21 |
| Working example78 | | | | | Br | 50 | 3 |
| Working example83 | 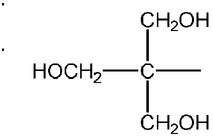 | H | H | H | H₂PO₄⁻ | 50 | 20 |
| Working example29 | 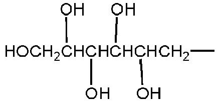 | H | H | H | CH₃(CH₂)₁₀COO⁻ | 50 | 11 |
| Comparative example1 | CH₃(CH₂)₃ | CH₃(CH₂)₃ | CH₃(CH₂)₃ | CH₃(CH₂)₃ | CH₃CH(OH)COO⁻ | 50 | 0 |
| Comparative example2 | CH₃ | CH₃ | CH₃ | (CH₂)₂OH | CH₃COO⁻ | 50 | 2 |
| Comparative example3 | BMI-Br | | | | | 50 | 0 |
| Comparative example4 | Glucose | | | | | 50 | 0 |
| Comparative example6 | Albumin | | | | | 50 | 0 |
| Comparative example8 | Buffer | | | | | 10mM | 0 |

### 3. Protein long-term stability

A 50% aqueous solution of each compound shown in Table 13 was prepared, followed by adding and dissolving cytochrome C thereinto so that the protein concentrations shown in Table 13 would be achieved, and then distilling away water under a reduced pressure. After distilling away water at a reduced pressure, a solid sample obtained was placed in a thermo-hygrostat set to a condition of 40°C, 80%RH which were higher than a temperature and humidity generally employed to preserve proteins. After leaving the sample therein for a given period of time, each sample was then collected so as to measure the IR spectrum and UV spectrum of the protein preserved in each compound by a method described below, thereby making it possible to confirm the long-term stability of the protein.

At first, changes in amide absorption by IR spectrum were precisely observed, and there were confirmed the higher-order structures of protein (turn, α-helix, random coil, β-sheet). The amide I region (1,600 to 1,700 cm⁻¹) and the amide II region (1,500 to 1,600 cm⁻¹) were measured by ATR method using a Fourier transform infrared spectrophotometer (FT/IR-6100 by JASCO Corporation), and peaks were then detected based on a difference(s) between a cytochrome C-free organic ammonium salt sample (blank) and a cytochrome C-containing organic ammonium salt sample (sample).

Further, the activity state of cytochrome C (Fe²⁺: reduced form, Fe³⁺: oxidized form) was confirmed via absorptions in UV spectrum. The measurement was conducted using an ultraviolet-visible spectrophotometer and a quartz cell having an optical path width of 2 mm, immediately after diluting the cytochrome C-containing organic ammonium salt sample to 1% with a 50 mM phosphate buffer of pH 7.4 (prepared using 50 mM potassium dihydrogen phosphate and 50 mM dipotassium hydrogen phosphate).

The results thereof are shown in Table 13. There, "o" was given to samples exhibiting a long-term stability with regard to protein; "x" was given to samples not exhibiting a long-term stability with regard to protein.

In the identification of amide absorption by IR spectrum, the higher-order structures of protein were confirmed by comparison to literature values (Chem. Commun 2005, 4804-4806, Biomacromolecules 2010, 11, 2944-2948, Protein Science Society of Japan archive, 2, e054 (2009)). As a result of conducting an IR measurement on the powder of cytochrome C alone, absorptions were observed at 1,645 cm⁻¹ in the amide I region and at 1,537 cm⁻¹ in the amide II region; the protein had denatured to random coil. Next, the cytochrome C in the phosphate buffer did not denature (amide I region: 1,653 cm⁻¹, amide II region: 1,547 cm⁻¹), and was confirmed to have an α-helix structure. The cytochrome C in the organic ammonium salt of each working example exhibited measurement results of a level similar to that of the cytochrome C in the phosphate buffer (amide I region: 1,652 to 1,655 cm⁻¹, amide II region: 1,545 to 1,549 cm⁻¹); the cytochrome C preserved in the organic ammonium salt of each working example was confirmed to have maintained an α-helix structure rather than a denatured structure. That is, by dissolving a solid of an inactive and denatured protein (cytochrome C) with the organic ammonium salt of the product of the present invention, the activity of the protein was expressed; it was indicated that a refolding effect was exhibited such that cytochrome was able to be retained without undergoing denaturalization.

Cytochrome C is such that when conducting electron transfer in the cells, the state thereof shall be reversibly changed between Fe²⁺ (reduced form) and Fe³⁺ (oxidized form); and that when in an active state, a secondary structure is maintained, where in terms of absorption in a UV spectrum, the reduced form respectively has peaks near 550 nm in the α band, 521 nm in the β band and 415 nm in the γ band, whereas the oxidized form has no clear peaks in the α band and β band, and exhibits a shift to a lower wavelength near 396 nm in the γ band. In a deactivated state, cytochrome C will denature such that the peaks in the α, β and γ bands will disappear. As compared to the peaks in the comparative examples, peaks of the reduced form were observed in each of the α, β and γ bands in the working examples. In this way, it was confirmed that the cytochrome C preserved in the organic ammonium salt of the product of the present invention had maintained a secondary structure in an active state of the reduced from.

That is, it was indicated that the product of the present invention is an excellent preserving material for proteins, which does not denature proteins over a long period of time even under a high-temperature condition; and that the product is also useful as a protein refolding agent.

**[Table 13]**

| Compound | 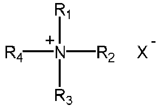 | | | | | Protein long-term stability (after 30 days) | | |
|---|---|---|---|---|---|---|---|---|
| | | | | | | Stabilizer: Protein = 10 : 1 | Stabilizer: Protein =1:1 | Stabilizer: Protein = 0.1 : 1 |
| | R₁ | R₂ | R₃ | R₄ | X⁻ | | | |
| Working example23 | (CH₂)₂OH | H | H | H | CH₃(CH₂)₁₀COO⁻ | O | O | O |
| Working example24 | (CH₂)₂OH | (CH₂)₂OH | H | H | CH₃(CH₂)₁₀COO⁻ | O | O | O |
| Working example25 | (CH₂)₂OH | (CH₂)₂OH | (CH₂)₂OH | H | CH₃(CH₂)₁₀COO⁻ | O | O | O |
| Working example26 | 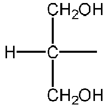 | H | H | H | CH₃(CH₂)₁₀COO⁻ | O | O | O |
| Working example27 | 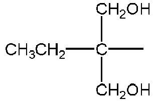 | H | H | H | CH₃(CH₂)₁₀COO⁻ | O | O | O |
| Working example35 | | | | | HOCH₂COO⁻ | O | O | O |
| Working example3 | 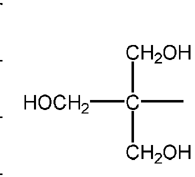 | H | H | H | CH₃COO⁻ | O | O | O |
| Working example28 | | | | | CH₃(CH₂)₁₀COO⁻ | O | O | O |
| Working example36 | | | | | HOCH₂COO⁻ | O | O | O |
| Working example48 | | | | | HOOC(CH₂)₂COO⁻ | O | O | O |
| Working example60 | | | | | Ph-COO⁻ | O | O | O |
| Working example78 | | | | | Br⁻ | O | O | O |
| Working example83 | | | | | H₂PO₄⁻ | O | O | O |
| Working example29 | 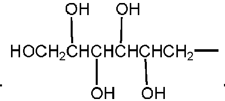 | H | H | H | CH₃(CH₂)₁₀COO⁻ | O | O | O |
| Comparative example1 | CH₃(CH₂)₃ | CH₃(CH₂)₃ | CH₃(CH₂)₃ | CH₃(CH₂)₃ | CH₃CH(OH)COO⁻ | × | × | × |
| Comparative example2 | CH₃ | CH₃ | CH₃ | (CH₂)₂OH | CH₃COO⁻ | × | × | × |
| Comparative example3 | BMI-Br | | | | | × | × | × |
| Comparative example4 | Glucose | | | | | × | × | × |
| Comparative example5 | Gelatin | | | | | × | × | × |
| Comparative example6 | Albumin | | | | | × | × | × |
| Comparative example9 | No additive (protein only) | | | | | × | × | × |
| Reference example1 | (CH₂)₂OH | (CH₂)₂OH | (CH₂)₂OH | H | CH₃COO⁻ | O | - (Protein insoluble) | - (Protein insoluble) |
| Reference example2 | 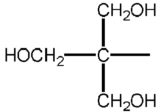 | H | H | H | CH₃CH(OH)COO⁻ | O | - (Protein insoluble) | - (Protein insoluble) |

Further, as can be seen from the results in Table 14 concerning the state of an aqueous solution and under the condition of 40°C, it was confirmed that even in the cases of the 50% aqueous solutions containing the compounds of the present invention, a refolding effect as well as a secondary structure retention effect were exhibited as were the cases where the protein was preserved in the solid samples.

That is, it was indicated that the product of the present invention, even when used as an aqueous solution, is an excellent preserving material for proteins, which does not denature proteins over a long period of time even under a high-temperature condition; and that the product is also useful as a protein refolding agent.

**[Table 14]**

| Compound | 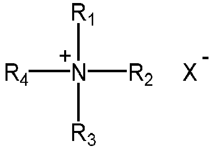 | | | | | Aqueous solution concentration (wt%) | Protein long-term stability (after 30 days) |
|---|---|---|---|---|---|---|---|
| | | | | | | | Stabilizer: Protein =1:1 |
| | R₁ | R₂ | R₃ | R₄ | X⁻ | | |
| Working example23 | (CH₂)₂OH | H | H | H | CH₃(CH₂)₁₀COO⁻ | 50 | O |
| Working example24 | (CH₂)₂OH | (CH₂)₂OH | H | H | CH₃(CH₂)₁₀COO⁻ | 50 | O |
| Working example25 | (CH₂)₂OH | (CH₂)₂OH | (CH₂)₂OH | H | CH₃(CH₂)₁₀COO⁻ | 50 | O |
| Working example26 | 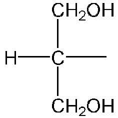 | H | H | H | CH₃(CH₂)₁₀COO⁻ | 50 | O |
| Working example27 | 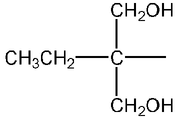 | H | H | H | CH₃(CH₂)₁₀COO⁻ | 50 | O |
| Working example35 | | | | | HOCH₂COO⁻ | 50 | O |
| Working example3 | 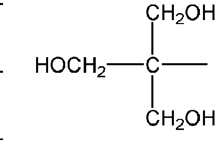 | H | H | H | CH₃COO⁻ | 50 | O |
| Working example28 | | | | | CH₃(CH₂)₁₀COO⁻ | 50 | O |
| Working example36 | | | | | HOCH₂COO⁻ | 50 | O |
| Working example48 | | | | | HOOC(CH₂)₂COO⁻ | 50 | O |
| Working example60 | 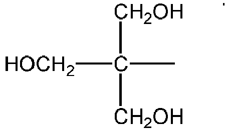 | H | H | H | Ph-COO⁻ | 50 | O |
| Working example78 | | | | | Br⁻ | 50 | O |
| Working example83 | | | | | H₂PO₄⁻ | 50 | O |
| Working example29 | 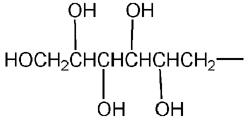 | H | H | H | CH₃(CH₂)₁₀COO⁻ | 50 | O |
| Comparative example1 | CH₃(CH₂)₃ | CH₃(CH₂)₃ | CH₃(CH₂)₃ | CH₃(CH₂)₃ | CH₃CH(OH)COO^{~} | 50 | × |
| Comparative example2 | CH₃ | CH₃ | CH₃ | (CH₂)₂OH | CH₃COO⁻ | 50 | × |
| Comparative example3 | BMI-Br | | | | | 50 | × |
| Comparative example4 | Glucose | | | | | 50 | × |
| Comparative example6 | Albumin | | | | | 50 | × |
| Comparative example10 | Buffer | | | | | 10mM | × |

### 4. DNA long-term stability

A 50% aqueous solution of each compound shown in Table 15 was prepared, followed by adding and dissolving DNA thereinto so that the concentrations shown in Table 15 would be achieved, and then distilling away water under a reduced pressure. After distilling away water at a reduced pressure, a solid sample obtained was placed in a thermo-hygrostat set to a condition of 40°C, 80%RH which were higher than a temperature and humidity generally employed to preserve DNA. After leaving the sample therein for a given period of time, each sample was then collected so as to measure the UV spectrum of the DNA preserved in each compound by a method described below, thereby making it possible to confirm the long-term stability of the DNA.

The results thereof are shown in Table 15. There, "o" was given to samples exhibiting a long-term stability with regard to DNA; "x" was given to samples not exhibiting a long-term stability with regard to DNA.

DNA is such that when in an active state, the double-helical structure thereof is maintained, and peaks are observed near 260 nm in the absorption of UV spectrum; and that when denatured, a relative absorbance of DNA in terms of UV absorption will significantly increase. As for each of the compounds of the working examples shown in Table 15, peaks indicating absorption by the DNA in the organic ammonium salt were obtained at 258 nm based on a difference between a 0.1 wt% DNA solution and a sample with no DNA dissolved therein (blank), and the peaks were of a similar level as that of absorption (259 nm) by DNA dissolved in water (1 wt%); it was confirmed that the DNA dissolved in the organic ammonium salt had retained an active double-helical structure.

That is, it was indicated that the product of the present invention is superior in preservation stability with regard to DNA, and that it is thus useful as a preserving material for nucleic acids such as DNA.

**[Table 15]**

| Compound | 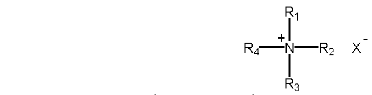 | | | | | DNA long-term stability (after 30 days) | | |
|---|---|---|---|---|---|---|---|---|
| | | | | | | Stabilizer: DNA =10:1 | Stabilizer:DNA =1:1 | Stabilizer: DNA =0.1:1 |
| | R₁ | R₂ | R₃ | R₄ | x⁻ | | | |
| Working example25 | (CH₂)₂OH | (CH₂)₂OH | (CH₂)₂OH | H | CH₃(CH₂)₁₀COO⁻ | ○ | ○ | ○ |
| Working example26 | 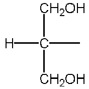 | H | H | H | CH₃(CH₂)₁₀COO⁻ | ○ | ○ | ○ |
| Working example27 | 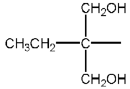 | H | H | H | CH₃(CH₂)₁₀COO⁻ | ○ | ○ | ○ |
| Working example35 | | | | | HOCH₂COO⁻ | ○ | ○ | ○ |
| Working example28 | 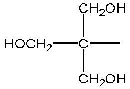 | H | H | H | CH₃(CH₂)₁₀COO⁻ | ○ | ○ | ○ |
| Working example48 | | | | | HOOC(CH₂)₂COO⁻ | ○ | ○ | ○ |
| Comparative example1 | CH₃(CH₂)₃ | CH₃(CH₂)₃ | CH₃(CH₂)₃ | CH₃(CH₂)₃ | CH₃CH(OH)COO⁻ | × | × | × |
| Comparative example2 | CH₃ | CH₃ | CH₃ | (CH₂)₂OH | CH₃COO⁻ | × | × | × |
| Comparative example3 | BMI-Br | | | | | × | × | × |
| Comparative example4 | Glucose | | | | | × | × | × |
| Comparative example5 | Gelatin | | | | | × | × | × |
| Comparative example6 | Albumin | | | | | × | × | × |
| Comparative example11 | No additive (DNA only) | | | | | × | × | × |
| Reference example1 | (CH₂)₂OH | (CH₂)₂OH | (CH₂)₂OH | H | CH₃COO⁻ | ○ | (DNA insoluble) | (DNA insoluble) |
| Reference example2 | 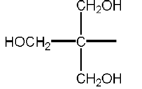 | H | H | H | CH₃CH(OH)COO⁻ | ○ | (DNA insoluble) | (DNA insoluble) |

Further, as can be seen from the results in Table 16 concerning the state of an aqueous solution and under the condition of 40°C, it was confirmed that even in the cases of the 50% aqueous solutions containing the compounds of the present invention, the DNA had retained an active double-helical structure as were the cases where the DNA was preserved in the solid samples.

That is, it was indicated that the product of the present invention, even when used as an aqueous solution, is superior in preservation stability with regard to DNA, and is thus useful as a preserving material for nucleic acids such as DNA.

**[Table 16]**

| Compound | 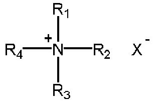 | | | | | Aqueous solution concentration (wt%) | DNA long-term stability (after 30 days) |
|---|---|---|---|---|---|---|---|
| | | | | | | | Stabilizer: DNA=1 : 1 |
| | R₁ | R₂ | R₃ | R₄ | X⁻ | | |
| Working example25 | (CH₂)₂OH | (CH₂)₂OH | (CH₂)₂OH | H | CH₃(CH₂)₁₀COO⁻ | 50 | ○ |
| Working example26 | 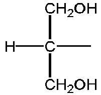 | H | H | H | CH₃(CH₂)₁₀COO⁻ | 50 | ○ |
| Working example27 | 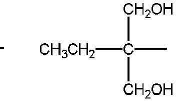 | H | H | H | CH₃(CH₂)₁₀COO⁻ | 50 | ○ |
| Working example35 | | | | | HOCH₂COO⁻ | 50 | ○ |
| Working example28 | 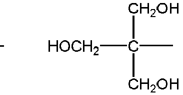 | H | H | H | CH₃(CH₂)₁₀COO⁻ | 50 | ○ |
| Working example48 | | | | | HOOC(CH₂)₂COO⁻ | 50 | ○ |
| Comparative example1 | CH₃(CH₂)₃ | CH₃(CH₂)₃ | CH₃(CH₂)₃ | CH₃(CH₂)₃ | CH₃CH(OH)COO⁻ | 50 | × |
| Comparative example2 | CH₃ | CH₃ | CH₃ | (CH₂)₂OH | CH₃COO⁻ | 50 | × |
| Comparative example3 | BMI-Br | | | | | 50 | × |
| Comparative example4 | Glucose | | | | | 50 | × |
| Comparative example6 | Albumin | | | | | 50 | × |
| Comparative example10 | Buffer | | | | | 10mM | × |

### 5. Enzyme long-term stability (2)

A method for preparing a solvent-distilled-away sample from each dissolution solution shown in Table 17 was such that a 50% aqueous solution of each compound was prepared, followed by adding and dissolving urease thereinto so that the enzyme concentration shown in Table 17 would be achieved, and then distilling away water under a reduced pressure to obtain a solid sample. Meanwhile, mixing by mortar was such that the product of the present invention and urease of the enzyme concentration shown in Table 17 were mixed by means of a mortar to obtain a solid sample.

Next, each sample was left in a thermo-hygrostat set to a condition of 40°C, 80%RH which were higher than a temperature and humidity generally employed to preserve enzymes. After leaving the sample therein for a given period of time, each sample was then collected so as to measure the activity retention rate of the enzyme preserved in each compound by a method described below, thereby making it possible to confirm the retainability of the steric structure of the enzyme in each compound and the stabilization effect.

As compared to the samples obtained by performing mixing using the mortar, higher activity retention rates were observed in the cases of the solid samples produced from the dissolution solutions. As can be seen from the above results, by dissolving the product of the present invention with an enzyme, the steric structure inside the enzyme can be retained, and there can thus be obtained a biological sample treatment agent exhibiting a high activity retainability.

**[Table 17]**

| Compound | 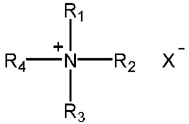 | | | | | Enzyme activity retention rate(%) (after 30 days) Stabilizer: Enzyme=1:1 | |
|---|---|---|---|---|---|---|---|
| | | | | | | Solvent distilled away from dissolution solution | Mixed by mortar |
| | R₁ | R₂ | R₃ | R₄ | X⁻ | | |
| Working example23 | (CH₂)₂OH | H | H | H | CH₃(CH₂)₁₀COO^{~} | 5 | 0 |
| Working example24 | (CH₂)₂OH | (CH₂)₂OH | H | H | CH₃(CH₂)₁₀COO⁻ | 25 | 5 |
| Working example25 | (CH₂)₂OH | (CH₂)₂OH | (CH₂)₂OH | H | CH₃(CH₂)₁₀COO⁻ | 41 | 5 |
| Working example26 | 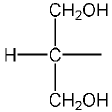 | H | H | H | CH₃(CH₂)₁₀COO⁻ | 81 | 13 |
| Working example27 | 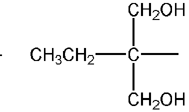 | H | H | H | CH₃(CH₂)₁₀COO⁻ | 74 | 10 |
| Working example35 | | | | | HOCH₂COO⁻ | 16 | 0 |
| Working example3 | 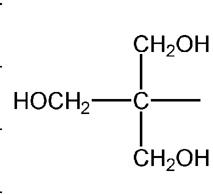 | H | H | H | CH₃COO⁻ | 50 | 11 |
| Working example28 | | | | | CH₃(CH₂)₁₀COO⁻ | 65 | 12 |
| Working example36 | | | | | HOCH₂COO⁻ | 41 | 7 |
| Working example48 | | | | | HOOC(CH₂)₂COO⁻ | 48 | 9 |
| Working example60 | | | | | Ph-COO⁻ | 20 | 0 |

| Compound | 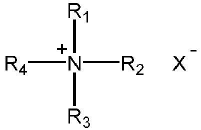 | | | | | Enzyme activity retention rate (%)(after 30 days) Stabilizer: Enzyme = 1 : 1 | |
|---|---|---|---|---|---|---|---|
| | | | | | | Solvent distilled away from dissolution solution | Mixed by mortar |
| | R₁ | R₂ | R₃ | R₄ | X⁻ | | |
| Working example78 | 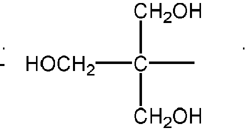 | H | H | H | Br⁻ | 4 | 0 |
| Working example83 | | | | | H₂PO₄⁻ | 24 | 0 |
| Working example29 | 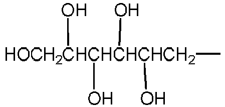 | H | H | H | CH₃(CH₂)₁₀COO⁻ | 18 | 0 |
| Comparative example1 | OH₃(OH₂)₃ | CH₃(CH₂)₃ | CH₃(CH₂)₃ | CH₃(CH₂)₃ | CH₃CH(OH)COO⁻ | 0 | 0 |
| Comparative example2 | CH₃ | CH₃ | CH₃ | (CH₂)₂OH | CH₃COO⁻ | 3 | 0 |
| Comparative example3 | BMI-Br | | | | | 0 | 0 |
| Comparative example4 | Glucose | | | | | 0 | 0 |
| Comparative example5 | Gelatin | | | | | 0 | 0 |
| Comparative example6 | Albumin | | | | | 0 | 0 |
| Comparative example7 | No additive (enzyme only) | | | | | 0 | 0 |

### 6. Metal oxide dispersibility test

As for the compound of each working and comparative example shown in Table 18, 0.25 g of each compound, 0.50 g of water and 0.10 g of zirconium oxide (IV) (by Wako Pure Chemical Industries, Ltd.) were mixed by a rotation and revolution mixer (ARE-310 by THINKY CORPORATION) at 2,000 rpm under a condition of 1 min × 5 times, followed by visually confirming a dispersed state thereof. Examples where zirconium oxide was dispersed were evaluated as "o," whereas examples where zirconium oxide was not dispersed, but had precipitated were evaluated as "x". The results are shown in Table 18.

In the case of each compound of the working examples, zirconium oxide was able to be favorably dispersed, and a dispersion liquid was thus obtained. In contrast, in the case of the compound of the comparative example 12, zirconium oxide immediately precipitated, and therefore did not disperse.

That is, it is assumed that the reason that the organic ammonium salt of the present invention was able to favorably disperse zirconium oxide was because an affinity to the oxygen atoms in the hydrogen bond-accepting zirconium oxide was improved, and because there was an affinity between the hydroxy groups in the quaternary ammonium salt and the metal atoms in the coordinating zirconium oxide, owing to the structural characteristic of the quaternary ammonium cation that is comprised of a cation having many hydrogen bond-donating and coordinating hydroxy groups.

**[Table 18]**

| Compound | 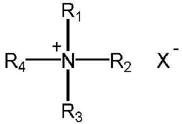 | | | | | Zirconium oxide dispersibility |
|---|---|---|---|---|---|---|
| | R₁ | R₂ | R₃ | R₄ | X⁻ | |
| Working example25 | (CH₂)₂OH | (CH₂)₂OH | (CH₂)₂OH | H | CH₃(CH₂)₁₀COO⁻ | ○ |
| Working example26 | 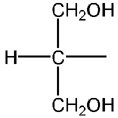 | H | H | H | CH₃(CH₂)₁₀COO⁻ | ○ |
| Working example27 | 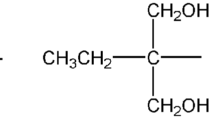 | H | H | H | CH₃(CH₂)₁₀COO⁻ | ○ |
| Working example35 | | | | | HOCH₂COO⁻ | ○ |
| Working example28 | 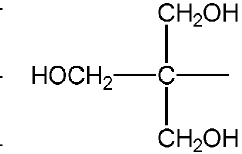 | H | H | H | CH₃(CH₂)₁₀COO⁻ | ○ |
| Working example36 | | | | | HOCH₂COO⁻ | ○ |
| Working example48 | | | | | HOOC(CH₂)₂COO⁻ | ○ |
| Working example1 09 | | | | | ⁻OOC(CH₂)₂COO⁻ | ○ |
| Comparative example12 | CH₃(CH₂)₃ | CH₃(CH₂)₃ | CH₃(CH₂)₃ | CH₃(CH₂)₃ | Br⁻ | × |

As for the compound of each working and comparative example shown in Table 19, 0.25 g of each compound, 0.50 g of water and 0.10 g of titanium oxide (IV) (by Wako Pure Chemical Industries, Ltd.) were mixed by a rotation and revolution mixer (ARE-310 by THINKY CORPORATION) at 2,000 rpm under a condition of 1 min × 5 times, followed by visually confirming a dispersed state thereof. Examples where titanium oxide was dispersed were evaluated as "o," whereas examples where titanium oxide was not dispersed, but had precipitated were evaluated as "x". The results are shown in Table 19.

In the case of each compound of the working examples, titanium oxide was able to be favorably dispersed, and a dispersion liquid was thus obtained. In contrast, in the case of the compound of the comparative example 12, a low dispersibility was observed as titanium oxide had precipitated.

That is, it is assumed that the reason that the organic ammonium salt of the present invention was able to favorably disperse titanium oxide was because an affinity to the oxygen atoms in the hydrogen bond-accepting titanium oxide was improved, and because there was an affinity between the hydroxy groups in the quaternary ammonium salt and the metal atoms in the coordinating titanium oxide, owing to the structural characteristic of the quaternary ammonium cation that is comprised of a cation having many hydrogen bond-donating and coordinating hydroxy groups.

These results indicate that the organic ammonium salt of the present invention is superior in affinity to inorganic materials such as metals and metal oxides having hydrogen bond-accepting functional groups, and is thus useful in, for example, treatment agents of these inorganic materials and cosmetic products.

**[Table 19]**

| Compound | 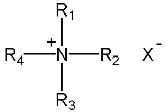 | | | | | Titanium oxide dispersibility |
|---|---|---|---|---|---|---|
| | R₁ | R₂ | R₃ | R₄ | X⁻ | |
| Working example25 | (CH₂)₂OH | (CH₂)₂OH | (CH₂)₂OH | H | CH₃(CH₂)₁₀COO⁻ | ○ |
| Working example26 | 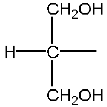 | H | H | H | CH₃(CH₂)₁₀COO⁻ | ○ |
| Working example27 | 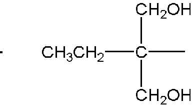 | H | H | H | CH₃(CH₂)₁₀COO⁻ | ○ |
| Working example35 | | | | | HOCH₂COO⁻ | ○ |
| Working example28 | 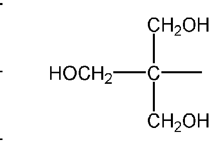 | H | H | H | CH₃(CH₂)₁₀COO⁻ | ○ |
| Working example36 | | | | | HOCH₂COO⁻ | ○ |
| Working example48 | | | | | HOOC(CH₂)₂COO⁻ | ○ |
| Working example109 | | | | | ⁻OOC(CH₂)₂COO⁻ | ○ |
| Comparative example12 | CH₃(CH₂)₃ | CH₃(CH₂)₃ | CH₃(CH₂)₃ | CH₃(CH₂)₃ | Br⁻ | × |

### 7. Carbon nanotube dispersibility test

As for the compound of each working and comparative example shown in Table 20, 0.25 g of each compound, 0.75 g of water and 0.025 g of carbon nanotubes (multi-walled, 3 to 20 nm) (by Wako Pure Chemical Industries, Ltd.) were mixed by a rotation and revolution mixer (ARE-310 by THINKY CORPORATION) at 2,000 rpm under a condition of 1 min × 5 times, followed by visually confirming a dispersed state thereof. Examples where the carbon nanotubes were dispersed were evaluated as "o," whereas examples where the carbon nanotubes were not dispersed, but had precipitated were evaluated as "x". The results are shown in Table 20.

As a result, in the case of each compound of the working examples, the carbon nanotubes were able to be favorably dispersed, and a low-viscosity dispersion liquid with a favorable handling property was thus obtained. In contrast, in the case of the compound of the comparative example 12, the carbon nanotubes did not disperse, but precipitated.

That is, it is assumed that the reason that the organic ammonium salt of the present invention was able to favorably disperse the carbon nanotubes was because an affinity to the carbon-carbon unsaturated bonds (π-electron system) in the hydrogen bond-accepting carbon nanotubes was improved, owing to the structural characteristic of the quaternary ammonium cation that is comprised of a cation having many hydrogen bond-donating hydroxy groups.

These results indicate that the organic ammonium salt of the present invention is superior in affinity to compounds and materials having carbon-carbon unsaturated bonds, and is thus useful in, for example, treatment agents of these materials.

**[Table 20]**

| Compound | 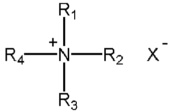 | | | | | Carbon nanotube dispersibility |
|---|---|---|---|---|---|---|
| | R₁ | R₂ | R₃ | R₄ | X⁻ | |
| Working example25 | (OH₂)₂OH | (CH₂)₂OH | (CH₂)₂OH | H | CH₃(CH₂)₁₀COO⁻ | ○ |
| Working example26 | 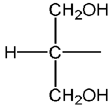 | H | H | H | CH₃(CH₂)₁₀COO⁻ | ○ |
| Working example27 | 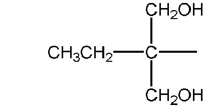 | H | H | H | OH₃(OH₂)₁₀COO⁻ | ○ |
| Working example35 | | | | | HOCH₂COO⁻ | ○ |
| Working example28 | 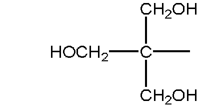 | H | H | H | CH₃(CH₂)₁₀COO⁻ | ○ |
| Working example48 | | | | | HOOC(CH₂)₂COO⁻ | ○ |
| Comparative example12 | CH₃(CH₂)₃ | CH₃(CH₂)₃ | CH₃(CH₂)₃ | CH₃(CH₂)₃ | Br | × |

### 8. Organic dye dispersibility test

As for the compound of each working and comparative example shown in Table 21, 0.2 g of each compound, 0.7 g of water and 0.1 g of Vali Fast Black 3830 as an organic dye (by ORIENT CHEMICAL INDUSTRIES CO., LTD.) were subjected to ultrasonic dispersion, followed by leaving them for 24 hours so as to then visually confirm a mixed and dispersed state thereof after such 24 hours. Examples where the organic dye was dispersed were evaluated as "o," whereas examples where particles had visually precipitated were evaluated as "x". The results are shown in Table 21.

As a result, in the case of each compound of the working examples, the organic dye was able to be favorably dispersed, and a uniform dispersion liquid was thus obtained. In contrast, in the case of the compound of the comparative example 12, the organic dye did not disperse, but precipitated.

That is, it is assumed that the reason that the organic ammonium salt of the present invention was able to favorably disperse the organic dye was because an affinity to the nitrogen and oxygen atoms in the hydrogen bond-accepting organic dye was improved, and because an affinity between the hydroxy groups in the quaternary ammonium salt and the metal atoms (chrome) in the coordinating organic dye was improved, owing to the structural characteristic of the quaternary ammonium cation that is comprised of a cation having many hydrogen bond-donating and coordinating hydroxy groups.

These results indicate that the organic ammonium salt of the present invention is useful in treatment agents of organic compounds and materials having hydrogen bond-accepting functional groups, such as an organic dye.

**[Table 21]**

| Compound | 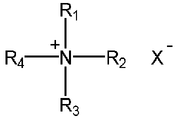 | | | | | Dye dispersibility |
|---|---|---|---|---|---|---|
| | R₁ | R₂ | R₃ | R₄ | X⁻ | |
| Working example25 | (CH₂)₂OH | (CH₂)₂OH | (CH₂)₂OH | H | CH₃(CH₂)₁₀COO⁻ | ○ |
| Working example26 | 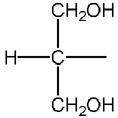 | H | H | H | CH₃(CH₂)₁₀COO⁻ | ○ |
| Working example28 | 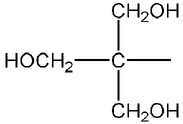 | H | H | H | CH₃(CH₂)₁₀COO⁻ | ○ |
| Working example36 | | | | | HOCH₂COO⁻ | ○ |
| Working example48 | | | | | HOOO(OH₂)₂OOO⁻ | ○ |
| Working example 109 | | | | | ⁻OOC(CH₂)₂COO⁻ | ○ |
| Comparative example12 | CH₃(CH₂)₃ | CH₃(CH₂)₃ | CH₃(CH₂)₃ | CH₃(CH₂)₃ | Br⁻ | × |

### 9. Redispersibility

Dispersion liquids of zirconium oxide, titanium oxide and Vali Fast Black 3830 as an organic dye that are prepared in each of the working examples 28, 36, 48, 109 and comparative example 12 shown in Table 22, were each subjected to dehydration at 50°C under a reduced pressure for three hours, thereby obtaining a dried compound. There, 0.50 g of water was added to the samples of zirconium oxide and titanium oxide, whereas 0.7 g of water was added to the samples of Vali Fast Black 3830 as an organic dye, followed by performing mixing with a rotation and revolution mixer (ARE-310 by THINKY CORPORATION) at 2,000 rpm under a condition of 1 min × 5 times, and then visually confirming a dispersed state thereof after mixing. As a result, in the case of each compound of the working examples, dispersion took place in a favorable manner, and a uniform dispersion liquid was thus obtained. In contrast, in the case of the compound of the comparative example, precipitates were observed immediately after performing mixing with the mixer.

As can be seen from these results, a favorable dispersibility was still confirmed even after performing redispersion after drying the compound that had once been subjected to dispersion. Further, in addition to the dispersion method concerning the system where the metal oxide or organic dye is to be added and dispersed into the aqueous solution of the organic ammonium salt of the present invention, a favorable dispersibility was also observed in the system where the metal oxide or organic dye is to be dispersed by adding water to the powdery state thereof; it was indicated that treatment agents of organic compounds and materials having hydrogen bond-accepting functional groups, cosmetic products and the like can be redispersed, and that the organic ammonium salt of the present invention is thus useful for these purposes.

Further, as the amine compounds and acids serving as compound raw materials, there were used compounds registered in Japanese Standards of Quasi-drug Ingredients; they are highly safe, and it was indicated that they are effective for these purposes.

**[Table 22]**

| Compound | 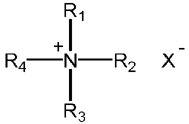 | | | | | Redispersibility | | |
|---|---|---|---|---|---|---|---|---|
| | | | | | | Zirconium oxide | Titanium oxide | Dye |
| | R₁ | R₂ | R₃ | R₄ | X⁻ | | | |
| Working example28 | 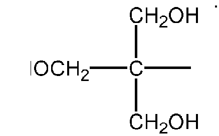 | H | H | H | CH₃(CH₂)₁₀COO⁻ | ○ | ○ | ○ |
| Working example36 | | | | | HOCH₂COO⁻ | ○ | ○ | ○ |
| Working example48 | | | | | HOOC(CH₂)₂COO⁻ | ○ | ○ | ○ |
| Working example1 09 | | | | | ⁻OOC(CH₂)₂COO⁻ | ○ | ○ | ○ |
| Comparative example12 | CH₃(CH₂)₃ | CH₃(CH₂)₃ | CH₃(CH₂)₃ | CH₃(CH₂)₃ | Br⁻ | × | × | x |

### 10. Metal oxide dispersibility test 2

As for the compound of each working and comparative example shown in Table 23, 0.25 g of each compound, 0.50 g of water and 0.10 g of zinc oxide (by ISHIHARA SANGYO KAISHA, LTD.) were mixed by a rotation and revolution mixer (ARE-310 by THINKY CORPORATION) at 2,000 rpm under a condition of 1 min × 5 times, followed by visually confirming a dispersed state thereof. Examples where zinc oxide was dispersed were evaluated as "o," whereas examples where zinc oxide was not dispersed, but had precipitated were evaluated as "x". The results are shown in Table 23.

In the case of each compound of the working examples, zinc oxide was able to be favorably dispersed, and a dispersion liquid was thus obtained. In contrast, in the case of the compound of the comparative example 12, zinc oxide did not disperse, but immediately precipitated.

That is, it is assumed that the reason that the organic ammonium salt of the present invention was able to favorably disperse zinc oxide was because an affinity to the oxygen atoms in the hydrogen bond-accepting zinc oxide was improved, and because there was an affinity between the hydroxy groups in the quaternary ammonium salt and the metal atoms in the coordinating zinc oxide, owing to the structural characteristic of the quaternary ammonium cation that is comprised of a cation having many hydrogen bond-donating and coordinating hydroxy groups.

**[Table 23]**

| Compound | 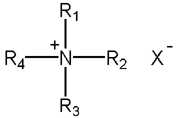 | | | | | Zinc oxide dispersibility |
|---|---|---|---|---|---|---|
| | R₁ | R₂ | R₃ | R₄ | X⁻ | |
| Working example28 | 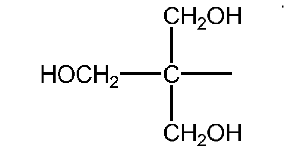 | H | H | H | CH₃(CH₂)₁₀COO⁻ | ○ |
| Working example36 | | | | | HOCH₂COO⁻ | ○ |
| Working example48 | | | | | HOOC(CH₂)₂COO⁻ | ○ |
| Working example1 09 | | | | | ⁻OOC(CH₂)₂COO⁻ | ○ |
| Comparative example12 | CH₃(CH₂)₃ | CH₃(CH₂)₃ | CH₃(CH₂)₃ | CH₃(CH₂)₃ | Br⁻ | × |

### 11. Metal oxide dispersibility test 3

As for the compound of each working and comparative example shown in Table 24, 0.25 g of each compound, 0.50 g of water and 0.10 g of barium titanate (by KANTO CHEMICAL CO., INC.) were mixed by a rotation and revolution mixer (ARE-310 by THINKY CORPORATION) at 2,000 rpm under a condition of 1 min × 5 times, followed by visually confirming a dispersed state thereof. Examples where barium titanate was dispersed were evaluated as "o," whereas examples where barium titanate was not dispersed, but had precipitated were evaluated as "x". The results are shown in Table 24.

In the case of each compound of the working examples, barium titanate was able to be favorably dispersed, and a dispersion liquid was thus obtained. In contrast, in the case of the compound of the comparative example 12, barium titanate did not disperse, but immediately precipitated.

That is, it is assumed that the reason that the organic ammonium salt of the present invention was able to favorably disperse barium titanate was because an affinity to the oxygen atoms in the hydrogen bond-accepting barium titanate was improved, and because there was an affinity between the hydroxy groups in the quaternary ammonium salt and the metal atoms in the coordinating zinc oxide, owing to the structural characteristic of the quaternary ammonium cation that is comprised of a cation having many hydrogen bond-donating and coordinating hydroxy groups.

**[Table 24]**

| Compound | 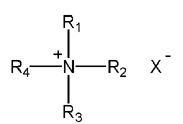 | | | | | Barium titanate dispersibility |
|---|---|---|---|---|---|---|
| | R₁ | R₂ | R₃ | R₄ | X⁻ | |
| Working example28 | 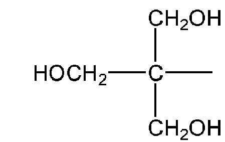 | H | H | H | CH₃(CH₂)₁₀COO⁻ | ○ |
| Working example36 | | | | | HOCH₂COO⁻ | ○ |
| Working example48 | | | | | HOOC(CH₂)₂COO⁻ | ○ |
| Working example1 09 | | | | | ⁻OOC(CH₂)₂COO⁻ | ○ |
| Comparative example12 | CH₃(CH₂)₃ | CH₃(CH₂)₃ | CH₃(CH₂)₃ | CH₃(CH₂)₃ | Br⁻ | × |

## Claims

1. An organic ammonium salt that is solid at 25°C, comprising an anion and an ammonium cation represented by the following formula (I):
[Chemical formula 1]
N ⁺ R ₙ H _{4 - n} (I)
wherein R independently represents a hydroxyalkyl group in which there is at least one hydroxy group, an alkyl moiety is a linear or branched moiety having 1 to 10 carbon atoms, and the alkyl moiety may contain an oxygen atom(s); a carboxyalkyl group in which there is at least one carboxy group, an alkyl moiety is a linear or branched moiety having 1 to 10 carbon atoms, and the alkyl moiety may contain an oxygen atom(s); or a hydroxycarboxyalkyl group in which there are at least one hydroxy group and at least one carboxy group, an alkyl moiety is a linear or branched moiety having 1 to 10 carbon atoms, and the alkyl moiety may contain an oxygen atom(s), and
wherein n represents an integer of 0 to 4.

2. The organic ammonium salt according to claim 1, wherein n in the formula (I) represents an integer of 1 to 4.

3. The organic ammonium salt according to claim 2, wherein R represents a linear or branched monohydroxyalkyl or monocarboxyalkyl group having 1 to 10 carbon atoms, and n represents an integer of 1 to 4.

4. The organic ammonium salt according to claim 2, wherein at least one R represents a linear or branched polyhydroxyalkyl group having at least two hydroxy groups and 1 to 10 carbon atoms, and n represents an integer of 1 to 4.

5. The organic ammonium salt according to any one of claims 1 to 4, wherein the anion of the organic ammonium salt is a carboxylic acid anion, halide anion, sulfur-based anion, fluorinebased anion, nitrogen oxide-based anion, phosphorus-based anion or cyano-based anion.

6. A hydrogen-bonding material treatment agent containing the organic ammonium salt according to any one of claims 1 to 5.

7. The hydrogen-bonding material treatment agent according to claim 6, wherein the hydrogen-bonding material is a biological sample.

8. The hydrogen-bonding material treatment agent according to claim 7, wherein the biological sample is a biocatalyst, a protein or a nucleic acid.

9. A solid composition containing the hydrogen-bonding material treatment agent according to any one of claims 6 to 8 and a hydrogen-bonding material.

10. A biological sample solution containing the hydrogen-bonding material treatment agent according to any one of claims 6 to 9, a biological sample and a solvent.
